# EUROPEAN PATENT APPLICATION

(11) **EP 1 130 016 A1**
(43) Date of publication of application: **05.09.2001**
(21) Application number: 99971804.2
(22) Date of filing: 10.11.1999
(51) Int. Cl.: C07D 263/22, C07D 413/12, C07D 417/10, A61K 31/421, A61K 31/435, A61K 31/445, A61K 31/496, A61K 31/541, A61K 31/55, A61K 31/44

(54) **THIOCARBAMIC ACID DERIVATIVES**

(30) Priority: 11.11.1998 JP 32013798; 27.09.1999 JP 27323099
(71) Applicant: HOKURIKU SEIYAKU CO., LTD., Katsuyama-shi, Fukui 911-0813 (JP)
(72) Inventor: KADO, Noriyuki, Katsuyama-shi, Fukui 911-0813 (JP); TOKUYAMA, Ryukou, Katsuyama-shi, Fukui 911-0813 (JP); TSUBOUCHI, Masatoshi, Katsuyama-shi, Fukui 911-0813 (JP); TOMITA, Yayoi, Katsuyama-shi, Fukui 911-0813 (JP)
(74) Representative: Sternagel, Fleischer, Godemeyer & Partner Patentanwälte
(86) International application number: JP9906260
(87) International publication number: WO0027830

(57) **Abstract**

Thiocarbamic acid derivatives represented by the following general formula or salts thereof which are useful as antibacterial agents: 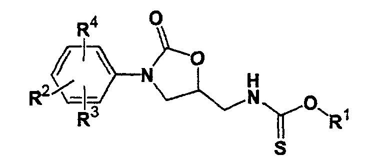 wherein R¹ represents an alkyl group which may be substituted, or a cycloalkyl group which may be substituted; and R², R³, and R⁴ independently represent hydrogen atom, a halogen atom, an alkyl group which may be substituted, an alkogyl group which may be substituted, an amino group which may be substituted, an alkanoyl group which may be substituted, a cycloalkyloxy group which has a heteroatom as a ring constituting atom and which may be substituted, or a saturated heterocyclic group which may be substituted; or any two of R², R³, and R⁴ may bind to each other to form, together with the benzene ring, a condensed hydrocarbon ring which may be substituted.

## Description

### Technical Field

The present invention relates to novel thiocarbamic acid derivatives or salts thereof which are useful as medicaments, particularly as antibacterial agents.

### Background Art

As compounds having the 3-aryl-2-oxooxazolidine structure which are analogous to the compounds according to the present invention, Japanese Patent Unexamined Publication No.(Sho) 60-8277, Journal of Medicinal Chemistry, Vol. 39, p.673 (1996) and the like disclose N-[(3-aryl-2-oxooxazolidin-5-yl)methyl]acetamide derivatives; Current Pharmaceutical Design, Vol. 2, p.175 (1996), Journal of Medicinal Chemistry, Vol. 32, p.1673 (1989) and the like disclose 3-aryl-5-hydroxymethyl-2-oxooxazolidine derivatives, 3-aryl-5-halogenomethyl-2-oxooxzolidine derivatives and the like; and Japanese Patent Unexamined Publication No.(Hei) 9-316073 and the like disclose N-(3-heteroaryl-2-oxooxazolidin-5-yl)methylthioacetamide derivatives, N-(3-heteroaryl-2-oxooxazolidin-5-yl)methyl-N'-methylthiourea derivatives and the like. These compounds are described to have antibacterial activities against Gram-positive bacteria. However, the antibacterial activities of these compounds are insufficient, and development of more excellent antibacterial agents has been desired.

Various antibacterial agents such as antibiotics and synthetic antibacterial agents, each having different mechanism of action, have been clinically used as therapeutic agents for infectious diseases. Upon the use of these antibacterial agents, appearance of multiple drug-resistant bacteria including bacteria such as Methicillin-resistant Staphylococcus aureus (MRSA) has become a worldwide problem. In patients having an underlying disease and being subjected to chemotherapy, those being subjected to administration of an immunosuppressive agent in organ transplantation, and those suffering from AIDS or another disease as so-called immunocompromized hosts, increase in opportunistic infection has been recognized, and development of chemotherapy for diseases caused by atypical acid-fast microorganism has especially desired for which only a few effective antibacterial - agents are available. Moreover, need of chemotherapy of infectious diseases caused by Mycobacterium avium complex (Mycobacterium avium, Mycobacterium intracellulare) among atypical acid-fast microorganisms have become a serious problem. The object of the present invention is to provide compounds which have excellent antibacterial activity against clinically isolated strains and atypical acid-fast microorganisms including multiple drug-resistant bacteria as well as type bacteria.

### Disclosure of the Invention

The inventors of the present invention made intensive studies to achieve the aforementioned object. As a result, they found that novel thiocarbamic acid derivatives represented by the following general formula or salts thereof have excellent antibacterial activity against clinically isolated strains and atypical acid-fast microorganisms including multiple drug-resistant bacteria as well as type bacteria. The present invention was achieved on the basis of the above findings.

The present invention thus relates to novel thiocarbamic acid derivatives represented by the following general formula (I) or salts thereof: 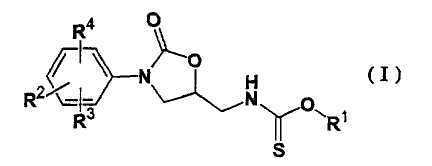 wherein R¹ represents an alkyl group which may be substituted, or a cycloalkyl group which may be substituted; and R², R³ and R⁴ independently represent hydrogen atom, a halogen atom, an alkyl group which may be substituted, an alkoxyl group which may be substituted, an amino group which may be substituted, an alkanoyl group which may be substituted, a cycloalkyloxy group which has a heteroatom as a ring constituting atom and which may be substituted, or a saturated heterocyclic group which may be substituted; or any two of R², R³ and R⁴ may bind to each other to form, together with the benzene ring, a condensed hydrocarbon ring which may be substituted.

According to the second embodiment of the present invention, there are provided novel thiocarbamic acid derivatives represented by the following general formula (II) or salts thereof: 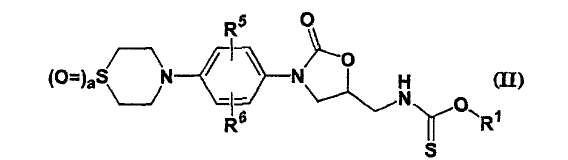 wherein R⁵ and R⁶ independently represent hydrogen atom, or a halogen atom; and symbol "a" represents an integer of from 0 to 2; and R¹ has the same meaning as that defined above.

According to the third embodiment of the present invention, there are provided novel thiocarbamic acid derivatives represented by the following general formula (III) or salts thereof: 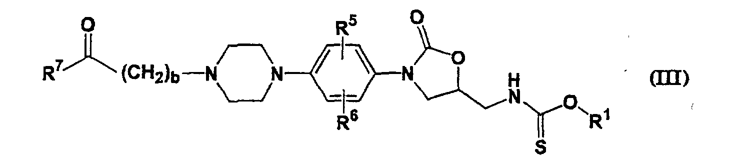 wherein R⁷ represents an alkyl group which may be substituted, an amino group which may be substituted, or an alkoxyl group which may be substituted; and symbol "b" represents an integer of from 1 to 4; and R¹, R⁵ and R⁶ have the same meanings as those defined above.

According to the forth embodiment of the present invention, there are provided novel thiocarbamic acid derivatives represented by the following general formula (IV) or salts thereof: 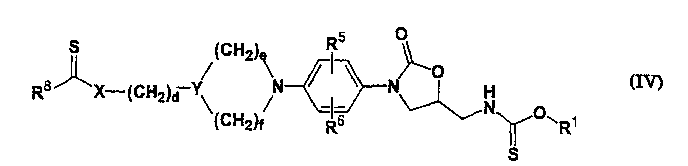 wherein R⁸ represents an alkyl group which may be substituted, a cycloalkyl group which may be substituted, an alkenyl group which may be substituted, an alkynyl group which may be substituted, an alkoxyl group which may be substituted, an alkylthio group which may be substituted, an amino group which may be substituted, a saturated heterocyclic group which may be substituted, an aryl group which may be substituted, or an aralkyl group which may be substituted; Y represents CH or nitrogen atom; X represents NH or single bond; symbol "d" represents an integer of from 0 to 3; symbols "e" and "f" independently represent an integer of from 1 to 3; and R¹, R⁵ and R⁶ have the same meanings as those defined above.

According to another aspect of the present invention, there is provided a medicament which comprises as an active ingredient the aforementioned thiocarbamic acid derivative or the salt thereof. The medicament provided by the present invention can be suitably used, for example, as an antibacterial agent.

According to a further aspect, there are provided a use of the aforementioned thiocarbamic acid derivative or the salt thereof for the manufacture of the aforementioned medicament; and a method for preventive and/or therapeutic treatment of infectious diseases, which comprises the step of administering a preventively and/or therapeutically effective amount of the aforementioned thiocarbamic acid derivative or the salt thereof to a mammal including a human.

### Best Mode for Carrying Out the Invention

Specific explanations of the novel thiocarbamic acid derivatives represented by the aforementioned general formulas (I) to (IV) of the present invention will be given below. The compounds represented by the aforementioned general formulas (II) to (IV) are characterized in that any two of R², R³ and R⁴ are independently hydrogen atom or a halogen atom in the aforementioned general formula (I). However, the scope of the present invention is not limited to the compounds represented by the aforementioned general formulas (II) to (IV), and it should be understood that any compounds which have, as any two of R², R³ and R⁴, substituents other than hydrogen atom or a halogen atom among those defined in the aforementioned general formula (I) fall within the scope of the present invention.

In the aforementioned general formulas (I) to (IV) according to the present invention, the alkyl group represented by R¹, R², R³, R⁴, R⁷ or R⁸ includes a straight or branched chain alkyl group, or cyclic alkyl group or an alkyl group as a combination thereof having from 1 to 6 carbon atoms, preferably a straight or branched chain alkyl group. Examples include methyl group, ethyl group, n-propyl group, isopropyl group, n-butyl group, isobutyl group, sec-butyl group, tert-butyl group, n-pentyl group, isopentyl group, neopentyl group, n-hexyl group and the like. The cycloalkyl group represented by R¹ or R⁸ includes a cycloalkyl group having from 3 to 6 carbon atoms. Examples include cyclopropyl group, cyclobutyl group, cyclopentyl group, cyclohexyl group and the like. The halogen atom represented by R², R³, R⁴, R⁵ or R⁶ includes fluorine atom, chlorine atom, bromine atom, and iodine atom. The alkoxyl group represented by R², R³, R⁴, R⁷ or R⁸ includes a straight or branched chain alkyl group having from 1 to 6 carbon atoms. Examples include methoxy group, ethoxy group, n-propoxy group, isopropoxy group, n-butoxy group, isobutoxy group, sec-butoxy group, tert-butoxy group, n-pentyloxy group, isopentyloxy group, neopentyloxy group, n-hexyloxygroup and the like.

In the aforementioned general formulas (I) and (IV) according to the present invention, the alkanoyl group represented by R², R³ or R⁴ includes, for example, formyl group, acetyl group, propionyl group, butyryl group, isobutyryl group, valeryl group, isovaleryl group, hexanoyl group, heptanoyl group and the like. The cycloalkyloxy group represented by R², R³ or R⁴ which has a heteroatom as a ring constituting atom includes, for example, aziridinyloxy group, azetidinyloxy group, pyrrolidinyloxy group, piperidyloxy group, hexahydro-1H-azepin-1-yloxy group, oxetanyloxy group, tetrahydrofuranyloxy group, tetrahydropyranyloxy group, thietanyloxy group, tetrahydrothiophenyloxy group, tetrahydrothiopyranyloxy group, oxazolidinyloxy group, thiazolidinyloxy group, piperazinyloxy group, morpholinyloxy group, thiomorpholinyloxy group, 1-oxidothiomorpholinyloxy group, 1,1-dioxidothiomorpholinyloxy group, hexahydro-1H-1,4-diazepin-1-yloxy group, 3-azabicyclo[3.3.0]octanyloxy group, 3,7-diazabicyclo[3.3.0]octanyloxy group and the like. The saturated heterocyclic group represented by R², R³, R⁴ or R⁸ includes, for example, aziridinyl group, azetidinyl group, pyrrolidinyl group, oxazolidinyl group, thiazolidinyl group, piperidyl group, piperazinyl group, oxetanyl group, tetrahydrofuranyl group, tetrahydropyranyl group, thietanyl group, tetrahydrothiophenyl group, tetrahydrothiopyranyl group, thiomorpholinyl group, 1-oxidethiomorpholinyl group, 1,1-dioxidothiomorpholinyl group, hexahydro-1H-azepin-1-yl group, hexahydro-1H-1,4-diazepin-1-yl group, 3-azabicyclo[3.3.0]octanyl group, 3,7-diazabicyclo[3.3.0]octanyl group and the like.

In the aforementioned general formula (I) according to the present invention, when any two of R², R³ and R⁴ bind to each other to form a condensed hydrocarbon ring together with the benzene ring, the condensed ring group includes, for example, indan-5-yl group, 1-indanon-5-yl group, inden-5-yl group, inden-6-yl group, 1-indanon-6-yl group, 2-indanon-5-yl group, 1,3-indandion-5-yl group, naphthalen-2-yl group, 1(2H)-naphthalenon-6-yl group, 1(2H)-naphthalenon-7-yl group, 1,2,3,4-tetrahydronaphthalen-6-yl group, 1,2,3,4-tetrahydro-1-naphthalenon-6-yl group, 1,2,3,4-tetrahydro-1-naphthalenon-7-yl group, 1,2,3,4-tetrahydro-2-naphthalenon-6-yl group, 1,2,3,4-tetrahydro-2-naphthalenon-7-yl group, 1,2-naphthoquinon-6-yl group, 1,2-naphthoquinon-7-yl group, 1,4-naphthoquinon-6-yl group, fluoren-2-yl group, fluoren-3-yl group, fluorenon-2-yl group, fluorenon-3-yl group, anthracen-1-yl group, anthracen-2-yl group and the like.

In the aforementioned general formula (IV) according to the present invention, the alkenyl group represented by R⁸ includes a straight or branched chain alkenyl group having from 2 to 4 carbon atoms, for example, vinyl group, propenyl group, 2-methylpropenyl group, butenyl group, butadienyl group and the like. The alkynyl group includes an alkynyl group having from 2 to 4 carbon atoms, for example, ethynyl group, propynyl group, butynyl group and the like. The alkylthio group represented by R⁸ includes an alkylthio group which comprises a straight or branched chain alkyl group having from 1 to 6 carbon atoms, for example, methylthio group, ethylthio group, n-propylthio group, isopropylthio group, n-butylthio group, isobutylthio group, sec-butylthio group, tert-butylthio group, n-pentylthio group, isopentylthio group, neopentylthio group, n-hexylthio group and the like. The aryl group represented by R⁸ means an aromatic ring group which encompasses ring systems containing two or more rings optionally having a heteroatom as a ring constituting atom. Examples include phenyl group, pyridin-2-yl group, pyridin-3-yl group, pyridin-4-yl group, pyrazin-2-yl group, pyrimidin-2-yl group, pyrimidin-4-yl group, pyrimidin-5-yl group, furan-2-yl group, furan-3-yl group, thiophen-2-yl group, thiophen-3-yl group, pyrrol-1-yl group, pyrrol-2-yl group, pyrrol-3-yl group, pyrazol-1-yl group, pyrazol-3-yl group, pyrazol-4-yl group, imidazol-1-yl group, imidazol-2-yl group, imidazol-4-yl group, 1H-1,2,3-triazol-1-yl group, 1H-1,2,3-triazol-4-yl group, 1H-1,2,4-triazol-1-yl group, 1H-1,2,4-triazol-3-yl group, 1H-1,2,4-triazol-5-yl group, tetrazol-1-yl group, tetrazol-5-yl group, oxazol-2-yl group, oxazol-4-yl group, oxazol-5-yl group, thiazol-2-yl group, thiazol-4-yl group, thiazol-5-yl group, naphthalen-1-yl group, naphthalen-2-yl group, benzofuran-2-yl group, benzofuran-3-yl group, benzofuran-4-yl group, benzofuran-5-yl group, benzofuran-6-yl group, benzofuran-7-yl group, benzo[b]thiophen-2-yl group, benzo[b]thiophen-3-yl group, benzo[b]thiophen-4-yl group, benzo[b]thiophen-5-yl group, benzo[b]thiophen-6-yl group, benzo[b]thiophen-7-yl group, indol-1-yl group, indol-2-yl group, indol-3-yl group, indol-4-yl group, indol-5-yl group, indol-6-yl group, indol-7-yl group, benzimidazol-1-yl group, benzimidazol-2-yl group, benzimidazol-4-yl group, benzimidazol-5-yl group, benzotriazol-1-yl group, benzotriazol-4-yl group, benzotriazol-5-yl group, benzoxazol-2-yl group, benzoxazol-4-yl group, benzoxazol-5-yl group, benzoxazol-6-yl group, benzoxazol-7-yl group, benzothiazol-2-yl group, benzothiazol-4-yl group, benzothiazol-5-yl group, benzothiazol-6-yl group, benzothiazol-7-yl group and the like. The aralkyl group represented by R⁸ means an alkyl group having from 1 to 4 carbon atoms which is substituted with the aforementioned aryl group at any position. Examples include benzyl group, phenethyl group, phenylpropyl group, phenylbutyl group, triphenylmethyl group, (pyridin-2-yl)methyl group, (pyrazin-2-yl)methyl group, (pyrimidin-2-yl)methyl group, (furan-2-yl)methyl group, (thiophen-2-yl)methyl group, (pyrrol-1-yl)methyl group, (pyrazol-1-yl)methyl group, (imidazol-1-yl)methyl group, (1H-1,2,3-triazol-1-yl)methyl group, (1H-1,2,4-triazol-1-yl)methyl group, (tetrazol-5-yl)methyl group, (oxazol-2-yl)methyl group, (thiazol-2-yl)methyl group, (naphthalen-1-yl)methyl group, (benzofuran-2-yl)methyl group, (benzo[b]thiophen-2-yl)methyl group, (indol-1-yl)methyl group, (benzimidazol-1-yl)methyl group, (benzotriazol-1-yl)methyl group, (benzoxazol-2-yl)methyl group, (benzothiazol-2-yl)methyl group and the like.

In the present specification, a substituting/binding position of "cycloalkyloxy group which has a heteroatom as a ring constituting atom", "saturated heterocyclic group", "condensed hydrocarbon ring formed together with the benzene ring", "aryl group" and "aralkyl group" may be at any position on a substitutable/bindable atom among ring-consituting elements, otherwise a substituting/binding position is specifically limited as some examples given above.

In the present specification, when certain functional groups are defined as "which may be substituted," the number and kind of substituents are not particularly limited. When two or more substituents exist, they may be the same or different. Such substituents include, for example, alkyl groups, cycloalkyl groups, hydroxyl group, mercapto group, alkoxyl groups, alkylthio groups, halogen atoms, amino group, alkylamino groups, dialkylamino groups, cyano group, cyanoalkyl groups, nitro group, formyl group, alkoxycarbonyl groups, alkoxyalkyl groups, alkoxycarbonylalkyl groups, carboxyalkyl groups, hydroxyalkanoyl groups, alkoxyalkoxyl groups, alkoxyalkanoyl groups, benzyloxycarbonyl group, benzyloxyalkanoyl groups, alkylaminoalkoxyl groups, dialkylaminoalkoxyl groups, alkylaminoalkyl groups, dialkylaminoalkyl groups, halogenoalkyl groups, oxo group, hydroxyimino group, alkoxyimino groups, aryloxyimino groups, carboxyl group, alkanoyl groups, alkanoylalkyl groups, carbamoyl group, aryl groups, aralkyl groups, phthalimido group, phthalimidoalkyl groups, alkylsulfonylamino groups, alkylcarbonylamino groups, alkylthiocarbonyl groups, alkenylthiocarbonyl groups, alkoxythiocarbonyl groups, alkoxythiocarbonylalkyl groups, thiocarbamoyl group, N-alkylthiocarbamoyl groups, N,N-dialkylthiocarbamoyl groups, azetidinylthiocarbonyl group, pyrrolidinylthiocarbonyl group, piperazinylthiocarbonyl group, morpholinylthiocarbonyl group, thiomorpholinylthiocarbonyl group, alkylthiothiocarbonyl groups, arylthiocarbonyl groups, aralkylthiocarbonyl groups, alkylthiocarbonylamino groups, alkylthiocarbonylaminoalkyl groups, alkoxythiocarbonylamino groups, alkoxythiocarbonylaminoalkyl groups, N-alkylthiocarbamoylamino groups, N,N-dialkylthiocarbamoylamino groups, alkylthiothiocarbonylamino groups, alkylthiothiocarbonylaminoalkyl groups, arylthiocarbonylamino groups, aralkylthiocarbonylamino groups, thiocarbamoylalkyl groups, N-alkylthiocarbamoylalkyl groups, N,N-dialkylcarbamoylalkyl groups, alkanoylaminoalkyl groups, alkoxythiocarbonylaminoalkyl groups, alkylsulfonylaminoalkyl groups, alkanoylalkylthiocarbonyl groups, alkylthiocarbonylalkylthiocarbonyl groups, thiocarbamoylaminoalkyl groups, N-alkylthiocarbamoylaminoalkyl groups, N,N-dialkylthiocarbamoylaminoalkyl groups, alkanoylalkylaminothiocarbonyl groups, alkylthiocarbonylalkylaminothiocarbonyl groups, alkylthiocarbonylalkyl groups, alkylthiothiocarbonylalkyl groups, alkoxythiocarbonylalkyl groups, thiocarbamoylamino group, cycloalkylthiocarbonyl groups, cycloalkylthiocarbonylamino groups, alkynylthiocarbonyl groups, alkynylthiocarbonylamino groups, thiocarbamoylalkylaminothiocarbonyl groups, N-alkylthiocarbamoylalkylaminothiocarbonyl groups, N,N-dialkylthiocarbamoylalkylaminothiocarbonyl groups and the like.

The thiocarbamic acid derivatives of the present invention have one asymmetric carbon atom in the oxazolidine ring, and may further have one or more asymmetric carbons depending on type of a substituent. The asymmetric carbons existing in the compounds of the present invention may be independently in the (R)- or (S)-configuration, and stereoisomers such as optical isomers and diastereoisomers may exist on the basis of one or more asymmetric carbons. Any stereoisomers in pure forms, any mixure of the stereoisomers, racemates and the like fall within the scope of the present invention.

The thiocarbamic acid derivatives of the present invention can be converted into salts, preferably pharmacologically acceptable salts, if desired; and the salts generated can also be converted into compounds in free forms. The salts of the compounds of the present invention are preferably the pharmacologically acceptable salts. As the acid-additive salts, there can be used, for example, salts with mineral acids such as hydrochloric acid, hydrobromic acid, nitric acid, sulfuric acid, hydroiodic acid, and phosphoric acid; and salts with organic acids such as acetic acid, propionic acid, butyric acid, formic acid, valeric acid, maleic acid, fumaric acid, citric acid, oxalic acid, malic acid, methanesulfonic acid, ethanesulfonic acid, benzenesulfonic acid, trifluoroacetic acid, benzoic acid, p-toluenesulfonic acid, mandelic acid, 10-camphorsulfonic acid, tartaric acid, lactic acid, stearic acid, nicotinic acid, gluconic acid, 5-oxotetrahydrofuran-2-carboxylic acid, and 2-hydroxyglutaric acid. As the alkali-additive salts, there can be used, for example, inorganic alkali salts such as sodium salt, potassium salt, calcium salt, magnesium salt, and ammonium salt; and salts with organic bases such as ethanolamine, N,N-dialkylethanolamine, triethanolamine, piperidine, piperazine, morpholine, and thiomorpholine.

The thiocarbamic acid derivatives of the present invention or the salts thereof may exist as any crystal form depending on manufacturing conditions, or may exist as any hydrate or solvate. These crystal forms, hydrates, and solvates, and mixtures thereof fall within the scope of the present invention.

Preferred compounds of the present invention include the following compounds; however, the present invention is not limited to these examples. The abbreviations in the tables have the following meanings: Me: methyl group; Et: ethyl group; n-Pr: n-propyl group; i-Pr: isopropyl group; n-Bu: n-butyl group; i-Bu: isobutyl group; tert-Bu: tert-butyl group; n-Pent: n-pentyl group; n-Hex: n-hexyl group; Ph: phenyl group; Bn: benzyl group; Ms: mesyl group; and, Boc: tert-butoxycarbonyl group.

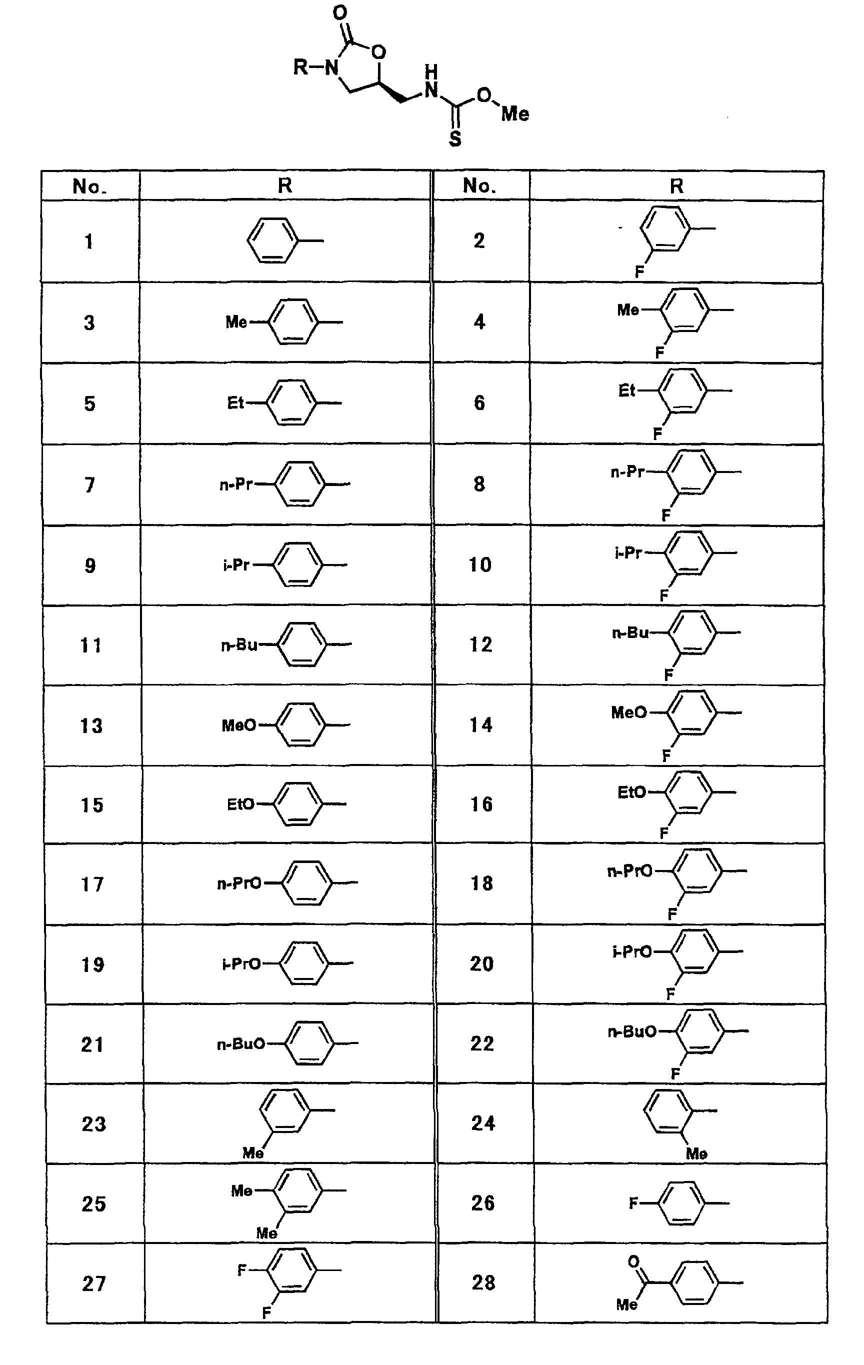

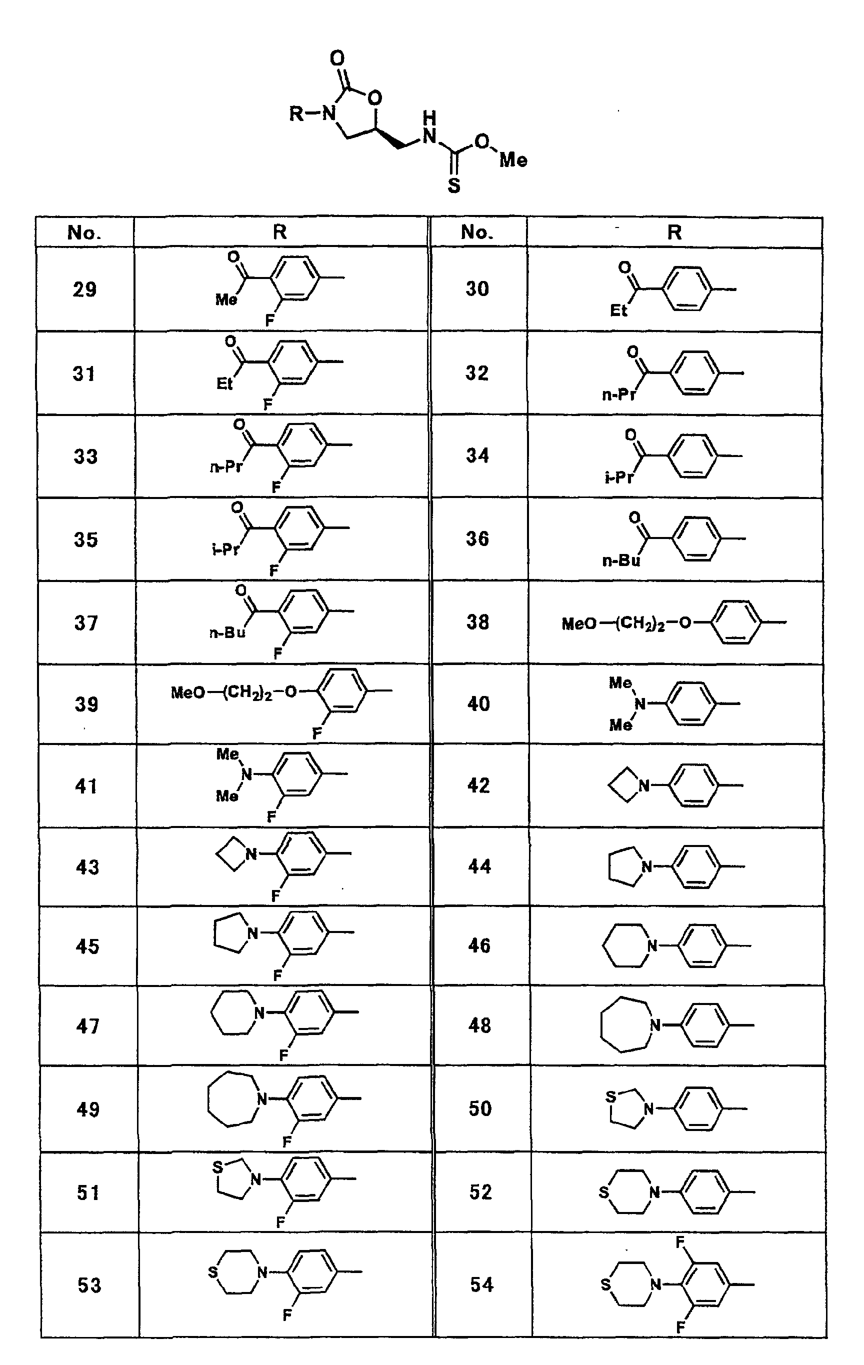

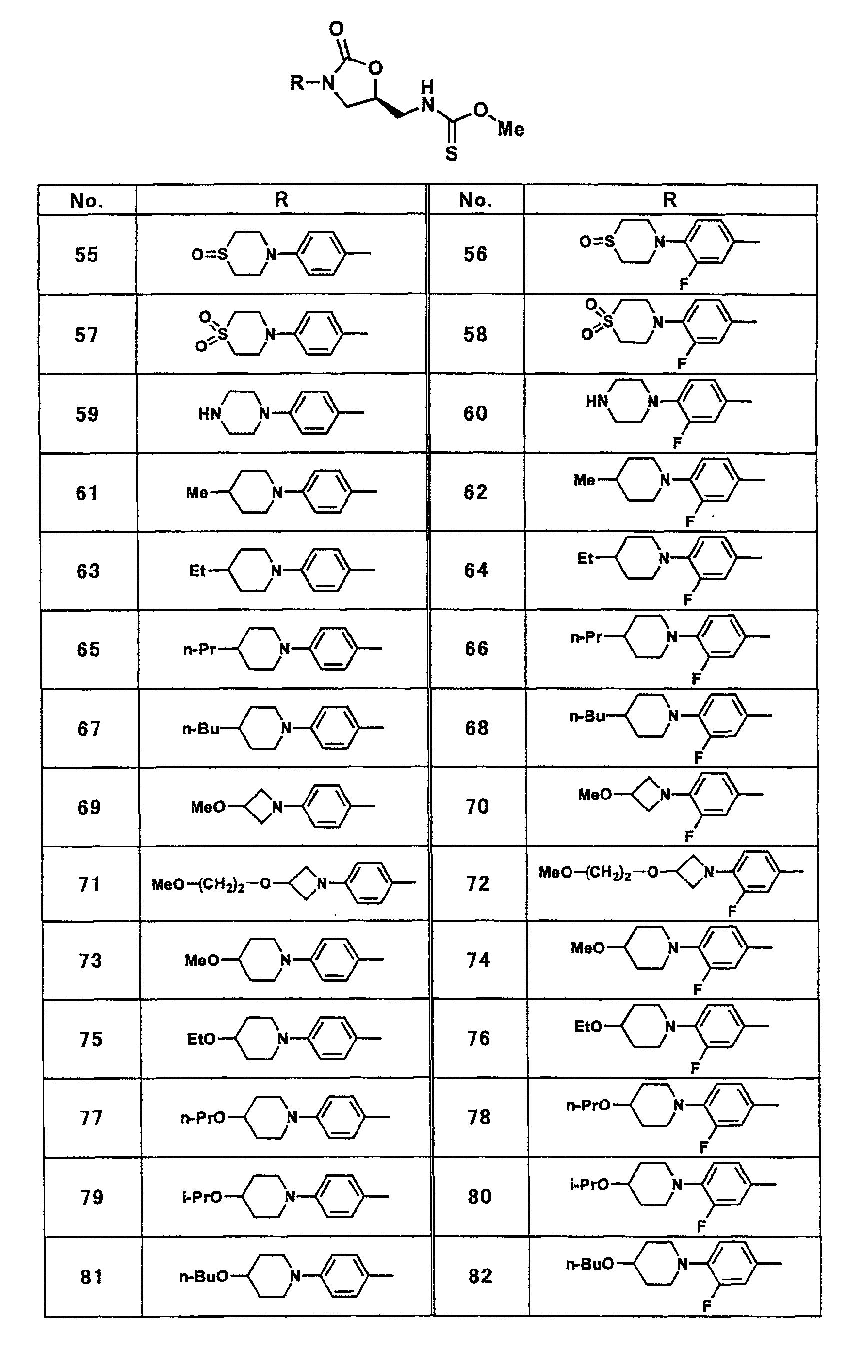

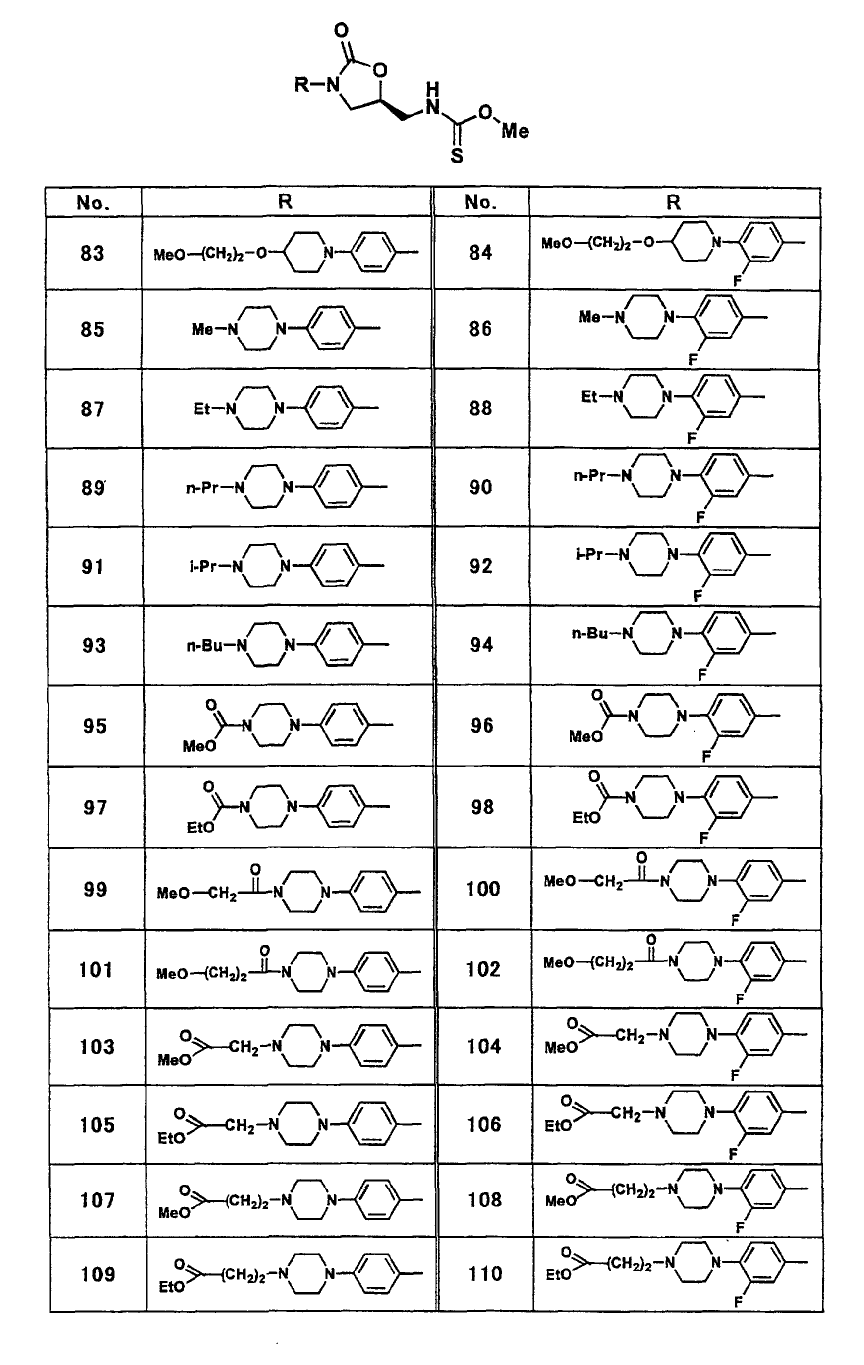

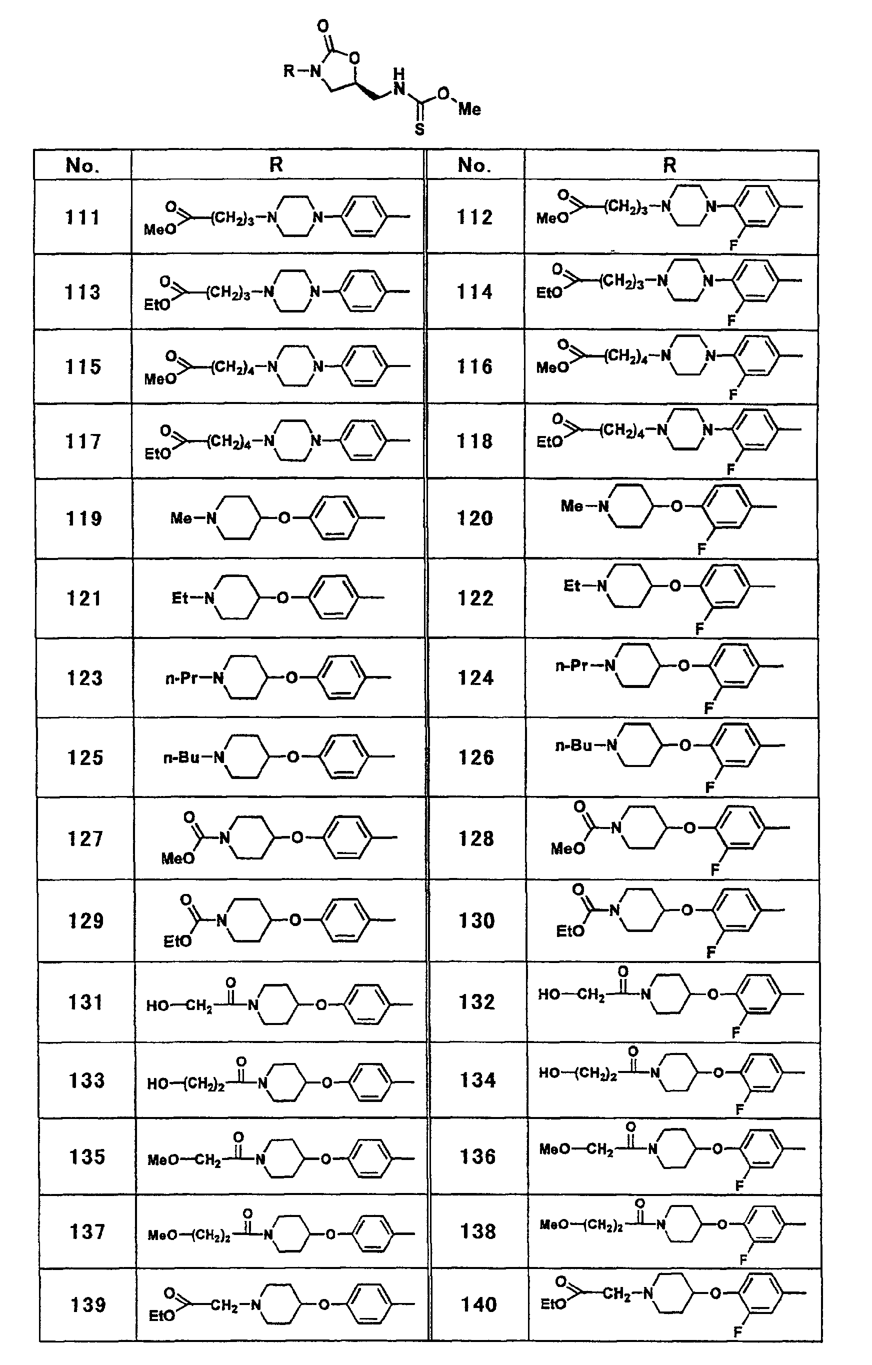

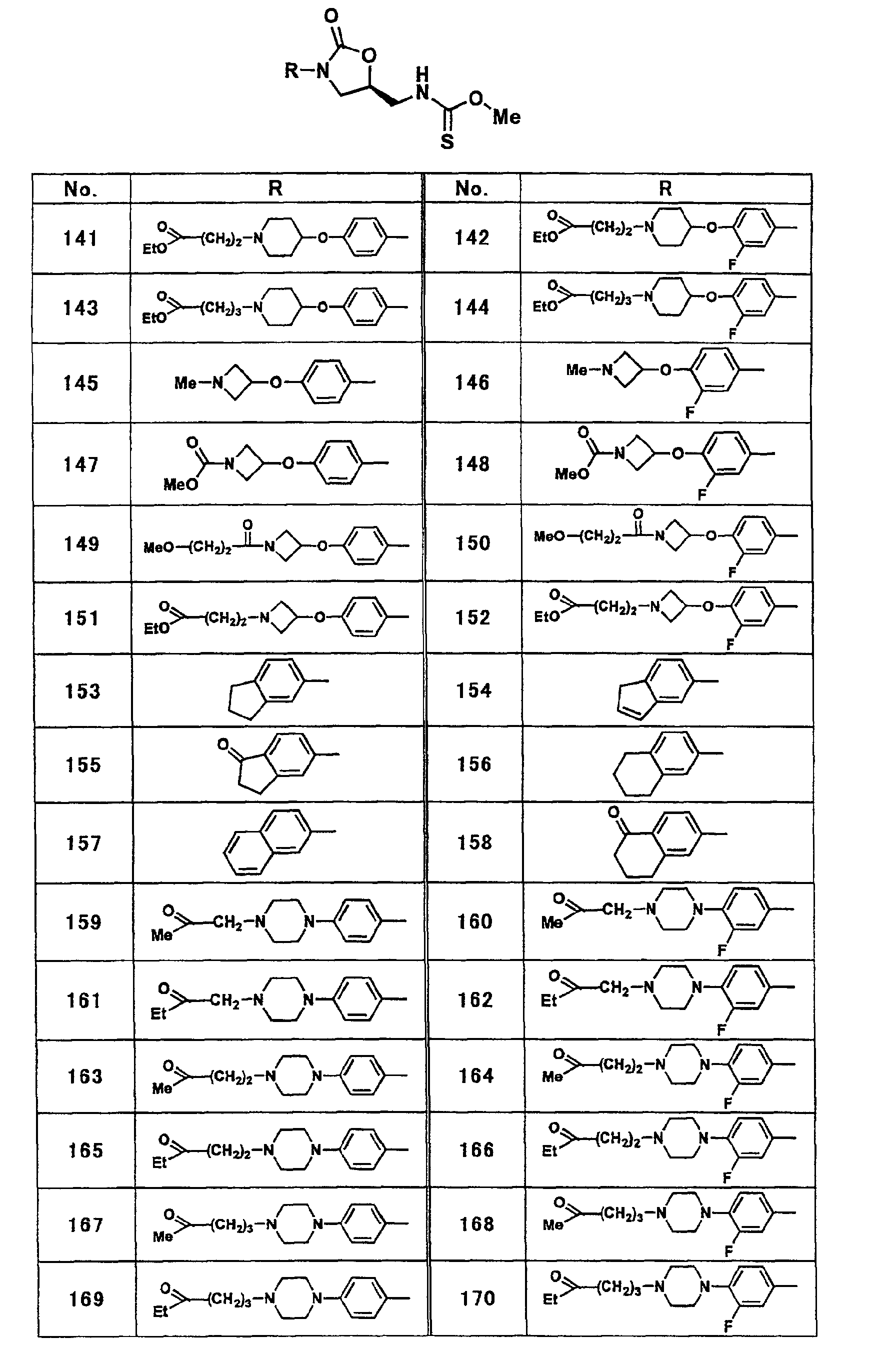

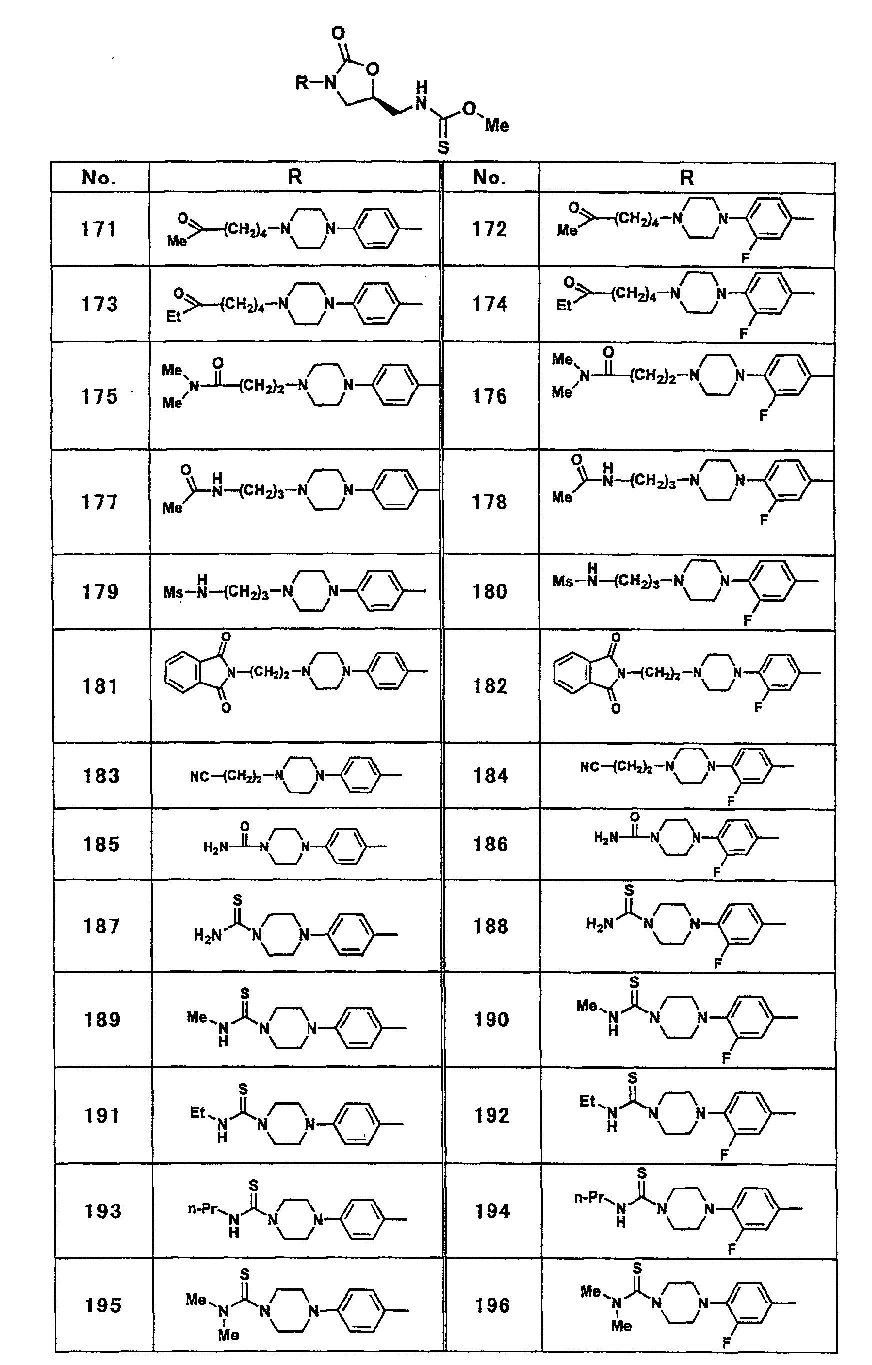

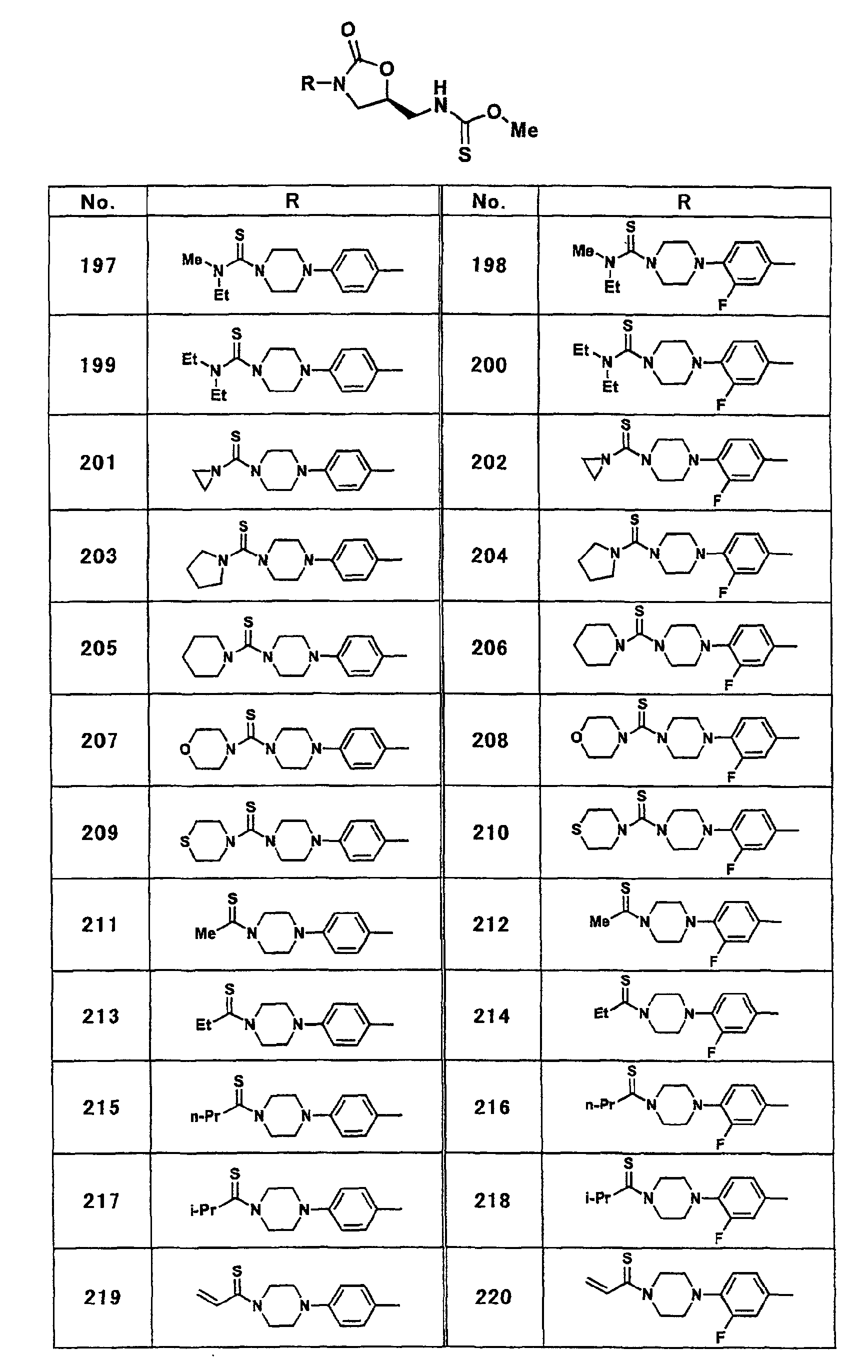

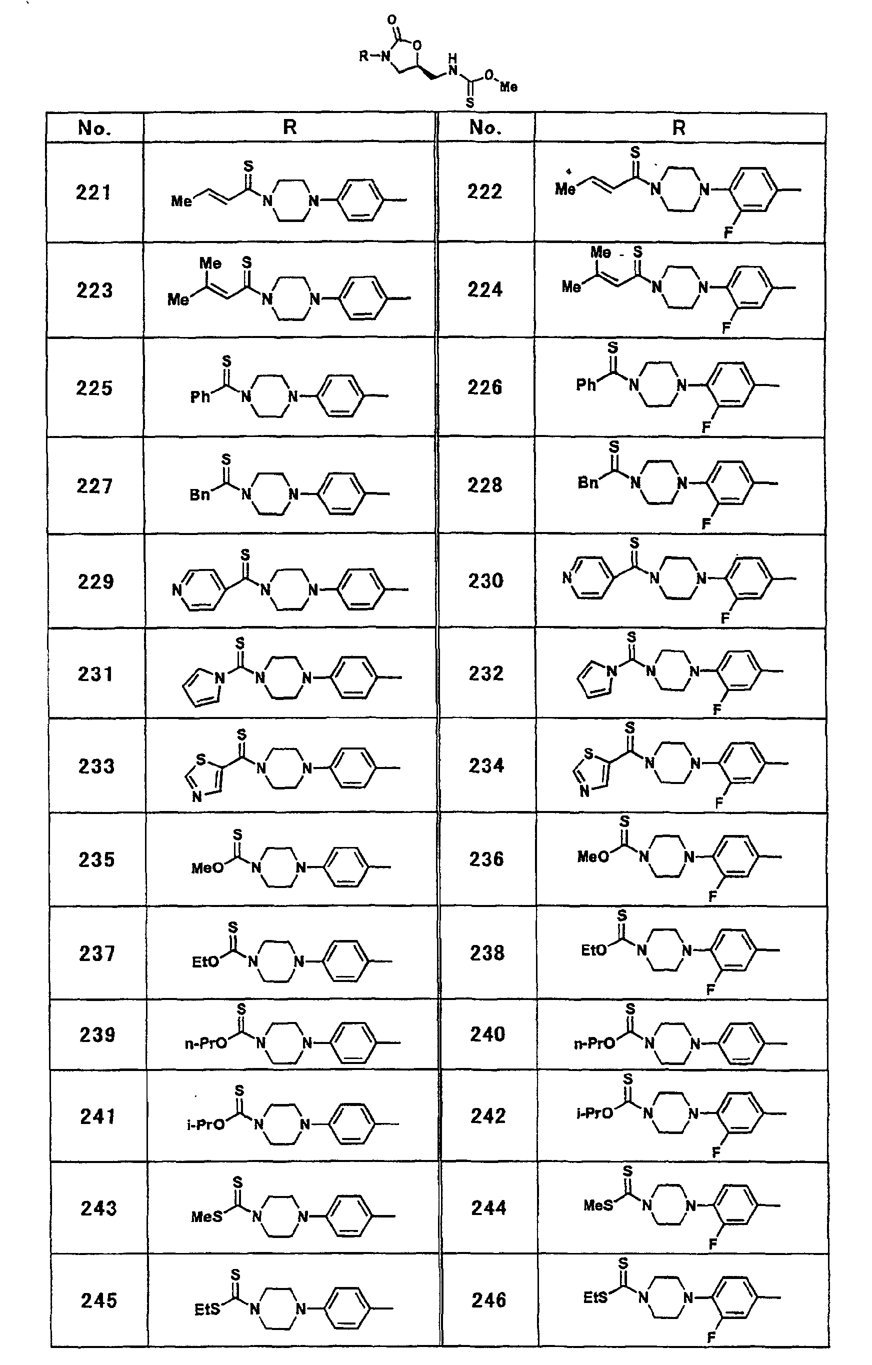

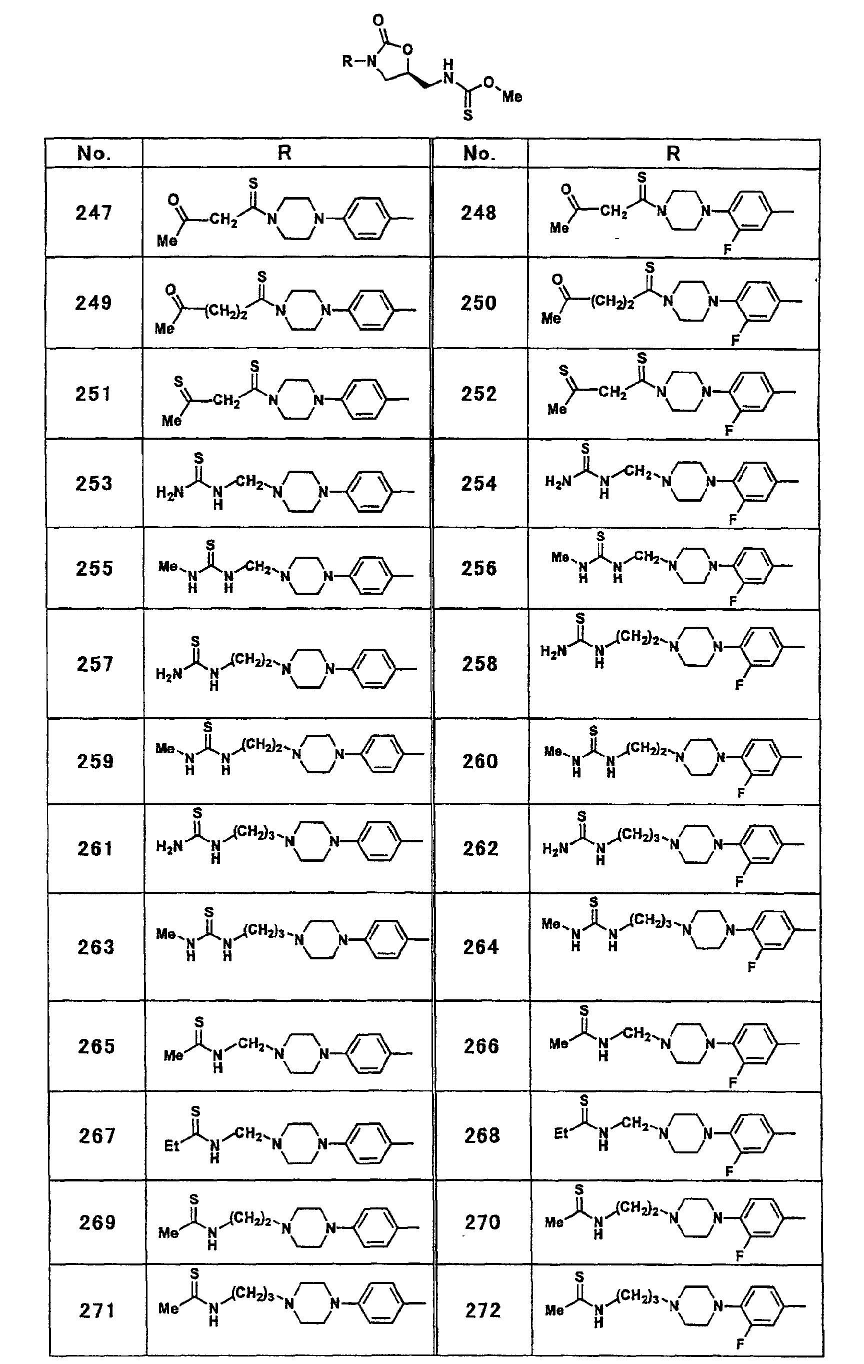

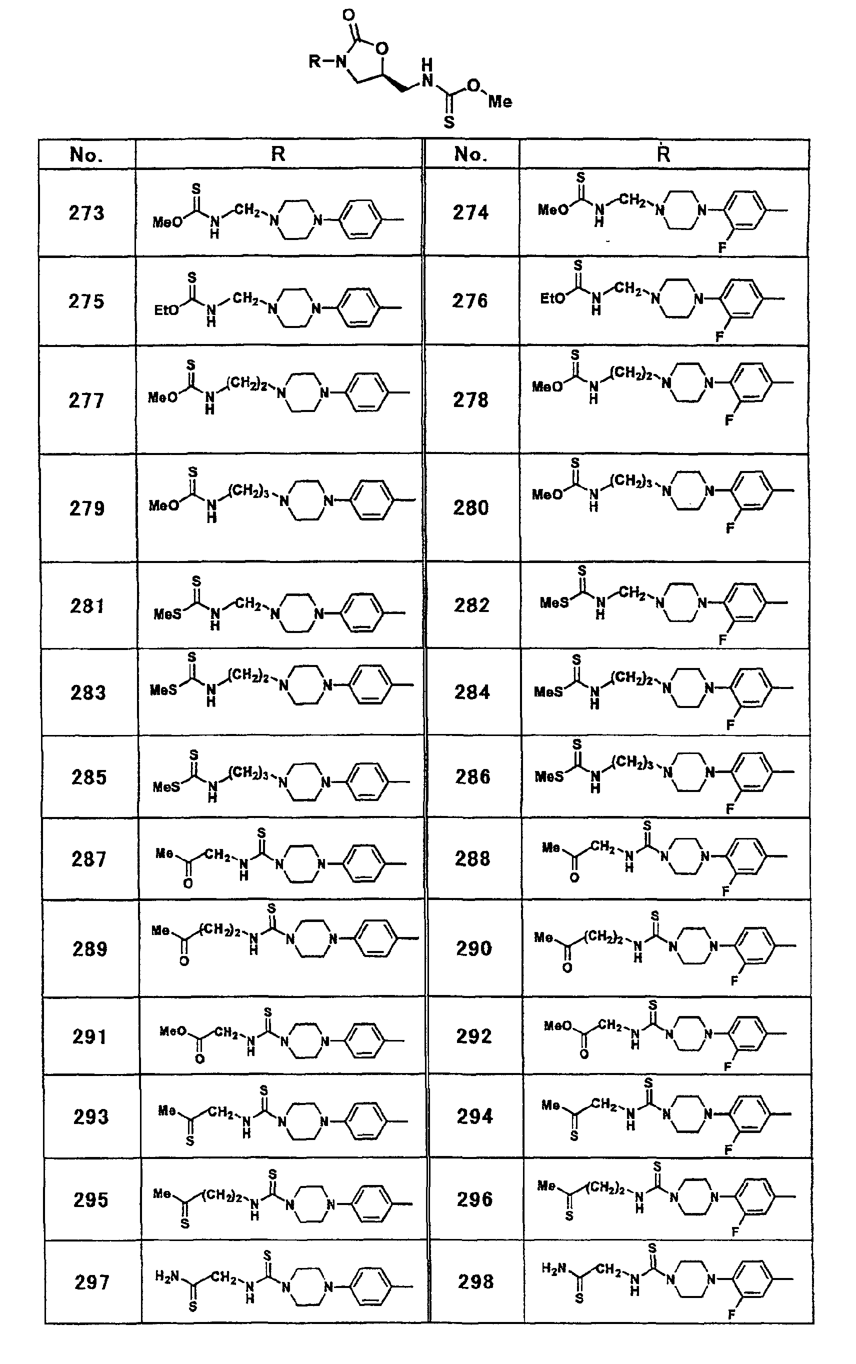

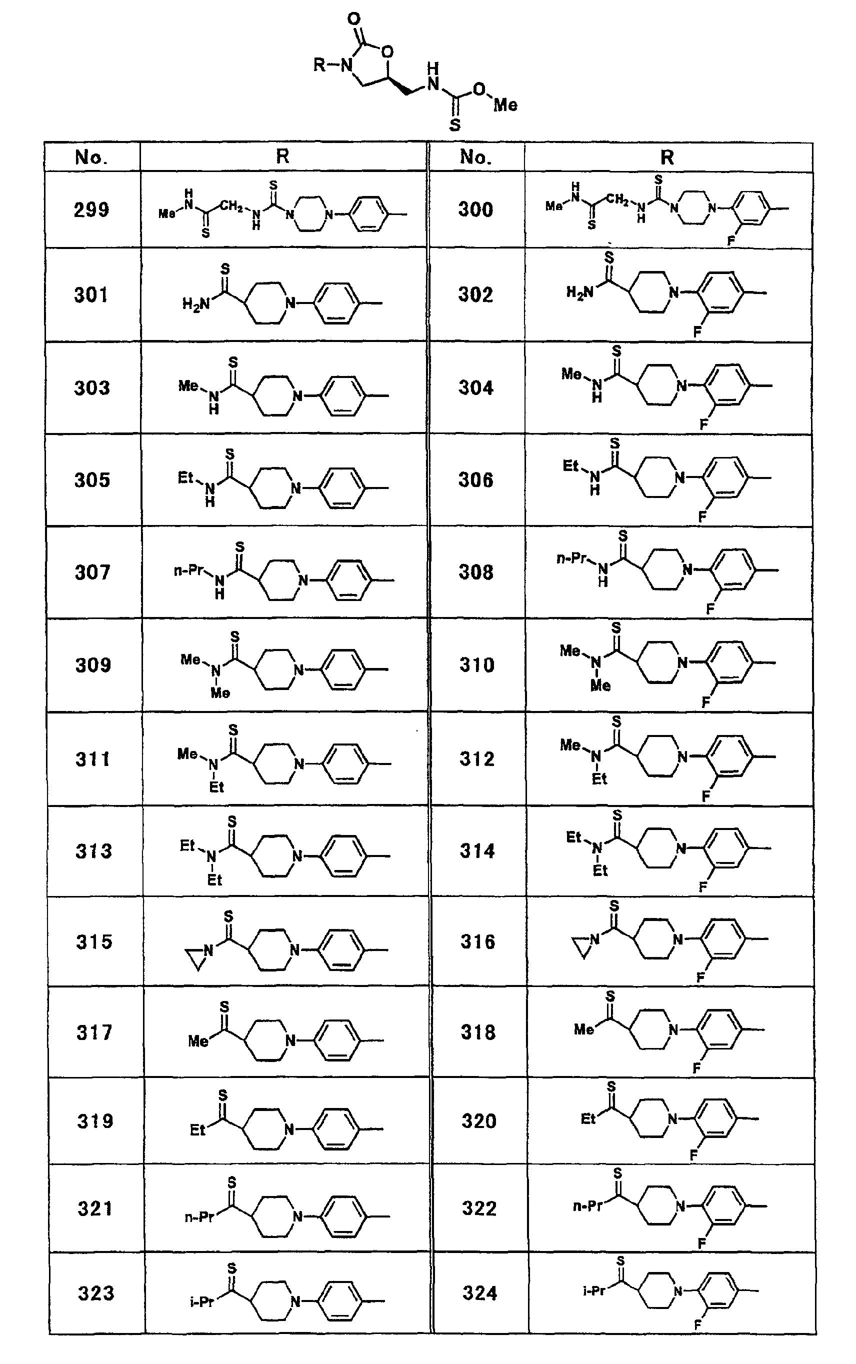

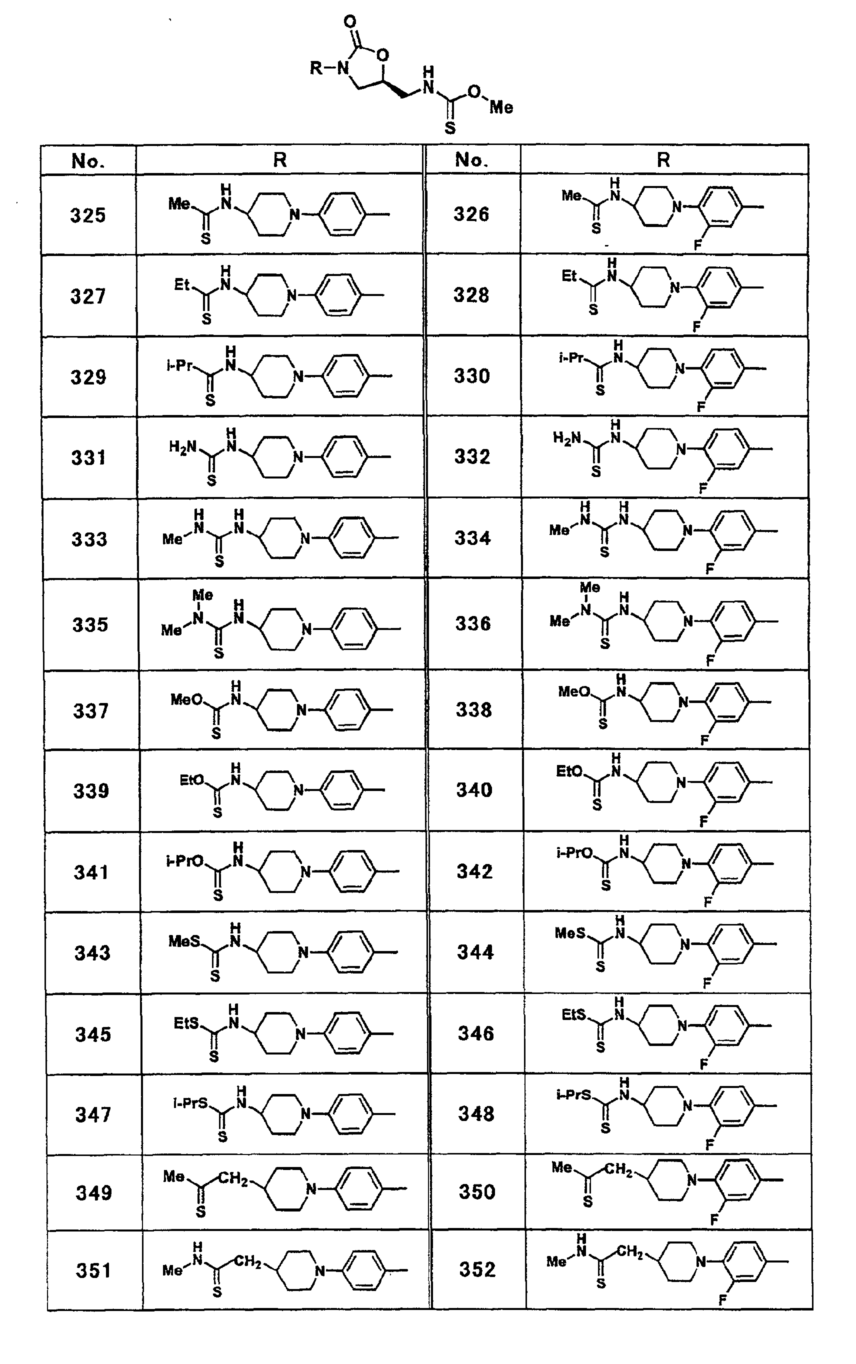

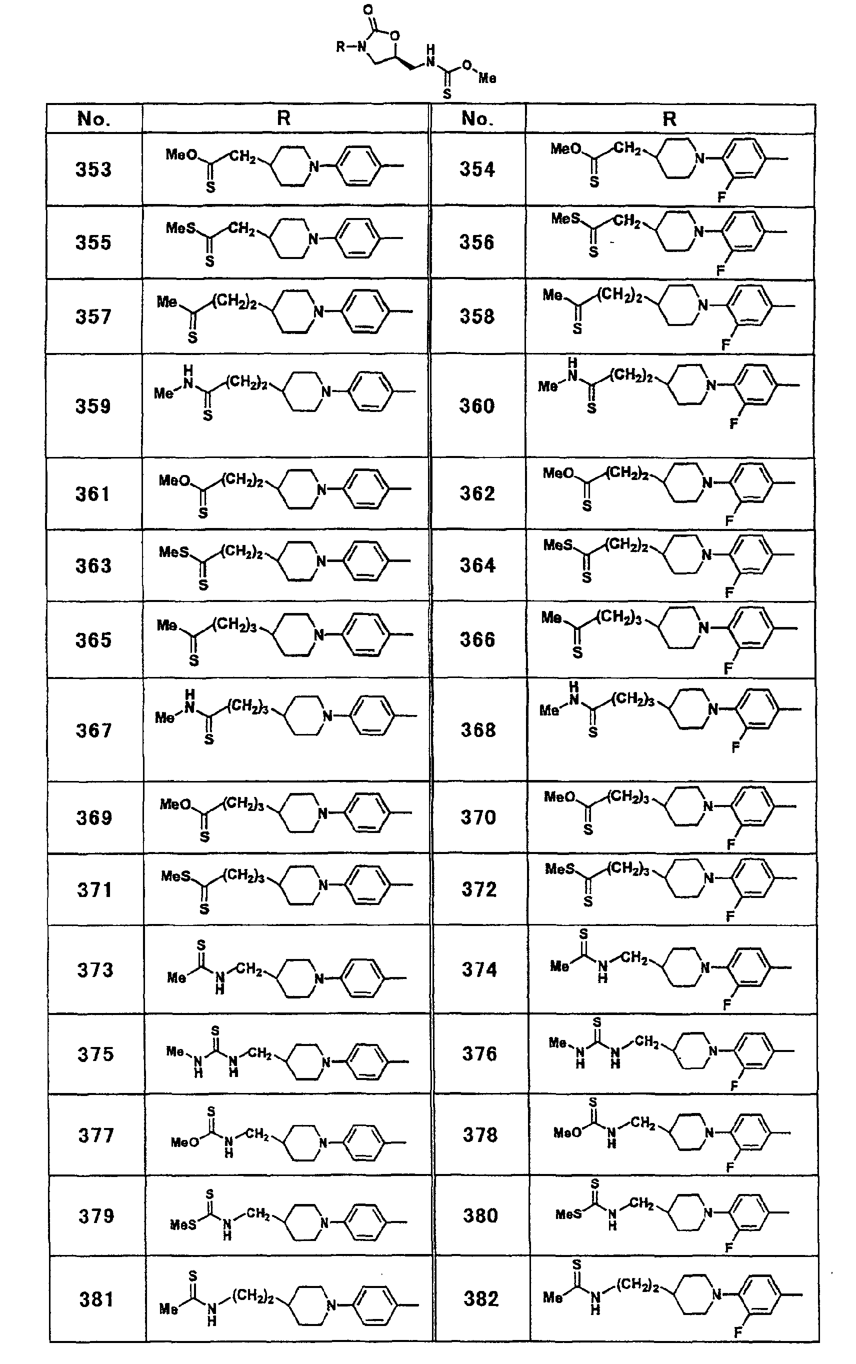

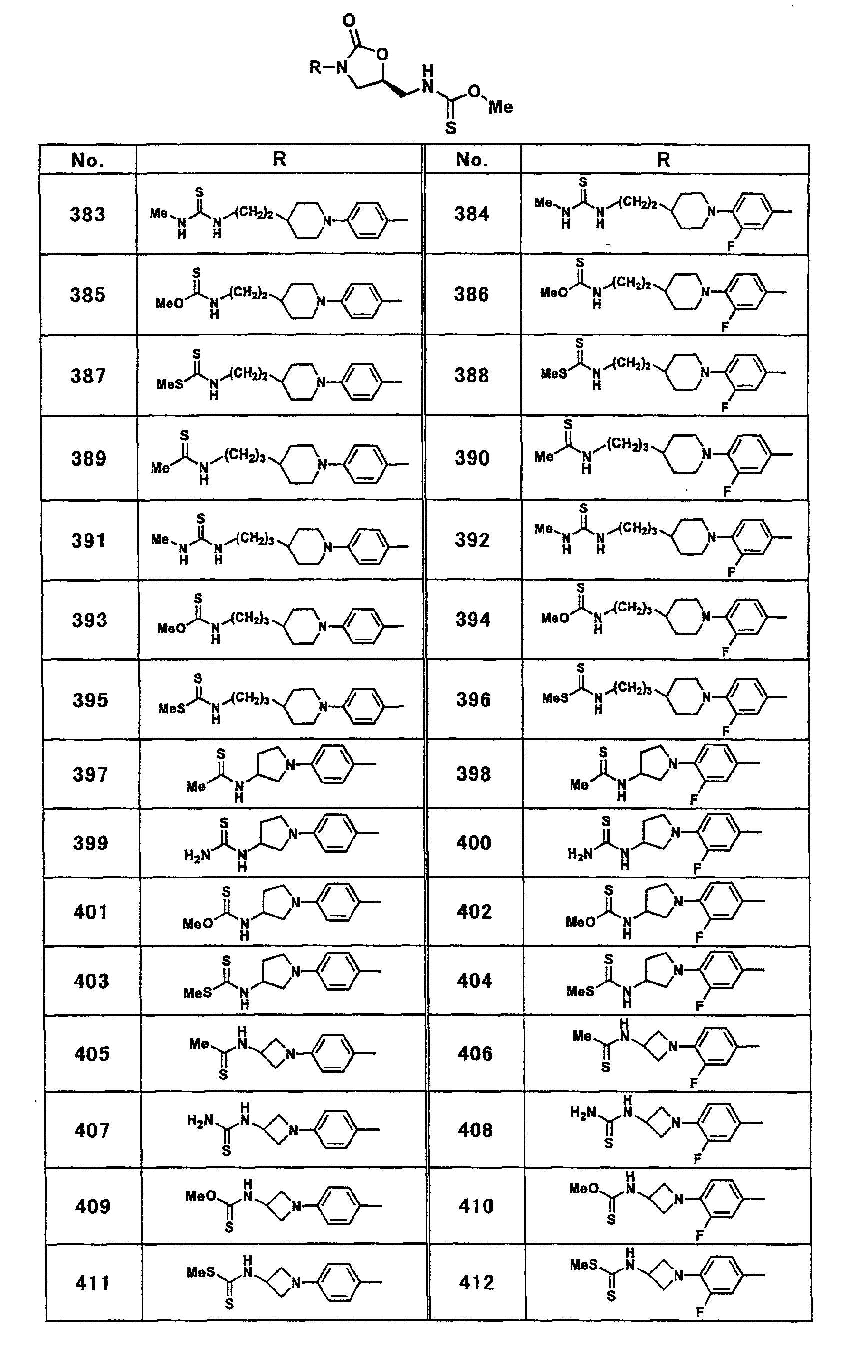

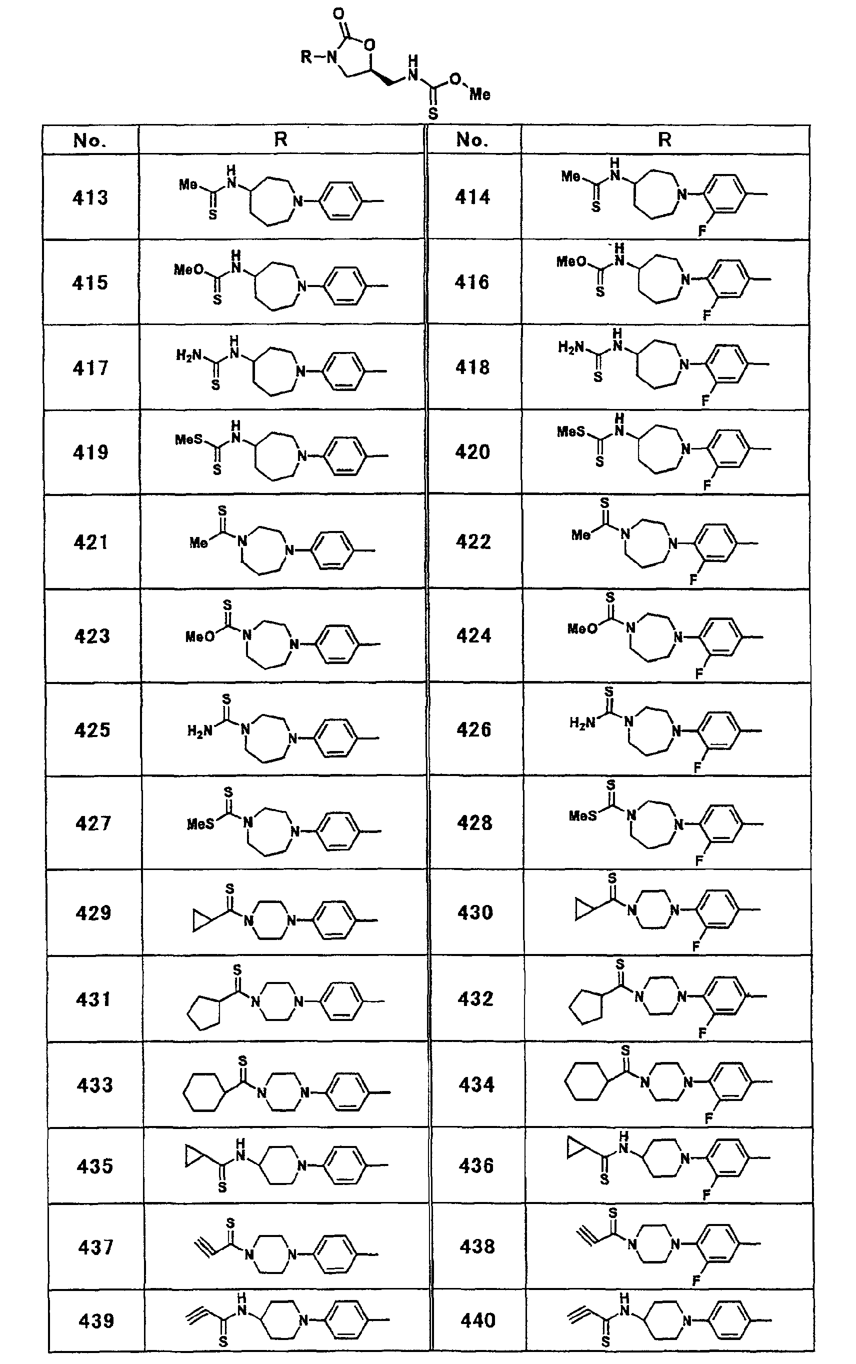

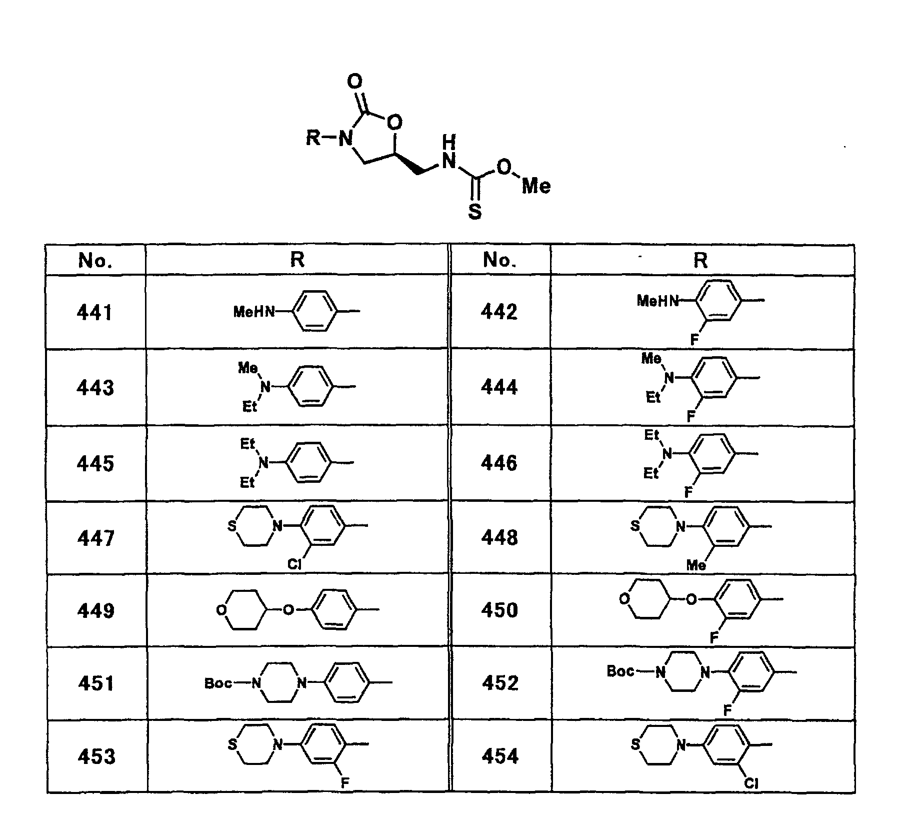

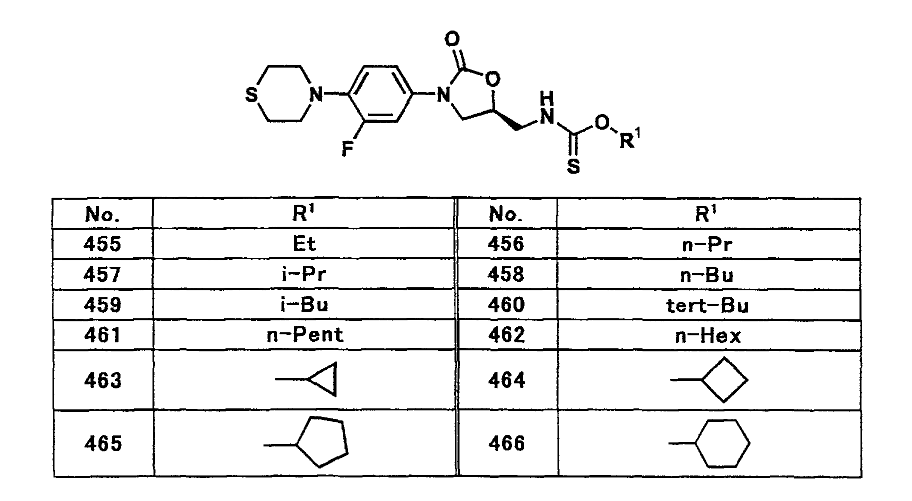

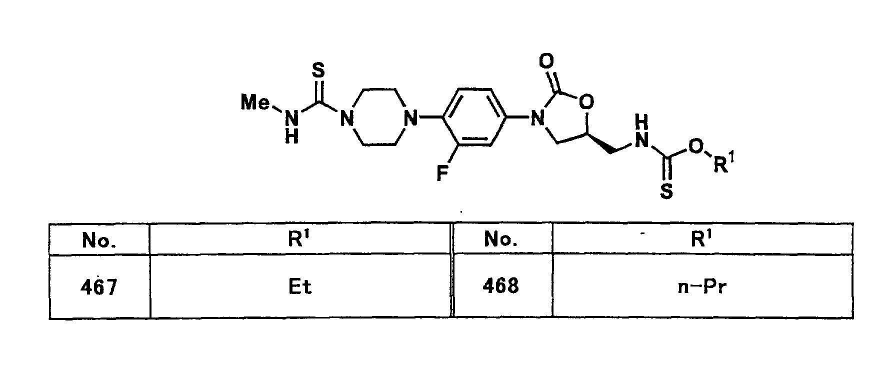

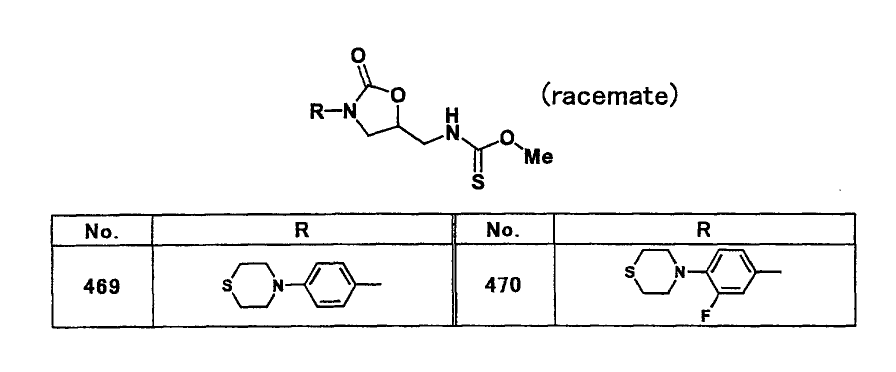

The thiocarbamic acid derivatives of the present invention which are represented by the aforementioned general formulas (I) to (IV) can be prepared, for example, by the following method. However, the preparation methods of the aforementioned compounds are not limited thereto. In the following preparation methods, specific explanations will be given as for the compounds represented by the aforementioned general formula (I), and it is obvious that these preparation methods include the compounds represented by the aforementioned general formulas (II) to (IV). In addition, specific and detailed explanations will be given in the examples of the present specification as for preparations of typical compounds of the thiocarbamic acid derivatives of the present invention. Accordingly, persons skilled in the art can readily prepare the compounds of the present invention which are encompassed within the aforementioned general formula (I) by referring to the following general explanation and specific explanation in the examples, appropriately choosing starting materials, reagents, and reaction conditions, and if necessary, suitably modifying or altering these methods.

The first preparation method of the compounds of the present invention include a method which involves the use of a compound represented by the general formula (V) as a starting material and synthesis of the thiocarbamic acid derivative represented by the general formula (I) via a novel compound represented by the general formula (VI): 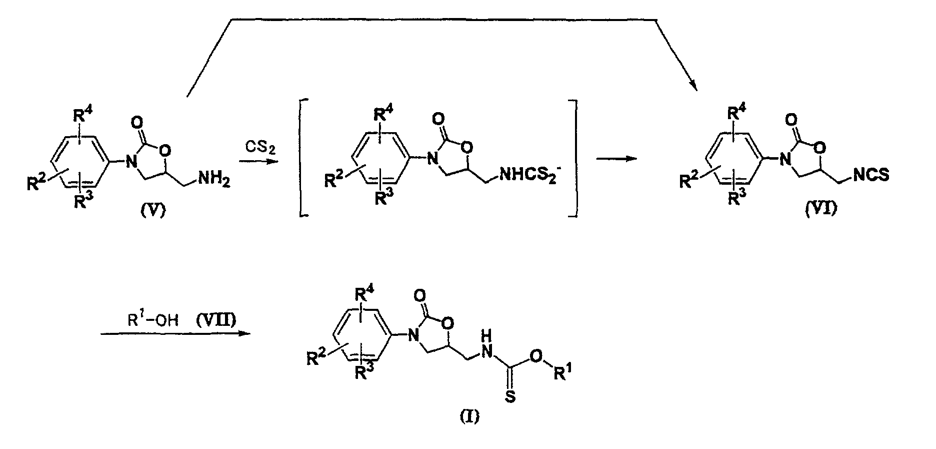 wherein R¹, R², R³ and R⁴ have the same meanings as those defined above.

In this method, the compound represented by the general formula (VI) can be prepared by reacting the compound represented by the general formula (V) with carbon disulfide in the presence of a base such as triethylamine in a solvent such as tetrahydrofuran, and reacting the resulting dithiocarbamate with ethyl chlorocarbonate, copper sulfate, iron nitrate, iron sulfate, zinc sulfide or other reagent at a temperature ranging from ice-cooling temperature to 200°C. As other preparation methods, the compound represented by the general formula (VI) can directly be synthesized by a preparation method which comprises the step of reacting the compound represented by the general formula (V) with thiophosgene in the presence of a base such as triethylamine in a solvent such as tetrahydrofuran, and by a method disclosed in Organic Synthesis Collective Volume, Vol. 1, p.447.

Then, the compound represented by the general formula (I) can be prepared by reacting the compound represented by the general formula (VI) with the compound represented by the general formula (VII) in the presence or absence of a base without solvent or in a solvent.

As the solvent used in the aforementioned reaction, any solvents may be used so far that they do not inhibit the reaction. Examples include aprotic polar solvents such as acetone, acetonitrile, N,N-dimethylformamide, N-methyl-2-pyrrolidone, dimethylsulfoxide, tetramethylene sulfone, tetramethylene sulfoxide, and hexamethylphosphoric triamide; etheric solvents such as diethyl ether, diisopropyl ether, and tetrahydrofuran; ester solvents such as methyl acetate and ethyl acetate; aromatic hydrocarbon solvents such as benzene and toluene; organic base solvents such as pyridine, picoline, lutidine, and collidine; halogenated hydrocarbon solvents such as dichloromethane, 1,2-dichloroethane, and chloroform; and mixtures thereof. Examples of the base include inorganic bases such as lithium, sodium, sodium hydride, potassium, potassium tert-butoxide, potassium carbonate, sodium carbonate, and sodium hydrogencarbonate; and organic bases such as triethylamine and diisopropylethylamine. The reaction is carried out at a temperature ranging from an ice-cooling temperature to 200°C.

The second preparation method of the compounds of the present invention includes a method comprising the step of reacting the compound represented by the general formula (V) with an appropriate O-alkyl chlorothiocarbonate in the presence of a base such as triethylamine in a solvent such as tetrahydrofuran at a temperature ranging from an ice-cooling temperature to a refluxing temperature of a solvent to obtain the compound of the general formula (I).

In the third preparation method of the compounds of the present invention, a compound represented by the general formula (I), wherein any one of R², R³ and R⁴ is a cycloalkyloxy group having a heteroatom as a ring constituting atom or a saturated heterocyclic group and wherein said group has a protected nitrogen atom, may be subjected to deprotection of the nitrogen to prepare the corresponding deprotected compound of the general formula (I).

The deprotection can be carried out by various methods depending on the type of a protecting group for the nitrogen atom. For example, when the protecting group is an amide-type protecting group such as an alkanoyl group and an arylcarbonyl group, the deprotection may be carried out by hydrolysis using an acid or a base to prepared the desired compound. The hydrolysis of the amide may be carried out by a known method. In acidic hydrolysis, an acid such as hydrochloric acid, sulfuric acid, and trifluoroacetic acid may be used, and in basic hydrolysis, a base such as sodium hydroxide and potassium hydroxide may be used. These acids or bases may be used as aqueous solutions. The deprotection may be carried out in an organic solvent including alcoholic solvents such as methanol, ethanol, n-butanol, sec-butanol, and tert-butanol; etheric solvents such as diethyl ether, diisopropyl ether, and tetrahydrofuran; and ester solvents such as methyl acetate and ethyl acetate, or in a water-containing organic solvent at a temperature ranging from room temperature to a refluxing temperature of a solvent. When the protecting group is an urethane-type protecting group such as a lower alkoxycarbonyl group, the deprotection can be carried out by treatment with an acid such as hydrochloric acid, hydrobromic acid, and trifluoroacetic acid without a solvent or in a solvent such as acetic acid, ethyl acetate, 1,4-dioxane, water, methanol, ethanol, and a mixture thereof at a temperature ranging from an ice-cooling temperature to 200°C to prepare the desired compound.

In the forth preparation method of the present invention, the compound of the general formula (I) obtained in the third preparation method wherein any one of R², R³ and R⁴ is a cycloalkyloxy group having a heteroatom as a ring constituting atom or a saturated heterocyclic group and wherein said group has a deprotected nitrogen atom can be subjected to appropriate alkylation, acylation, urethane-introduction, urea-introduction, thioacylation, thiourea-introduction, thiocarbamation or other reaction; or the compound can be subjected to an appropriate reaction such as acylation and urethane-introduction, and then the resulting carbonyl group can be converted into a thiocarbonyl group by using the Lawesson's reagent or other reagent, to obtain the corresponding compound of general formula (I) wherein the nitrogen atom is substituted.

The introduction of a substituent into the nitrogen atom can be carried out by various methods depending upon the kind of the substituent. For example, for alkylation, alkylation using an alkyl halide, alkyl sulfonate, or other reagent, or the Michael addition using an acrylic acid ester or other reagent can be carried out in the presence or absence of a base without a solvent or in a solvent to obtain the corresponding compound of the general formula (I). Alternatively, acylation or urethane-introduction using an acyl halide and the like; urea-introduction using sodium cyanate and the like; thioacylation using a thioacyl halide and the like; thiourea-introduction using an alkylisothiocyanate and the like; or thiocarbamation using an O-alkyl chlorothiocarbonate and the like can be carried out in the presence of a base without a solvent or in a solvent, and if necessary, conversion of the carbonyl group of the compound having a nitrogen atom substituted with an acyl group or the like into thiocarbonyl group can be carried out using the Lawesson's reagent without a solvent or in a solvent to obtain the corresponding compound of the general formula (I).

As for the solvents used in these reactions, any solvents may be used so far that they do not inhibit the reactions. Examples include water; alcoholic solvents such as methanol and ethanol; aprotic polar solvents such as acetone, acetonitrile, N,N-dimethylformamide, N-methyl-2-pyrrolidone, dimethylsulfoxide, tetramethylene sulfone, tetramethylene sulfoxide, and hexamethylphosphoric triamide; etheric solvents such as diethyl ether, diisopropyl ether, and tetrahydrofuran; ester solvents such as methyl acetate and ethyl acetate; aromatic hydrocarbon solvents such as benzene and toluene; organic acid solvents such as acetic acid; organic base solvents such as pyridine, picoline, lutidine, and collidine; halogenated hydrocarbon solvents such as dichloromethane, 1,2-dichloroethane, and chloroform; and mixtures thereof. The base includes, for example, inorganic bases such as lithium, sodium, potassium, potassium tert-butoxide, potassium carbonate, sodium carbonate, and sodium hydrogencarbonate; and organic bases such as triethylamine and diisopropylethylamine. The reaction is carried out at a temperature ranging from an ice-cooling temperature to 200°C.

Some of the compounds represented by the general formulas (V) which are used as starting materials for the preparation methods of the compounds of the present invention are known compounds, which are disclosed in Japanese Patent Unexamined Publication No.(Hei) 8-73455, Journal of Medicinal Chemistry, Vol. 39, p. 673 and 680 (1996) and the like, and can be prepared according to the method described therein. Several novel compounds can be prepared, for example, by the following method, and the details of the preparation will be described in reference examples: 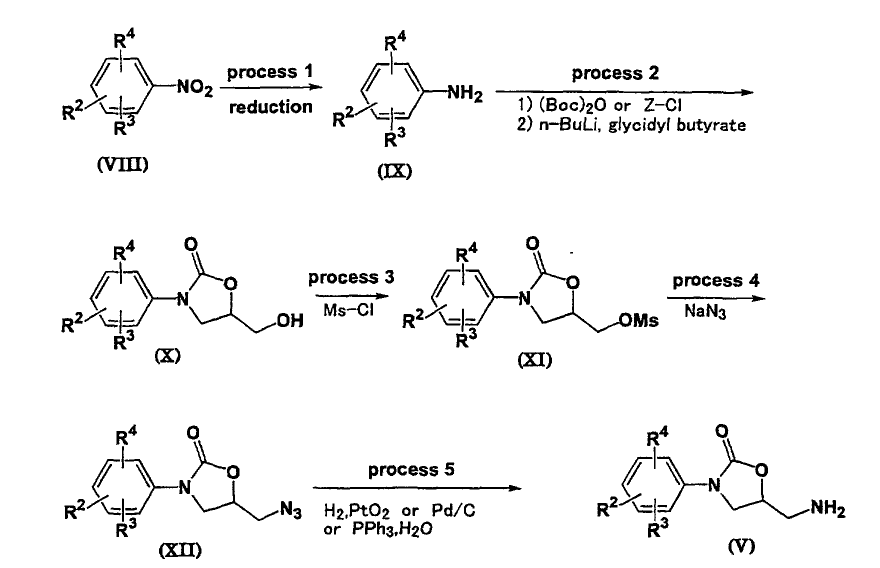 wherein Boc represents tert-butoxycarbonyl group; Z represents benzyloxycarbonyl group; n-Bu represents n-butyl group; Ms represents mesyl group; Ph represents phenyl group; and R², R³ and R⁴ have the same meanings as those defined above.

In process 1, the compound of the general formula (IX) can be obtained by reducing the nitro group of the compound of the general formula (VIII) by an appropriate reducing method, for example, a hydrogenation reduction which is carried out by using a catalyst such as platinum oxide, Raney nickel, and palladium-carbon in a solvent such as ethyl acetate and methanol at a temperature ranging from room temperature to 50°C under hydrogen pressure ranging from normal pressure to 50 atm; a reduction which is carried out by using iron powder and hydrochloric acid, acetic acid or another reagent; or other reducing method.

In process 2, the compound of the general formula (X) can be obtained by subjecting the amino group of the compound of the general formula (IX) to appropriate urethane-introduction, for example, using di-tert-butyl dicarbonate in an appropriate organic solvent such as methanol and tetrahydrofuran at a temperature ranging from an ice-cooling temperature to a refluxing temperature of a solvent; or using benzyloxycarbonyl chloride in the presence of a base such as triethylamine, potassium carbonate, sodium carbonate, and sodium hydrogencarbonate in a solvent such as water; an organic solvent including acetone, methanol, and tetrahydrofuran; and a mixed solvent thereof at a temperature ranging from an ice-cooling temperature to a refluxing temperature of a solvent; then treating the resulting compound with a base such as n-butyllithium in an appropriate aprotic organic solvent such as tetrahydrofuran and N,N-dimethylformamide at a temperature ranging from -78°C to room temperature, and then reacting the resulting compound with glycidyl butylate.

In process 3, the compound of the general formula (XI) can be obtained by reacting the compound of the general formula (X) with methanesulfonyl chloride in the presence of a base such as triethylamine in an appropriate organic solvent such as dichloromethane and tetrahydrofuran at a temperature ranging from an ice-cooling temperature to a refluxing temperature of a solvent.

In process 4, the compound of the general formula (XII) can be obtained by reacting the compound of the general formula (XI) with sodium azide in an appropriate organic solvent such as tetrahydrofuran and N,N-dimethylformamide at a temperature ranging from an ice-cooling temperature to a refluxing temperature of a solvent.

In the compound of the general formula (XI), when any one of R², R³ and R⁴ has a protected nitrogen atom in the substituent, deprotection may be carried out in accordance with the third preparation method of the compound of the present invention after the azidation in process 4, and then if necessary, appropriate alkylation, acylation, urethane-introduction, thioacylation, thiourea-introduction, thiocarbamation or other reaction may be carried out in accordance with the forth preparation method to obtain the respective corresponding compounds of the general formula (XII).

In the compound of the general formula (XI), when any one of R², R³ and R⁴ is thiomorpholinyl group, the sulfur atom may be subjected to appropriate oxidation for conversion into S-oxide or S,S-dioxide, and then the corresponding compound represented by the general formula (XII) can be prepared according to the process 4.

The oxidation of the sulfur atom can be carried out by various methods depending on target compounds. The conversion into S-oxide group can be carried out, for example, by using an oxidizing agent such as chromic acid, hydrogen peroxide, metachloroperbenzoic acid, sodium metaperiodate, and potassium metaperiodate in a solvent such as water, an organic solvent including tetrahydrofuran, methanol, acetonitrile, acetone, and dichloromethane, and a mixed solvent thereof at a temperature ranging from an ice-cooling temperature to a refluxing temperature of a solvent. The conversion into S,S-dioxide group can be carried out, for example, by using an oxidizing agent such as chromic acid, hydrogen peroxide, metachloroperbenzoic acid, osmium tetraoxide, and ruthenium tetraoxide in a solvent such as water, an organic solvent including tetrahydrofuran, methanol, acetone, and dichloromethane, and a mixed solvent thereof at a temperature ranging from an ice-cooling temperature to a refluxing temperature of a solvent.

In process 5, the compound represented by the general formula (V) can be obtained by reducing the azide group of the compound of the general formula (XII) by an appropriate reducing method, for example, a hydrogenation reduction which is carried out by using a catalyst such as platinum oxide and palladium carbon in a solvent such as methanol at a temperature ranging from room temperature to 50°C under hydrogen pressure ranging from normal pressure to 50 atom; a reducing method which is carried out by using triphenylphosphine and water in a solvent such as tetrahydrofuran at a temperature ranging from an ice-cooling temperature to a refluxing temperature of a solvent or other reducing method.

The medicament of the present invention is characterized to comprise the thiocarbamic acid derivative represented by the aforementioned general formulas (I) to (IV) or a salt thereof as an active ingredient. As the active ingredient of the medicament of the present invention, a substance selected from the group consisting of the aforementioned compound in free form and a pharmacologically acceptable salt thereof, and any crystal form thereof, a solvate thereof, and a hydrate thereof can be used, and two or more substances may be used in combination. As the medicament of the present invention, the aforementioned substance may be used *per se*. In general, the medicament is desirably provided in a form of a pharmaceutical composition which comprises the aforementioned substance as an active ingredient and one or more pharmaceutical additives.

The form of the pharmaceutical composition is not particularly limited, and the composition can be prepared, for example, as an oral preparation in the forms of capsules, tablets, fine granules, granules, powders, syrups and the like, or as a parenteral preparation in the forms of injections, suppositories, eye drops, eye ointments, ear drops, nasal drops, transdermal and transmucosal agents, inhalations, dermatologic preparation and the like. These preparations can be manufactured according to conventional methods after the addition of pharmacologically and pharmaceutically acceptable additives. For oral preparations and suppositories, pharmaceutical ingredients such as excipients such as lactose, D-mannitol, corn starch, and crystalline cellulose; disintegrators such as carboxymethylcellulose and carboxymethylcellulose calcium; binders such as hydroxypropyl cellulose, hydroxypropyl methylcellulose, and polyvinylpyrrolidone; lubricants such as magnesium stearate and talc; coating agents such as hydroxypropyl methylcellulose, sucrose, and titanium oxide; plasticizers such as poly(ethylene glycol); bases such as poly(ethylene glycol) and hard fat and the like may be used. For injections, eye drop, ear drop, or nasal drop, pharmaceutical ingredients including solubilizers or solubilizing aids which may constitute aqueous dosage forms or forms dissolved upon use such as distilled water for injection, physiological saline, and propylene glycol; pH modifiers such as inorganic or organic acids or bases; isotonicities such as sodium chloride, glucose, and glycerin; stabilizers and the like may be used. For eye ointments and dermatologic preparations, pharmaceutical ingredients which are suitable for ointments, creams, and patches such as white vaseline, macrogols, glycerin, liquid paraffin, and cotton cloth may be used.

The medicament of the present invention can be administered, for example, as an antibacterial agent for therapeutic or preventive treatment of infectious diseases of mammals including humans. A dose of the medicament of the present invention is not particularly limited, and an appropriate dose can be chosen depending on the kind of pathogenic bacteria, the age and body weight of a patient, severity of a disease and the like. For example, a daily dose is usually from about 10 to 2,000 mg for oral administration, and from about 1 to 1,000 mg for parenteral administration for an adult. The aforementioned dose can be administered once a day or several times a day as divided portions. However, it is desirable that the dose is suitably increased or decreased depending on purpose of therapeutic or preventive treatment, a body part suffering from infection, the kind of pathogenic bacteria, the age or symptom of a patient and the like.

### Examples

The present invention will be explained by referring to Reference examples and Working Examples. However, the scope of the present invention is not limited to these examples. The abbreviations in the tables have the following meanings: Me: methyl group; Et: ethyl group; n-Pr: n-propyl group; i-Pr: i-propyl group; n-Bu: n-butyl group; Boc: tert-butoxycarbonyl group; Z: benzyloxycarbonyl group; Ms: mesyl group; cyc-Hex: cyclohexyl group; Ph: phenyl group.

### Reference example 1

N-tert-Butoxycarbonyl-4-piperidinol

To a suspension of 50.0 g of 4-piperidinol in 250 ml of dried tetrahydrofuran, 125 ml of di-tert-butyl dicarbonate was added under ice-cooling and with stirring, and the mixture was stirred at room temperature for 30 minutes. After the reaction was completed, the solvent was evaporated under reduced pressure to obtain 120.5 g of a pale yellow liquid.
NMR spectrum (CDCl₃) δ ppm:
1.46(9H,s),1.47-1.50(2H,m),1.81-1.87(2H,m),3.01-3.10(2H,m),3.73-3.87(3H,m)
IR spectrum ν (liq.) cm⁻¹: 1698,3684
Mass spectrum (m/z) : 201(M⁺)

The compound of Reference example 2 was obtained in the same manner as in Reference example 1.

### Reference example 2

N-tert-Butoxycarbonyl-3-azetidinol
Appearance : yellow liquid
NMR spectrum (DMSO-d₆) δ ppm :
1.37(9H,s),3.55-3.60(2H,m),3.95-4.00(2H,m),4.30-4.40(1H,m),5.50(1H,d,J=6Hz)
IR spectrum ν (liq.) cm⁻¹: 1678,3416

### Reference example 3

N-tert-Butoxycarbonyl-4-methoxypiperidine

To a suspension of 8.77 g of 60% sodium hydride in 300 ml of dried N,N-dimethylformamide, a solution of 49.0 g of N-tert-butoxycarbonyl-4-piperidinol in 190 ml of dried N,N-dimethylformamide was added under stirring at room temperature. Then, the mixture was added dropwise with 30.4 ml of methyl iodide, and stirred at the same temperature for 5 hours. The reaction solution was poured into ice water, and extracted with ethyl acetate. The extract was washed with saturated brine and dried over sodium sulfate, and then the solvent was evaporated under reduced pressure. The residue was purified by column chromatography (silica gel, ethyl acetate : n-heptane = 1 : 2 to 1 : 1) to obtain 44.1 g of a colorless liquid.
NMR spectrum (CDCl₃) δ ppm :
1.45-1.55(2H,m),1.46(9H,s),1.80-1.90(2H,m),3.05-3.15(2H,m),3.30-3.40(1H,m),3.35(3H, s),3.70-3.80(2H,m)
IR spectrum ν (liq.) cm⁻¹: 1698
Mass spectrum (m/z) : 215 (M⁺)

### Reference example 4

N-tert-Butoxycarbonyl-3-(2-methoxyethoxy)azetidine

To a suspension of 0.25 g of 60% sodium hydride in 5 ml of dried N,N-dimethylformamide, a solution of 1.00 g of N-tert-butoxycarbonyl-3-azetidinol in 3 ml of dried N,N-dimethylformamide was added under stirring at room temperature. The mixture was stirred at room temperature for 30 minutes, then added dropwise with a solution of 0.98 g of 2-methoxyethyl methanesulfonate in 2 ml of dried N,N-dimethylformamide, and stirred at the same temperature for 4 hours. The reaction solution was poured into ice water, and extracted with ethyl acetate. The extract was washed successively with water and saturated brine, and dried over sodium sulfate, and then the solvent was evaporated under reduced pressure. The residue was purified by column chromatography (silica gel, ethyl acetate : n-heptane = 1 : 3) to obtain 0.67 g of a colorless liquid.
NMR spectrum (DMSO-d₆) δ ppm :
1.37(9H,s),3.25(3H,s),3.41-3.45(2H,m),3.46-3.49(2H,m),3.64(2H,dd,J=9,4Hz),3.98(2H,d d,J=9,6.5Hz),4.21-4.26(1H,m)
IR spectrum ν (liq.) cm⁻¹: 1706

### Reference example 5

4-Methoxypiperidine hydrochloride

To 220ml of 9 % hydrogen chloride ethyl acetate solution, a solution of 43.9 g of N-tert-butoxycarbonyl-4-methoxypiperidine in 220 ml of ethyl acetate was added under ice-cooling and with stirring, and the mixture was stirred under ice-cooling for 2.5 hours. After the reaction, the precipitated crystals were collected by filtration to obtain 29.1 g of colorless crystals.
NMR spectrum (CDCl₃) δ ppm :
1.95-2.05(2H,m),2.10-2.20(2H,m),3.15-3.30(4H,m),3.33(3H,s),3.50-3.60(1H,m)
IR spectrum ν (liq.) cm⁻¹: 3448
Mass spectrum (m/z) : 115(M⁺)

The compound of Reference example 6 was obtained in the same manner as in Reference example 5.

### Reference example 6

3-(2-Methoxyethoxy)azetidine hydrochloride
Appearance : pale yellow liquid
NMR spectrum (DMSO-d₆) δ ppm :
3.26(3H,s),3.43(2H,t,J=4.5Hz),3.54(2H,t,J=4.5Hz),3.75-3.80(2H,m),4.05-4.10(2H,m),4. 35-4.40(1H,m)
IR spectrum ν (liq.) cm⁻¹: 3436
Mass spectrum (m/z) : 131(M⁺)

### Reference example 7

3-Fluoro-4-(4-methoxypiperidin-1-yl)nitrobenzene

To a solution of 15.0 g of 3,4-difluoronitrobenzene and 41 ml of N,N-diisopropylethylamine in 150 ml of dried acetonitrile, 15.8 g of 4-methoxypiperidine hydrochloride was added, and the mixture was heated under reflux for 5 hours. The solvent was evaporated under reduced pressure, the residue was added with water and 10% aqueous sodium hydroxide, and the resulting alkaline mixture was extracted with ethyl acetate. The extract was washed with saturated brine, and dried over sodium sulfate, and then the solvent was evaporated under reduced pressure to obtain 24.1 g of a yellowish brown liquid.
NMR spectrum (DMSO-d₆) δ ppm :
1.54-1.62(2H,m),1.92-2.00(2H,m),3.08-3.16(2H,m),3.28(3H,s),3.38-3.46(1H,m),3.49-3.5 7(2H,m),7.16(1H,t,J=8.5Hz),7.95(1H,dd,J=14,3Hz),7.97(1H,dd,J=8.5,3Hz)
IR spectrum ν (liq.) cm⁻¹: 1336,1518
Mass spectrum (m/z): 254(M⁺)

The compounds of Reference examples 8 through 18 were obtained in the same manner as in Reference example 7.

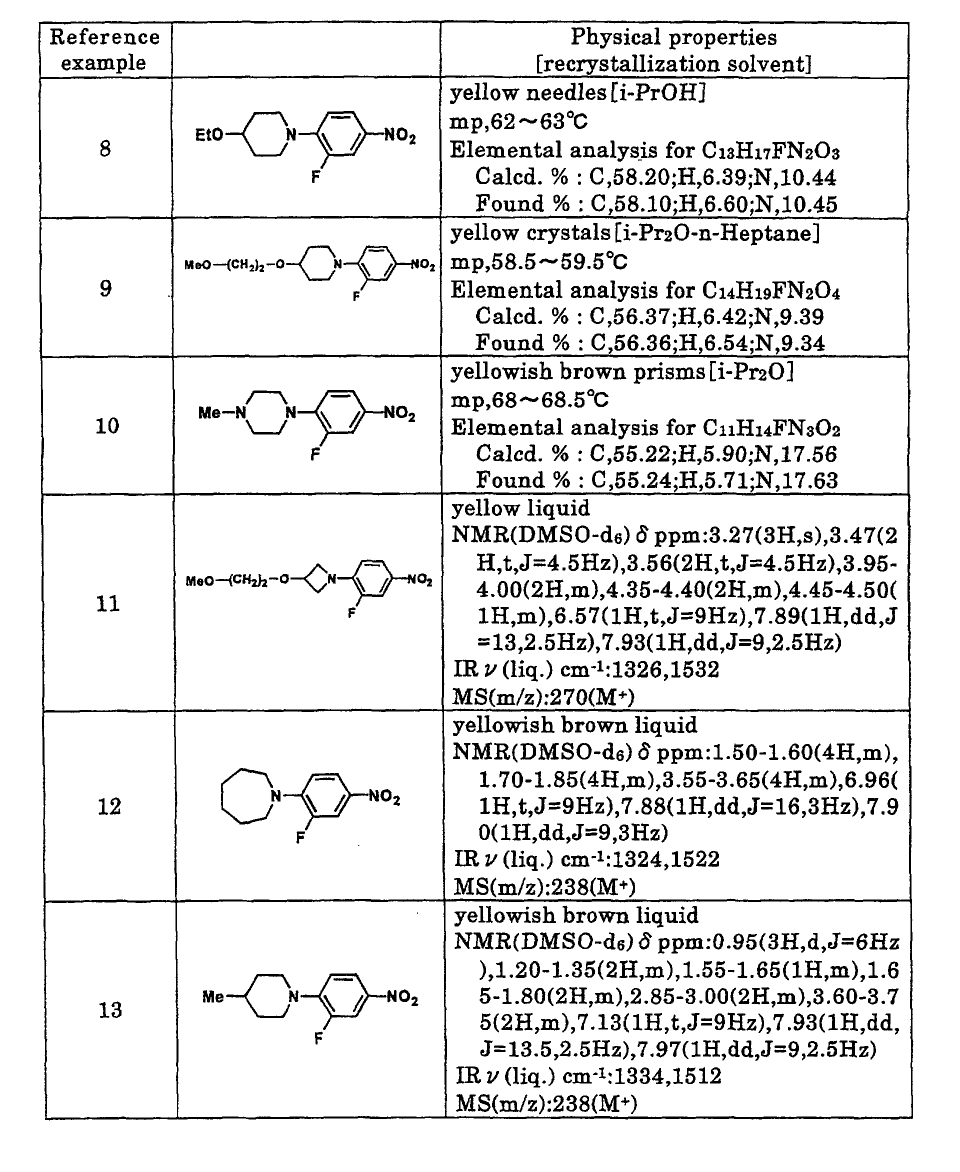

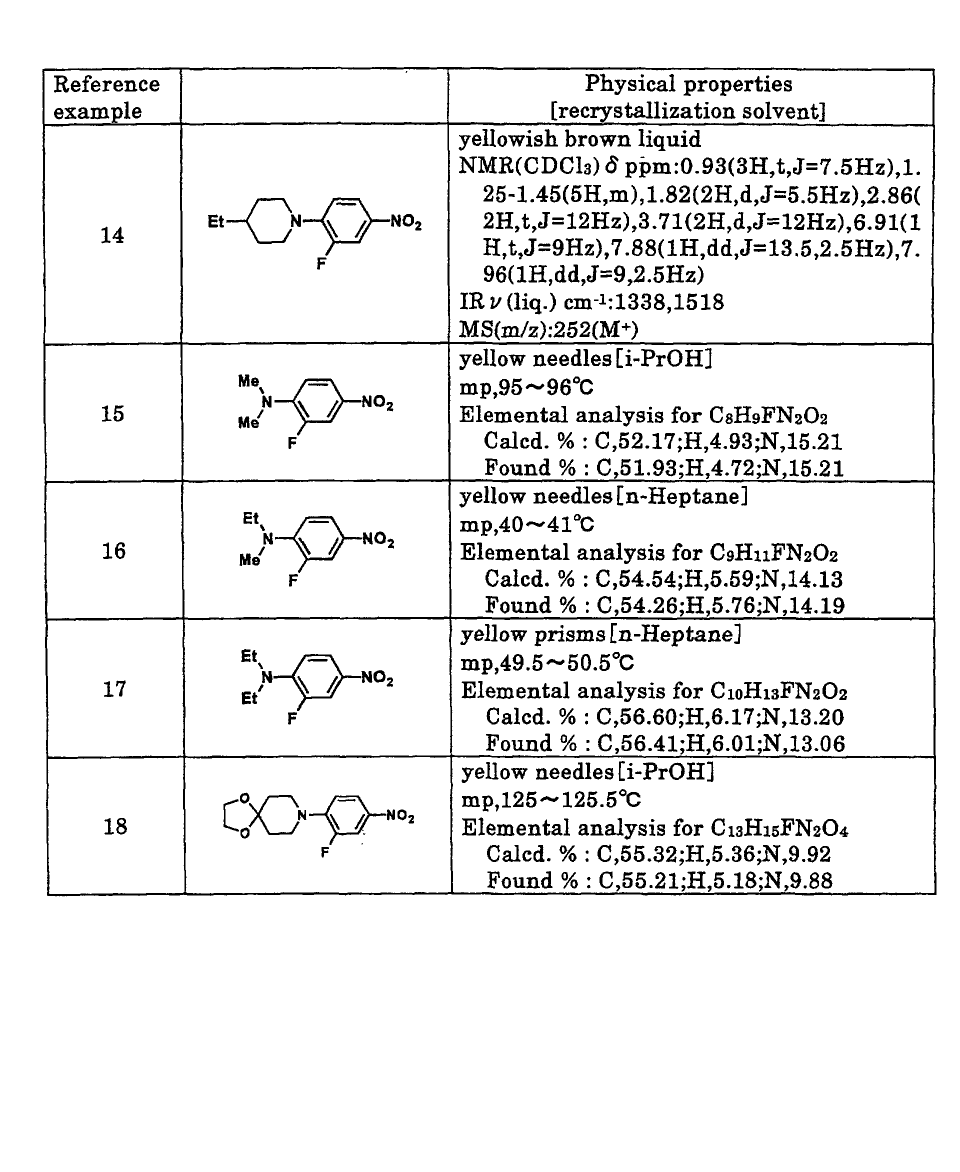

### Reference example 19

3-Fluoro-4-(2-methoxyethoxy)nitrobenzene

To a suspension of 4.20 g of 60 % sodium hydride in 100 ml of dried tetrahydrofuran, a solution of 7.90 g of ethylene glycol monomethyl ether in 50 ml of dried tetrahydrofuran was added dropwise under ice-cooling and with stirring, and the mixture was stirred at room temperature for 15 minutes. The reaction mixture was added dropwise with a solution of 15.0 g of 3,4-difluoronitrobenzene in 50 ml of dried tetrahydrofuran under ice-cooling and with stirring, and stirred at the same temperature for 30 minutes. The reaction solution was added with ice water, and extracted with ethyl acetate. The extract was washed with saturated brine, and dried over sodium sulfate, and then the solvent was evaporated under reduced pressure. The residue was washed with n-hexane to obtain 19.0 g of yellow crystals. Recrystallization from diisopropyl ether gave yellow needles having the melting point of from 62.5 to 63°C.

| Elemental analysis for C₉H₁₀FNO₄ | | | | | | |
|---|---|---|---|---|---|---|
| Calculated % | C, | 50.24; | H, | 4.68; | N, | 6.51 |
| Found % | C, | 50.18; | H, | 4.54; | N, | 6.50 |

The compounds of Reference examples 20 through 21 were obtained in the same manner as in Reference example 19.

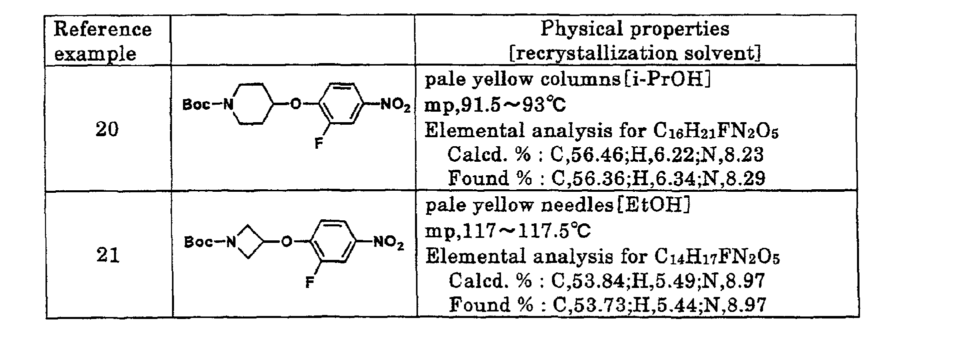

### Reference example 22

3-Fluoro-4-(4-methylpiperazin-1-yl)aniline

A suspension of 19.0 g of 3-fluoro-4-(4-methylpiperazin-1-yl)nitrobenzene and 0.190 g of platinum(IV) oxide in 190 ml of methanol was stirred at a hydrogen pressure of 2 atm at ordinary temperature for 2 hours. The catalyst was filtered off, and then the filtrate was concentrated under reduced pressure to obtain 17.0 g of dark brown crystals. Recrystallization from diisopropyl ether gave dark brown prisms having the melting point of from 87 to 88°C.

| Elemental analysis for C₁₁H₁₆FN₃ | | | | | | |
|---|---|---|---|---|---|---|
| Calculated % | C, | 63.13; | H, | 7.71; | N, | 20.08 |
| Found % | C, | 63.10; | H, | 7.46; | N, | 20.08 |

The compounds of Reference examples 23 through 36 were obtained in the same manner as in Reference example 22.

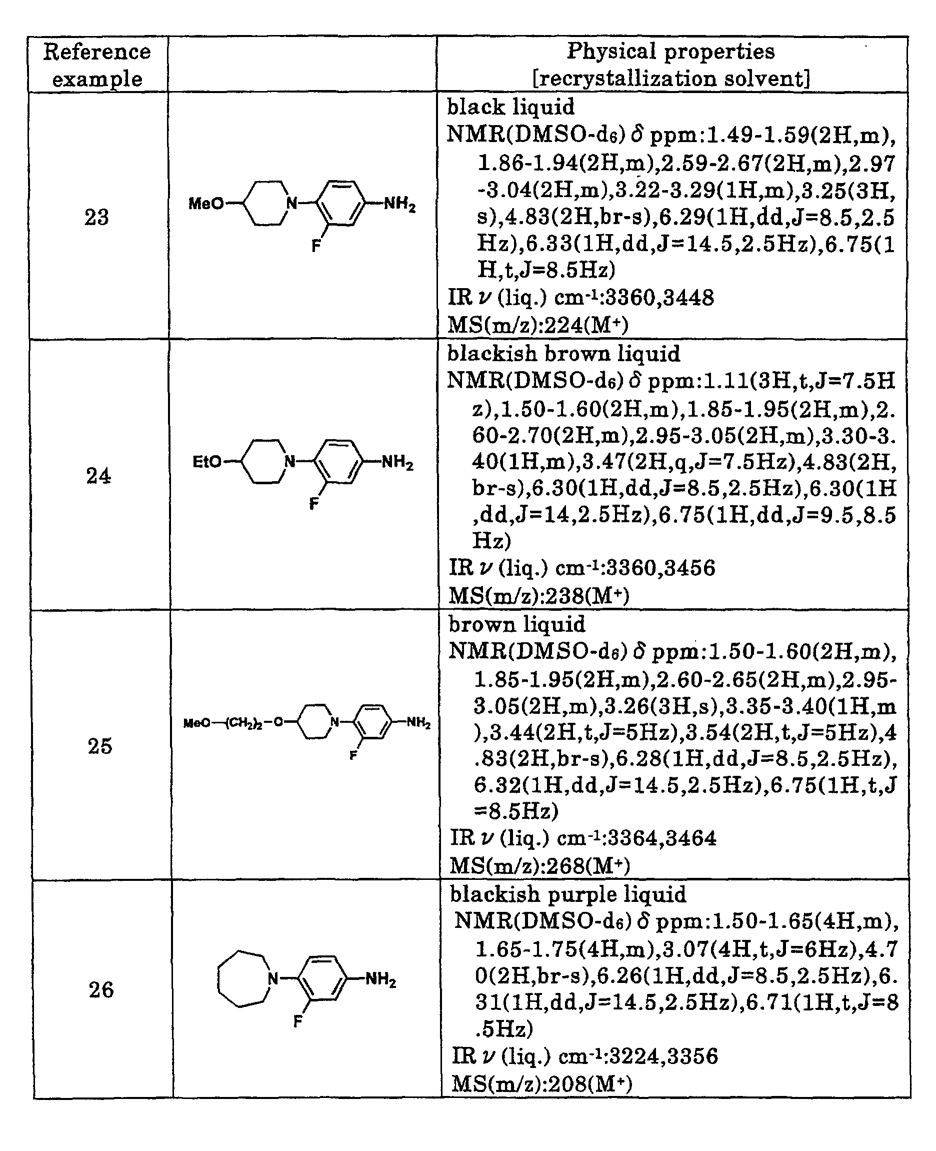

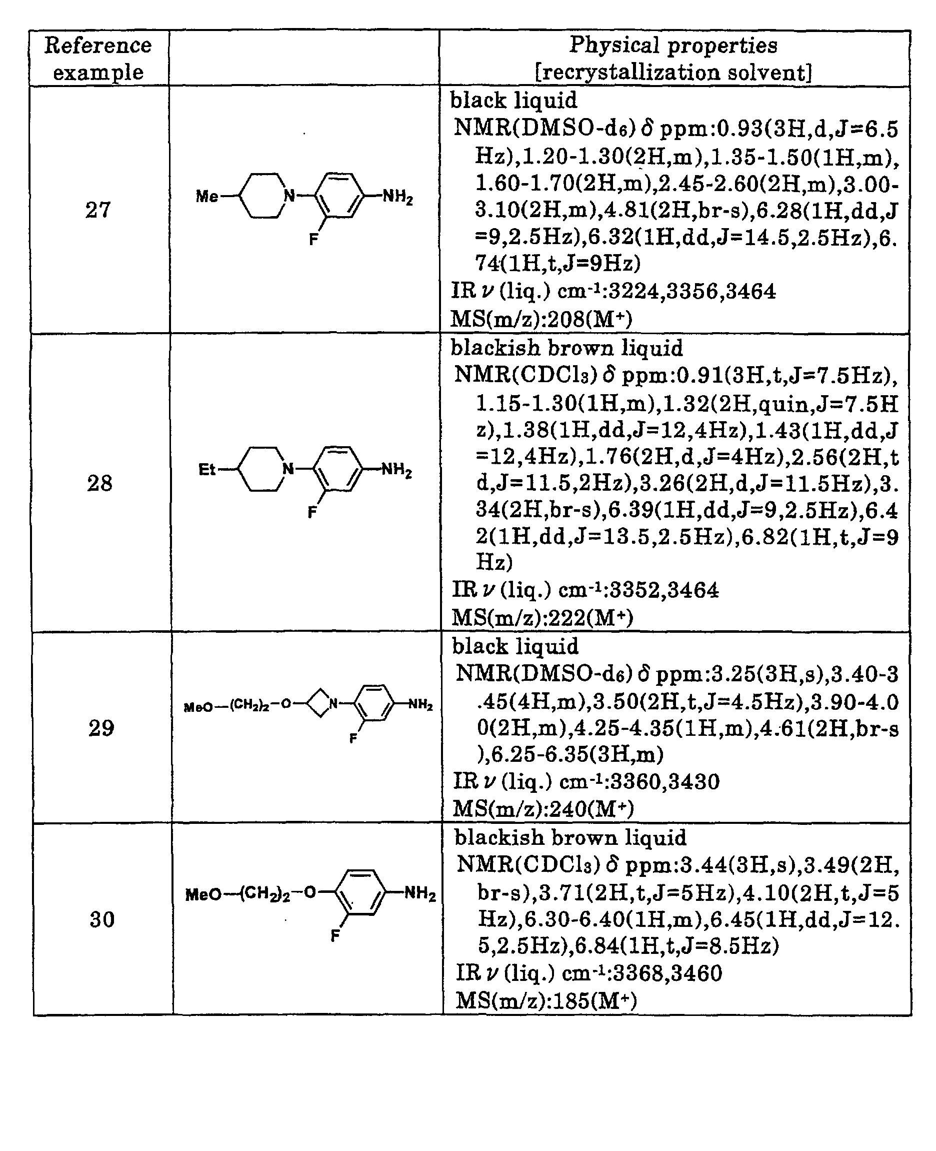

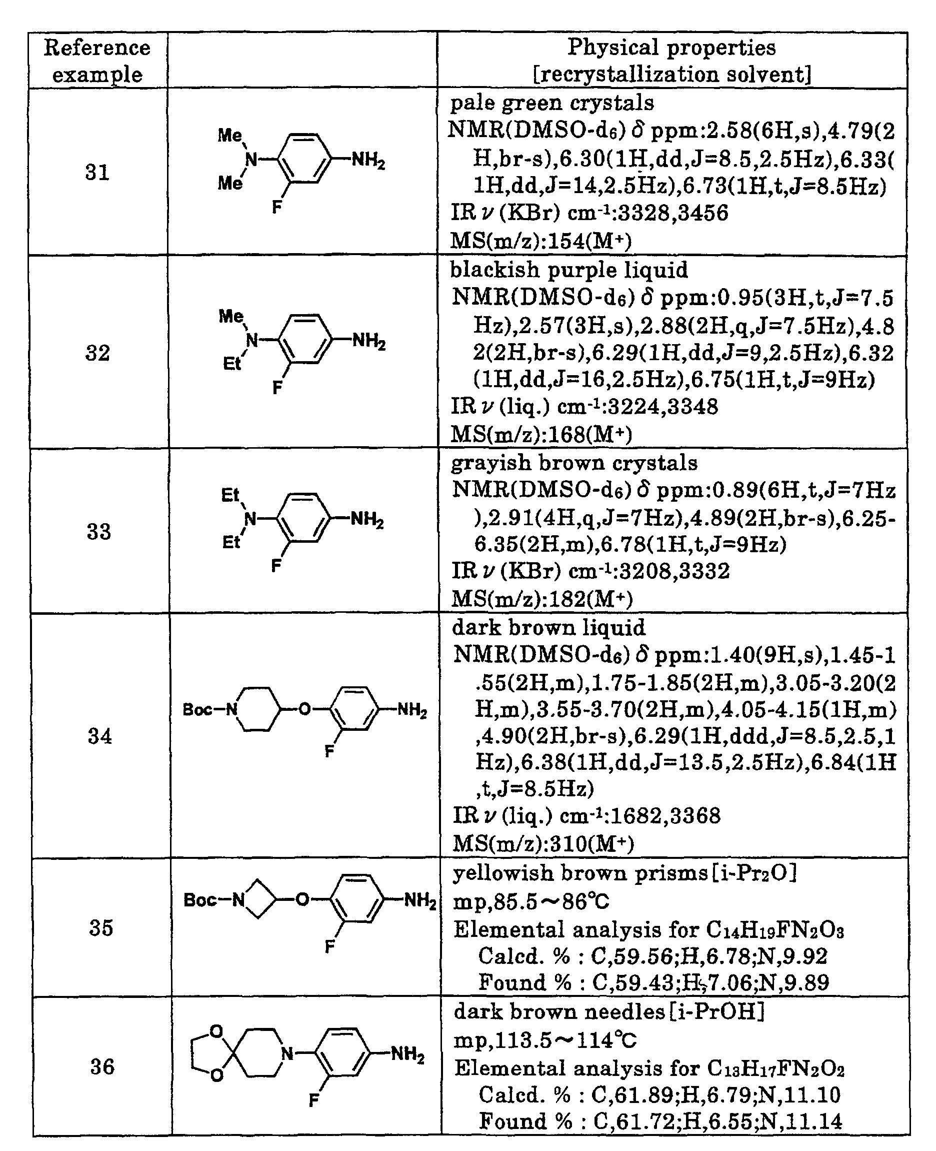

### Reference example 37

N-Benzyloxycarbonyl-4-(thiomorpholin-4-yl)aniline

To a mixture of a solution of 19.0 g of 4-(thiomorpholin-4-yl)aniline in 190 ml of 10% aqueous sodium carbonate and 190 ml of acetone, 21.0 ml of benzyloxycarbonyl chloride was added dropwise under ice-cooling and with stirring. The resulting mixture was stirred at room temperature for 30 minutes, and then the precipitated crystals were collected by filtration, and washed with diisopropyl ether to obtain 25.5 g of pale brown crystals. Recrystallization from a mixture of ethyl acetate-diisopropyl ether gave colorless needles having the melting point of from 145 to 146.5°C.

| Elemental analysis for C₁₈H₂₀N₂O₂S | | | | | | |
|---|---|---|---|---|---|---|
| Calculated % | C, | 65.83; | H, | 6.14; | N, | 8.53 |
| Found % | C, | 65.69; | H, | 6.12; | N, | 8.38 |

The compounds of Reference examples 38 through 53 were obtained in the same manner as in Reference example 37.

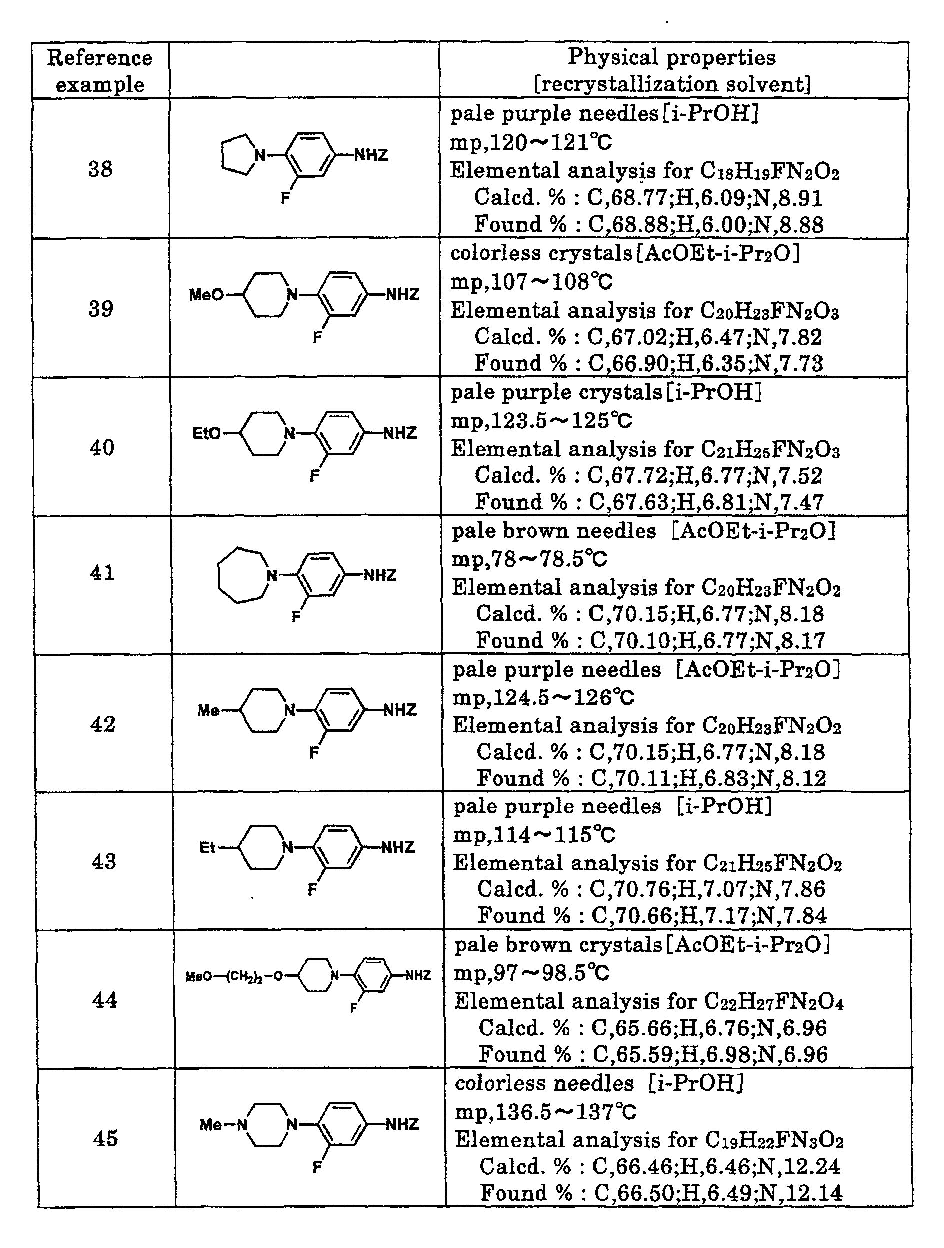

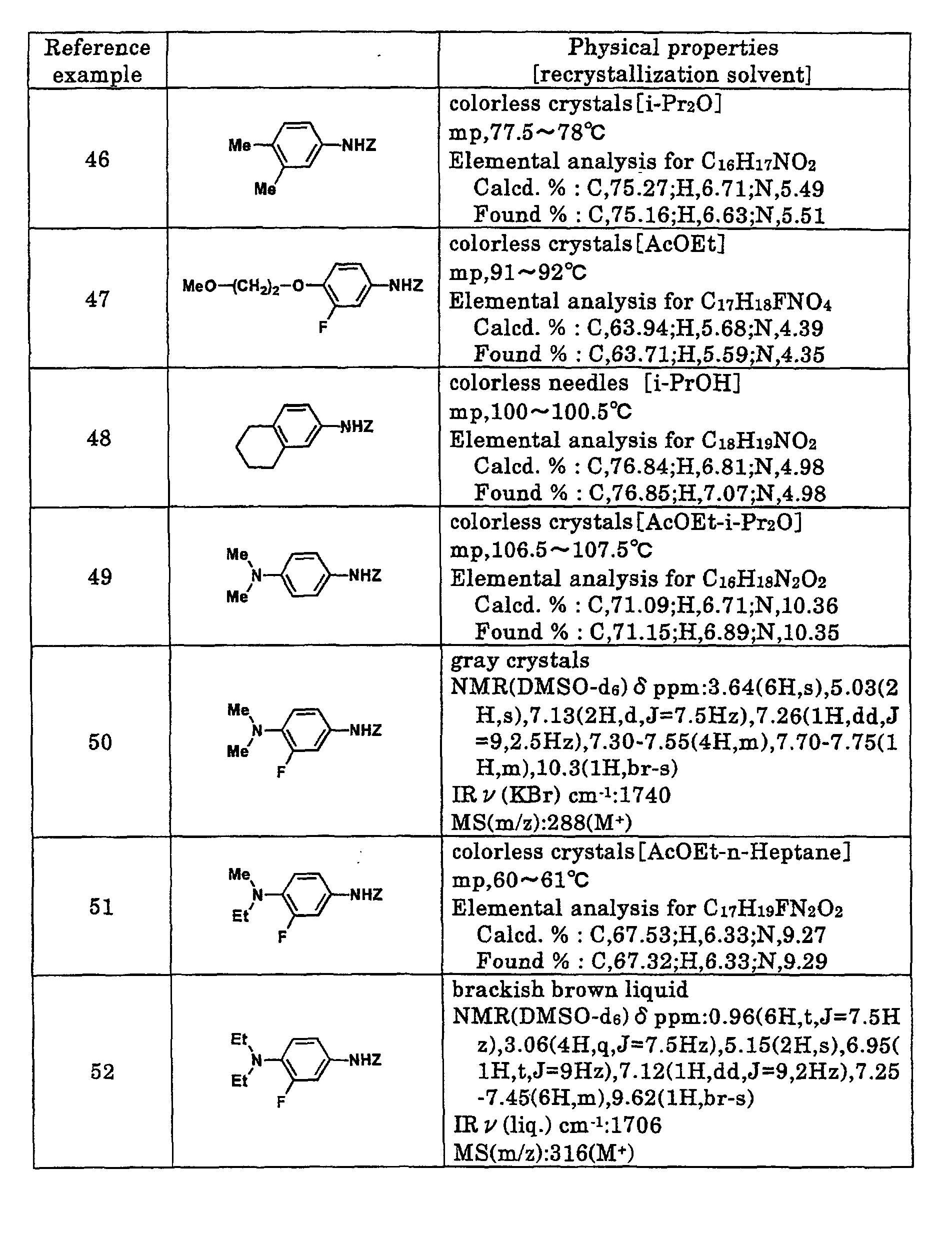

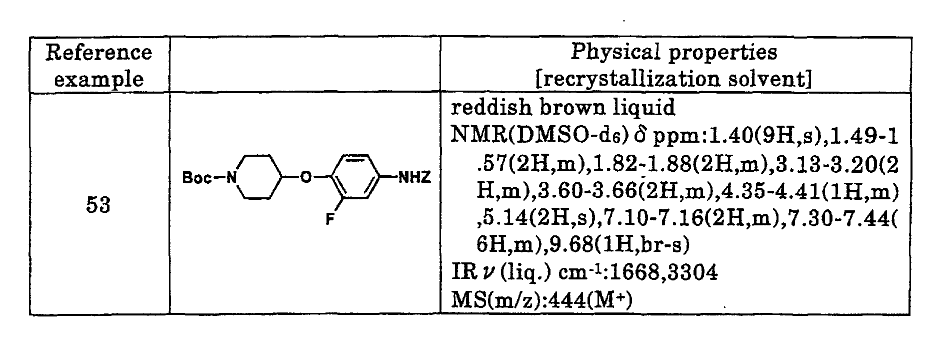

### Reference example 54

N,N'-Di-tert-butoxycarbonyl-3-fluoro-4-(piperazin-1-yl)aniline

To a solution of 5.56 g of di-tert-butyl dicarbonate in 10 ml of methanol, a solution of 2.00 g of 3-fluoro-4-(piperazin-1-yl)aniline in 10 ml of methanol was added dropwise under stirring at room temperature, and the mixture was stirred overnight at room temperature. The precipitated crystals were collected by filtration, and washed with ethanol to obtain 3.12 g of yellow crystals. Recrystallization from ethyl acetate gave pale yellow crystals having the melting point of from 194 to 195°C.

| Elemental analysis for C₂₀H₃₀FN₃O₄ | | | | | | |
|---|---|---|---|---|---|---|
| Calculated % | C, | 60.74; | H, | 7.65; | N, | 10.63 |
| Found % | C, | 60.47; | H, | 7.93; | N, | 10.53 |

The compounds of Reference examples 55 through 57 were obtained in the same manner as in Reference example 54.

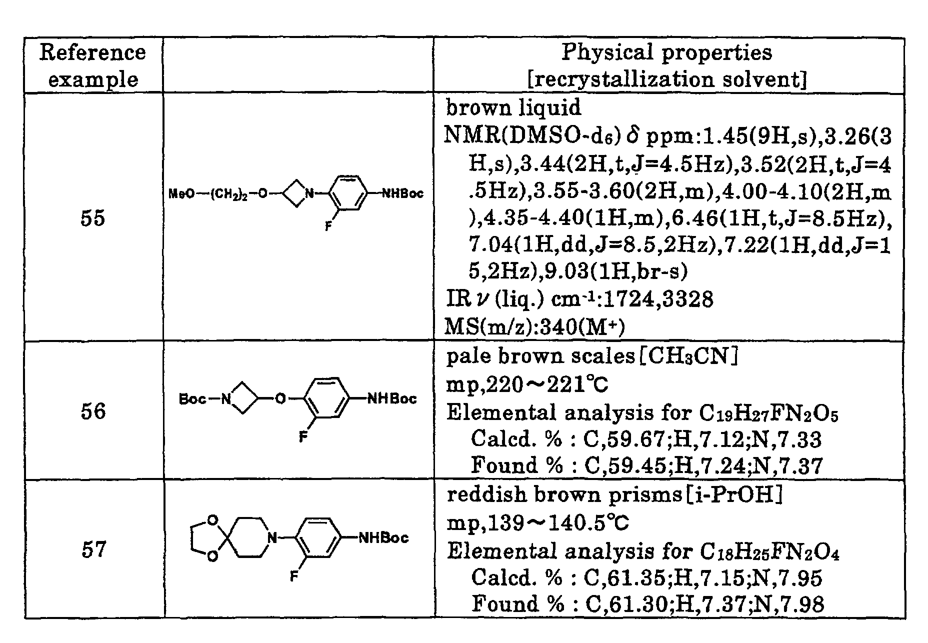

### Reference example 58

(R)-5-Hydroxymethyl-2-oxo-3-[4-(thiomorpholin-4-yl)phenyl]oxazolidine

To a solution of 25.0 g of N-benzyloxycarbonyl-4-(thiomorpholin-4-yl)aniline in 250 ml of dried tetrahydrofuran, 50 ml of 1.63 mol/L n-butyl lithium n-hexane solution was added dropwise with stirring at -78°C under nitrogen flow. Then, the mixture was stirred at the same temperature for 1 hour, added dropwise with 11.5 ml of (R)-(-)-glycidyl butyrate, and stirred at the same temperature for 1 hour, and then at room temperature for 23 hours. The reaction mixture was added with 250 ml of 10% aqueous ammonium chloride, and extracted with ethyl acetate. The extract was washed successively with water and saturated brine, and dried over sodium sulfate, and then the solvent was evaporated under reduced pressure. The residue was washed with diisopropyl ether to obtain 18.8 g of grayish brown crystals. Recrystallization from ethyl acetate gave colorless crystals having the melting point of from 126.5 to 127.5°C.

| Elemental analysis for C₁₄H₁₈N₂O₃S | | | | | | |
|---|---|---|---|---|---|---|
| Calculated % | C, | 57.12; | H, | 6.16; | N, | 9.52 |
| Found % | C, | 56.85; | H, | 6.13; | N, | 9.25 |

Specific rotation [α]_{D}²⁰ -40.9° (c = 0.1, DMSO)

The compounds of Reference examples 59 through 79 were obtained in the same manner as in Reference example 58.

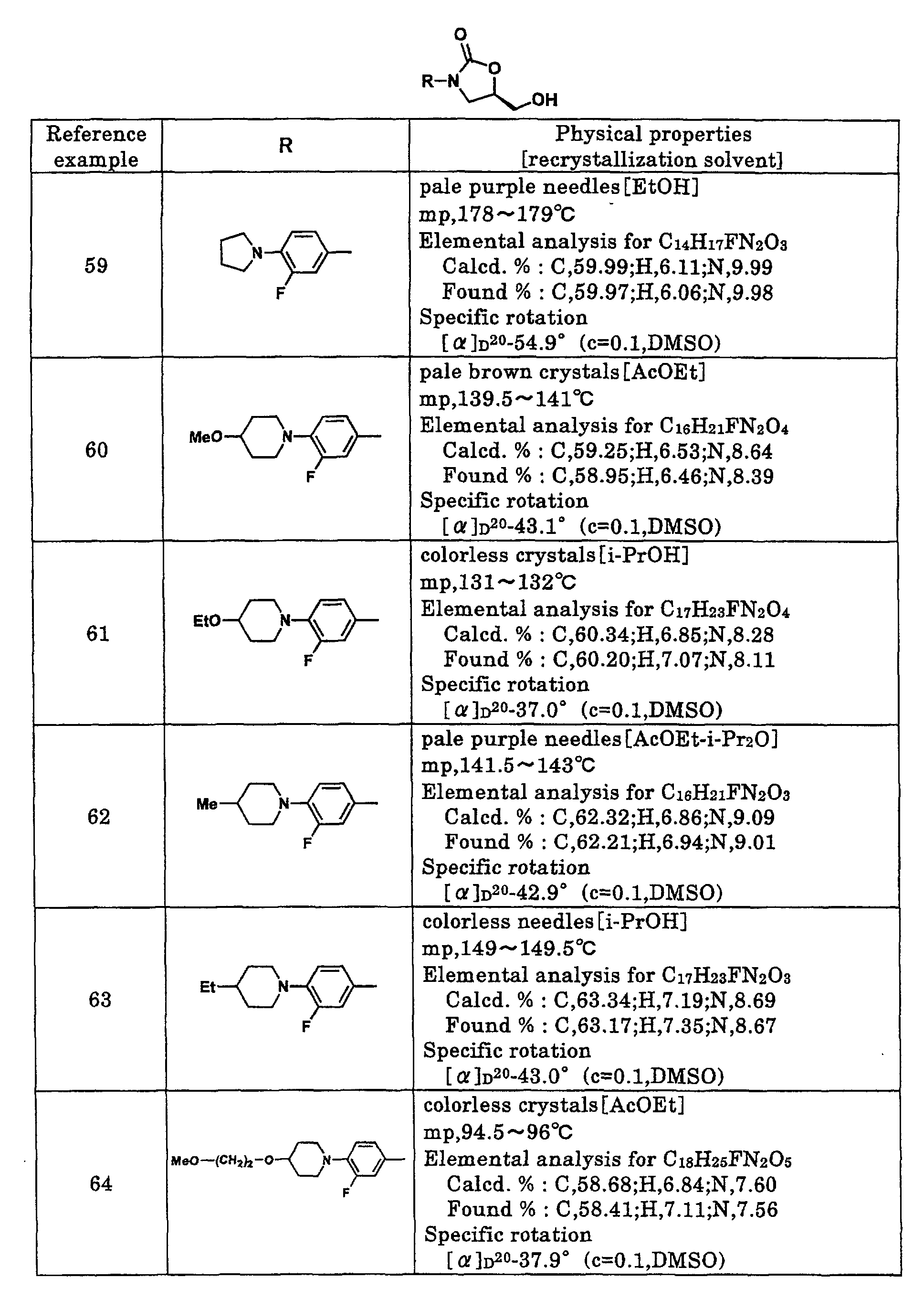

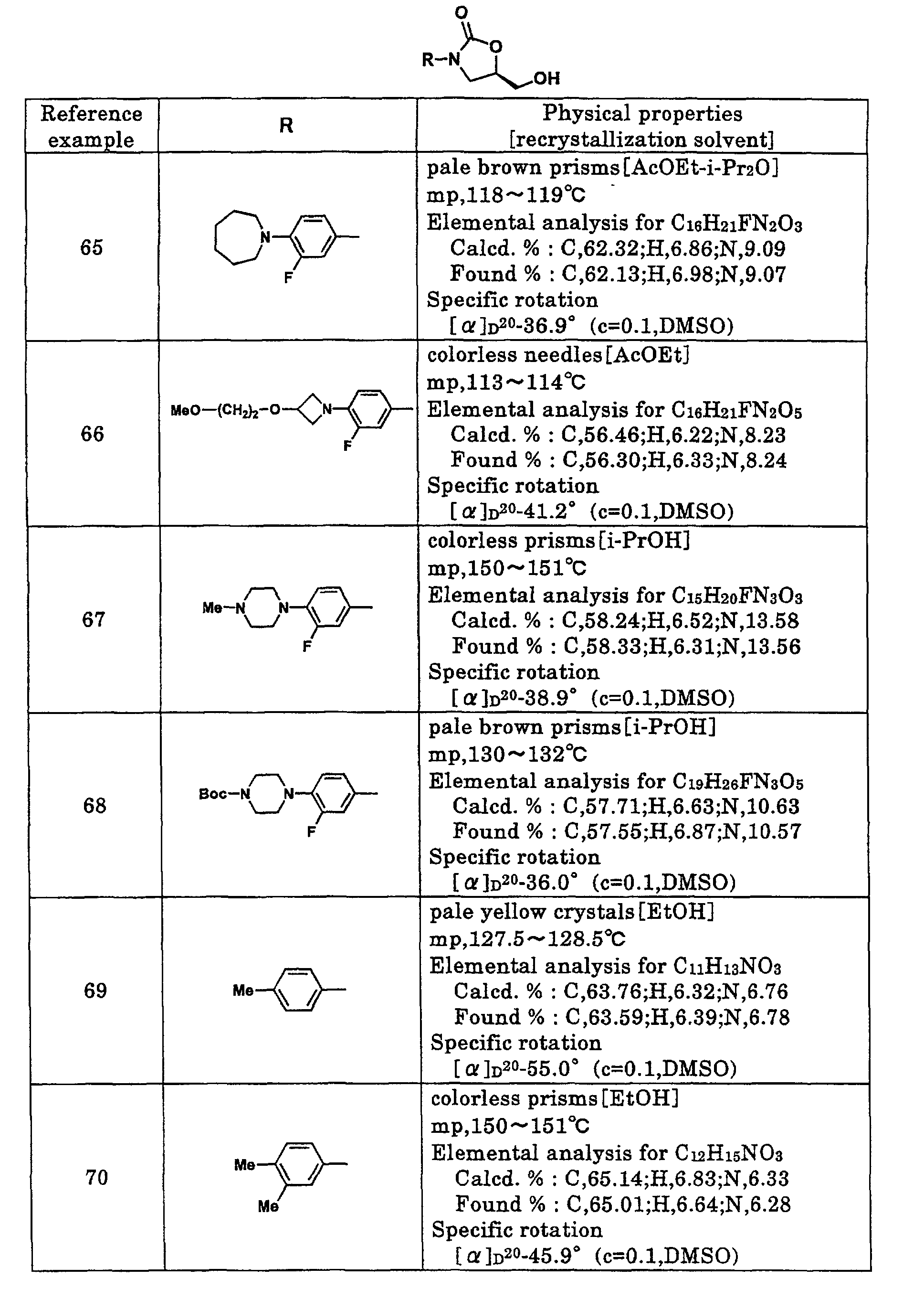

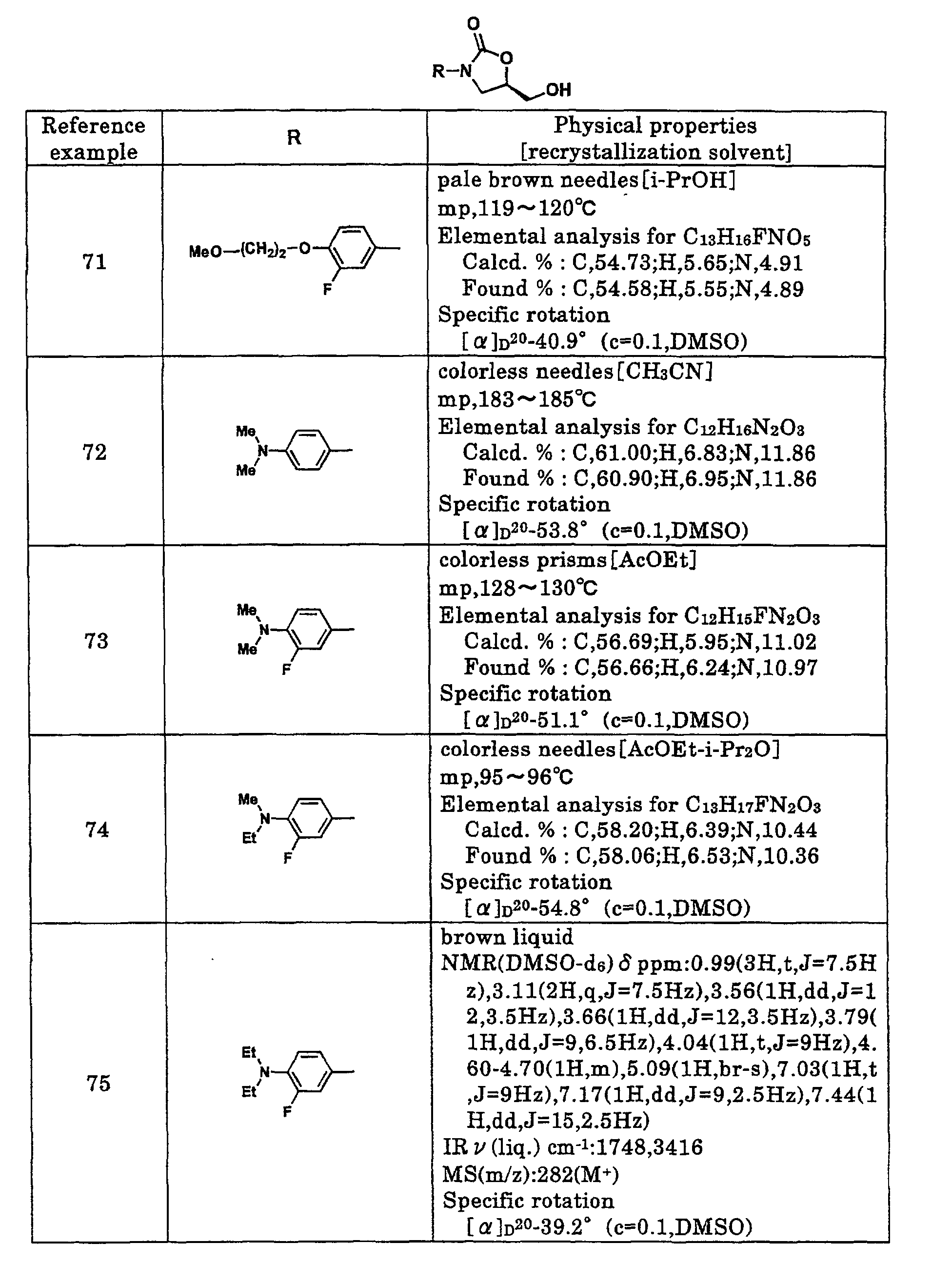

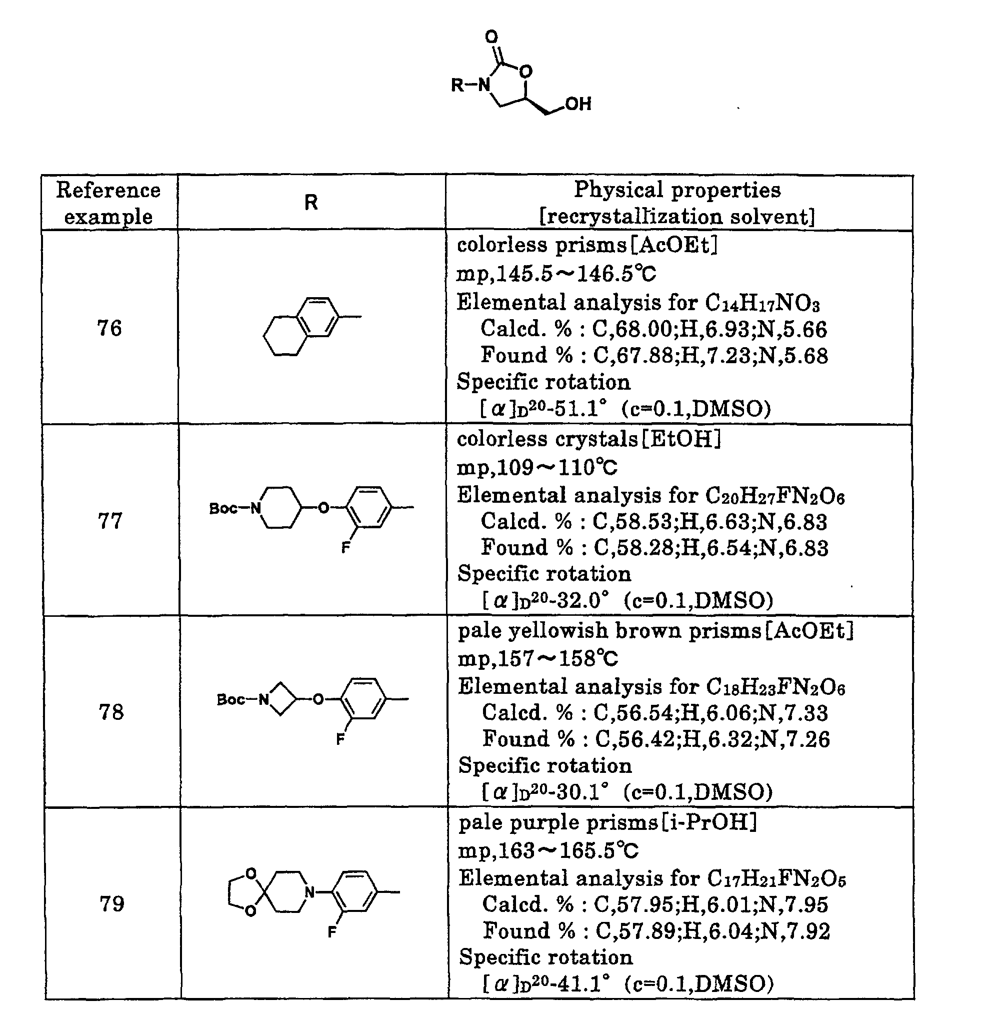

### Reference example 80

(R)-5-Mesyloxymethyl-2-oxo-3-[4-(thiomorpholin-4-yl)phenyl]oxazolidine

To a solution of 10.0 g of (R)-5-hydroxymethyl-2-oxo-3-[4-(thiomorpholin-4-yl)phenyl]oxazolidine and 10.5 ml of triethylamine in 200 ml of dichloromethane, 3.20 ml of methanesulfonyl chloride was added dropwise under ice-cooling and with stirring, and then the mixture was stirred at room temperature for 2 hours. The reaction solution was added with 200 ml of water, and extracted with dichloromethane. The extract was washed successively with water and saturated brine, and dried over sodium sulfate, and then the solvent was evaporated under reduced pressure. The residue was washed with diisopropyl ether to obtain 11.5 g of grayish brown crystals. Recrystallization from ethyl acetate gave colorless prisms having the melting point of from 174.5 to 175.5°C.

| Elemental analysis for C₁₅H₂₀N₂O₅S₂ | | | | | | |
|---|---|---|---|---|---|---|
| Calculated % | C, | 48.37; | H, | 5.41; | N, | 7.52 |
| Found % | C, | 48.41; | H, | 5.33; | N, | 7.36 |

Specific rotation [α]_{D}²⁰ -54.2° (c = 0.1, DMSO)

The compounds of Reference examples 81 through 101 were obtained in the same manner as in Reference example 80.

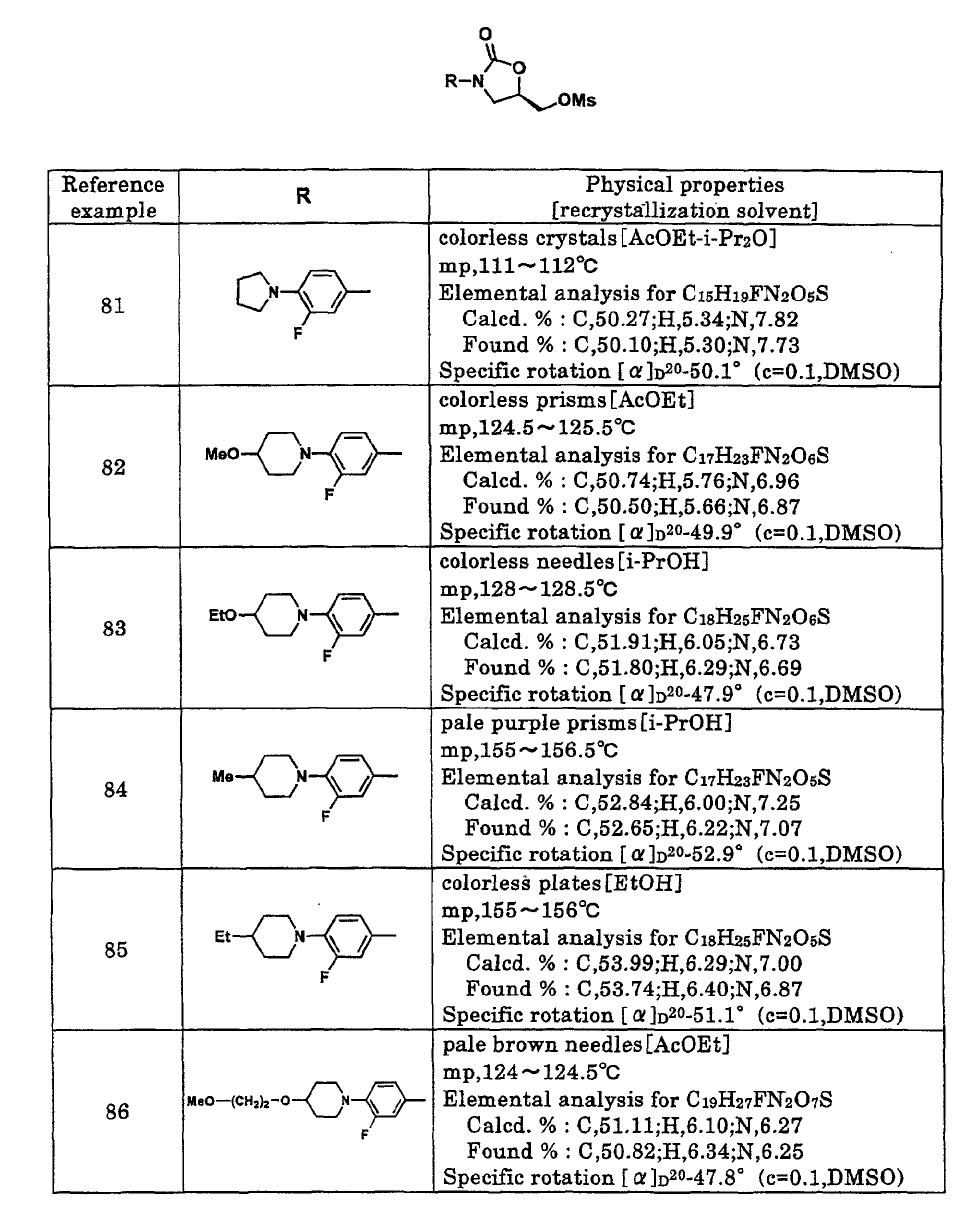

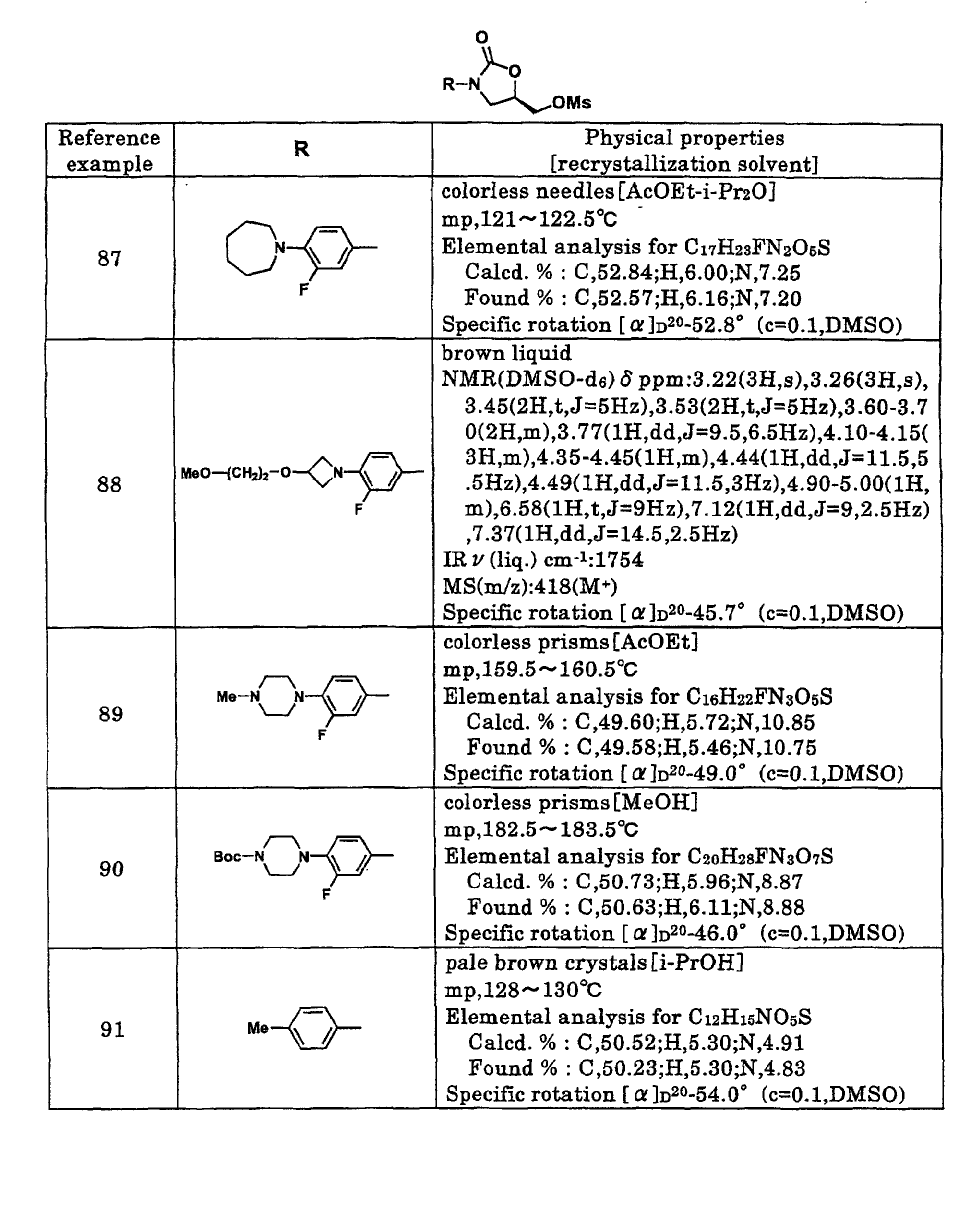

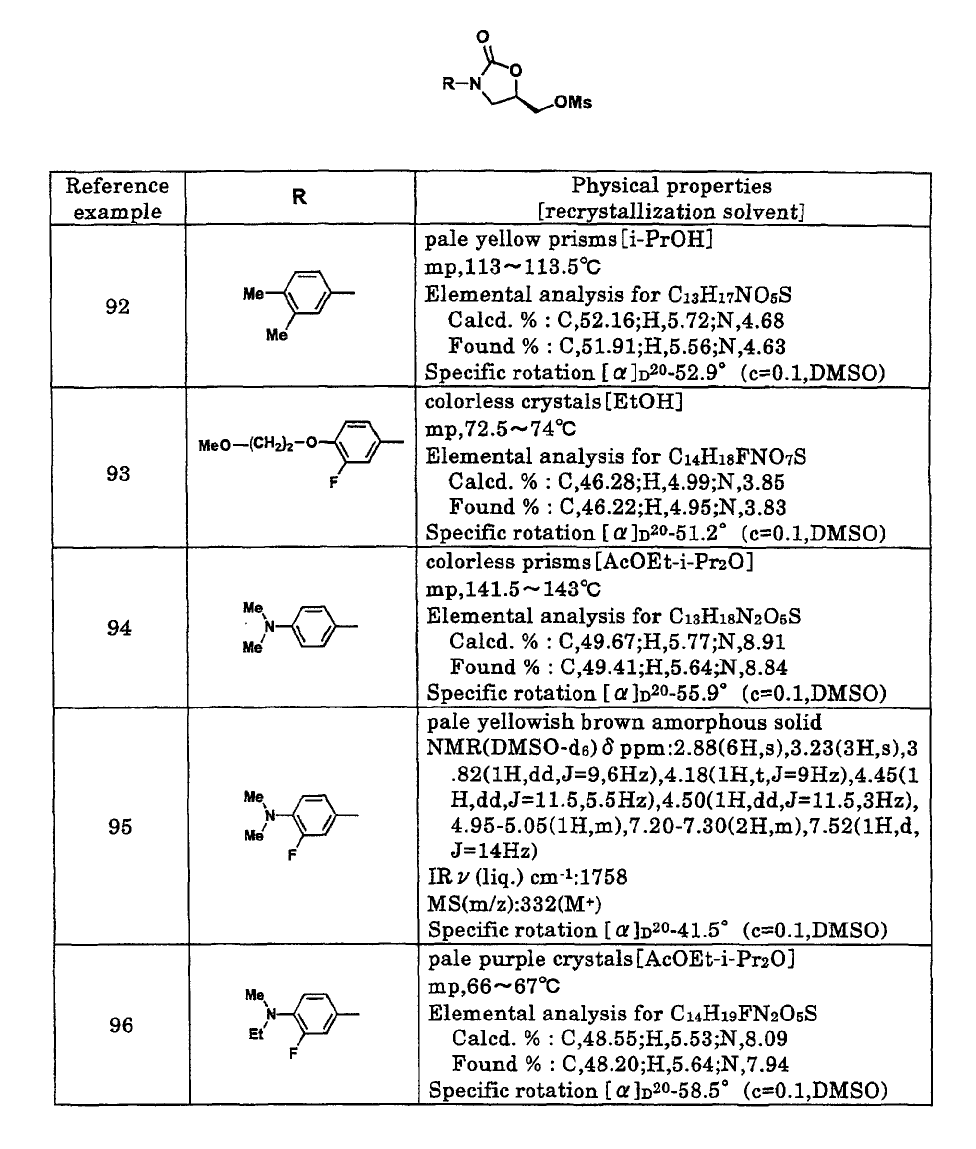

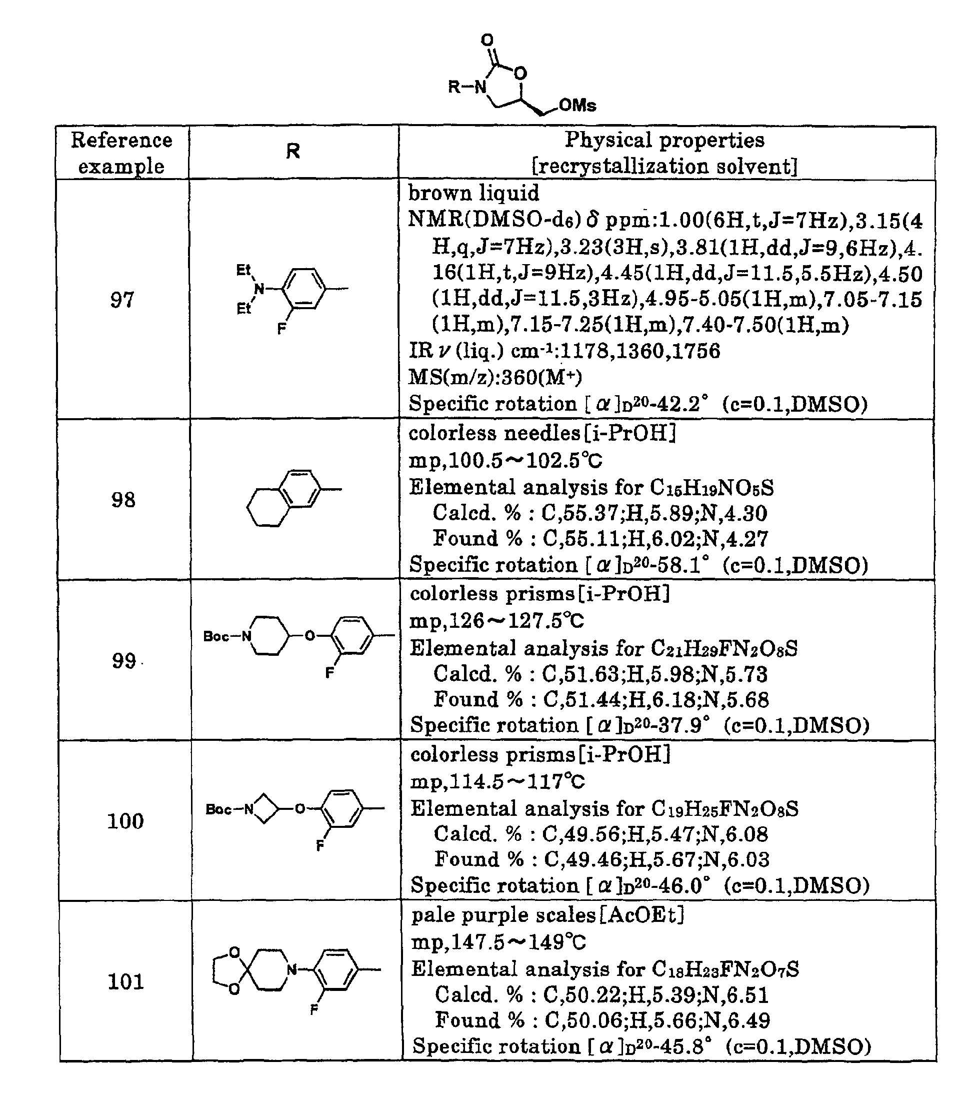

### Reference example 102

(R)-5-Azidomethyl-2-oxo-3-[4-(thiomorpholin-4-yl)phenyl]oxazolidine

A suspension of 11.5 g of (R)-5-mesyloxymethyl-2-oxo-3-[4-(thiomorpholin-4-yl)phenyl]oxazolidine and 8.35 g of sodium azide in 110 ml of dried N,N-dimethylformamide was stirred with heating to 65°C for 5 hours. The reaction solution was cooled, then added with 200 ml of water, and extracted with ethyl acetate. The extract was washed successively with water and saturated brine, and dried over sodium sulfate, and then the solvent was evaporated under reduced pressure. The residue was washed with diisopropyl ether to obtain 8.85 g of grayish brown crystals. Recrystallization from ethyl acetate gave colorless crystals having the melting point of from 110 to 111°C.

| Elemental analysis for C₁₄H₁₇N₅O₂S | | | | | | |
|---|---|---|---|---|---|---|
| Calculated % | C, | 52.65; | H, | 5.37; | N, | 21.93 |
| Found % | C, | 52.47; | H, | 5.35; | N, | 21.65 |

Specific rotation [α]_{D}²⁰ -124.4° (c = 0.1, DMSO)

The compounds of Reference examples 103 through 123 were obtained in the same manner as in Reference example 102.

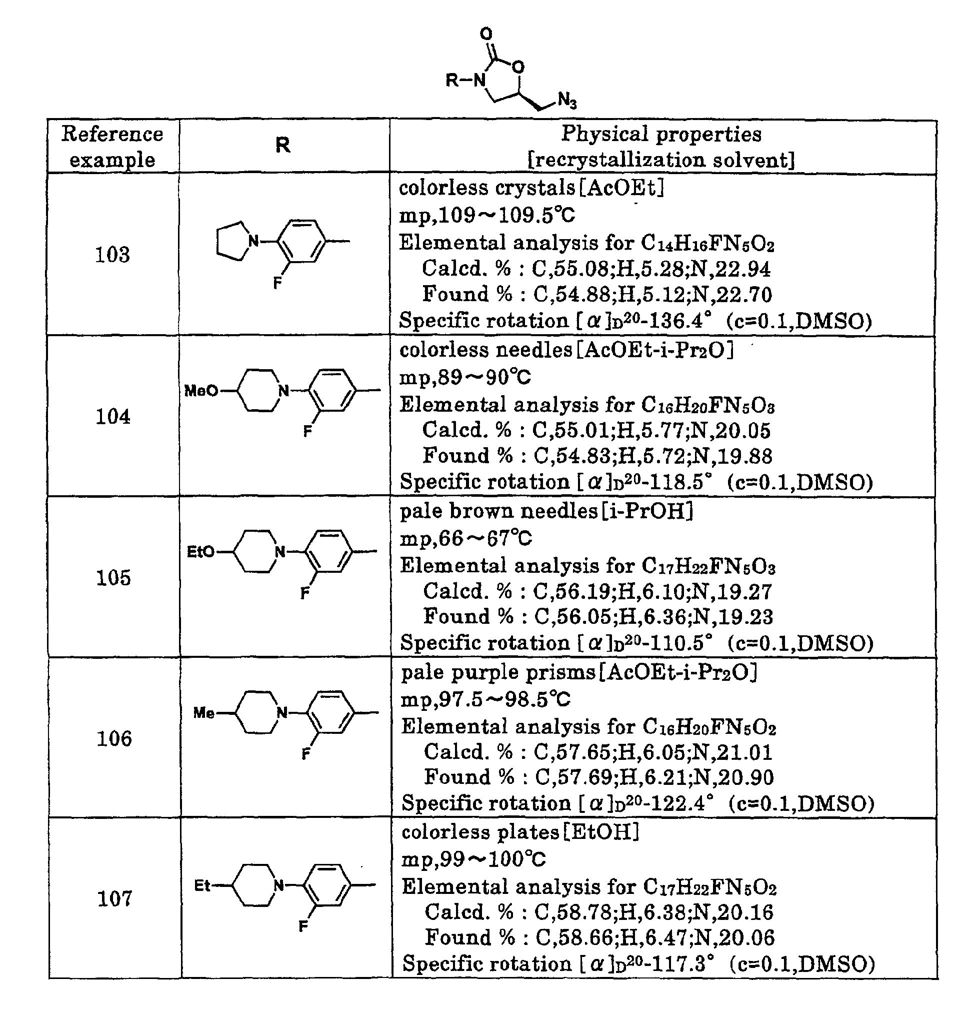

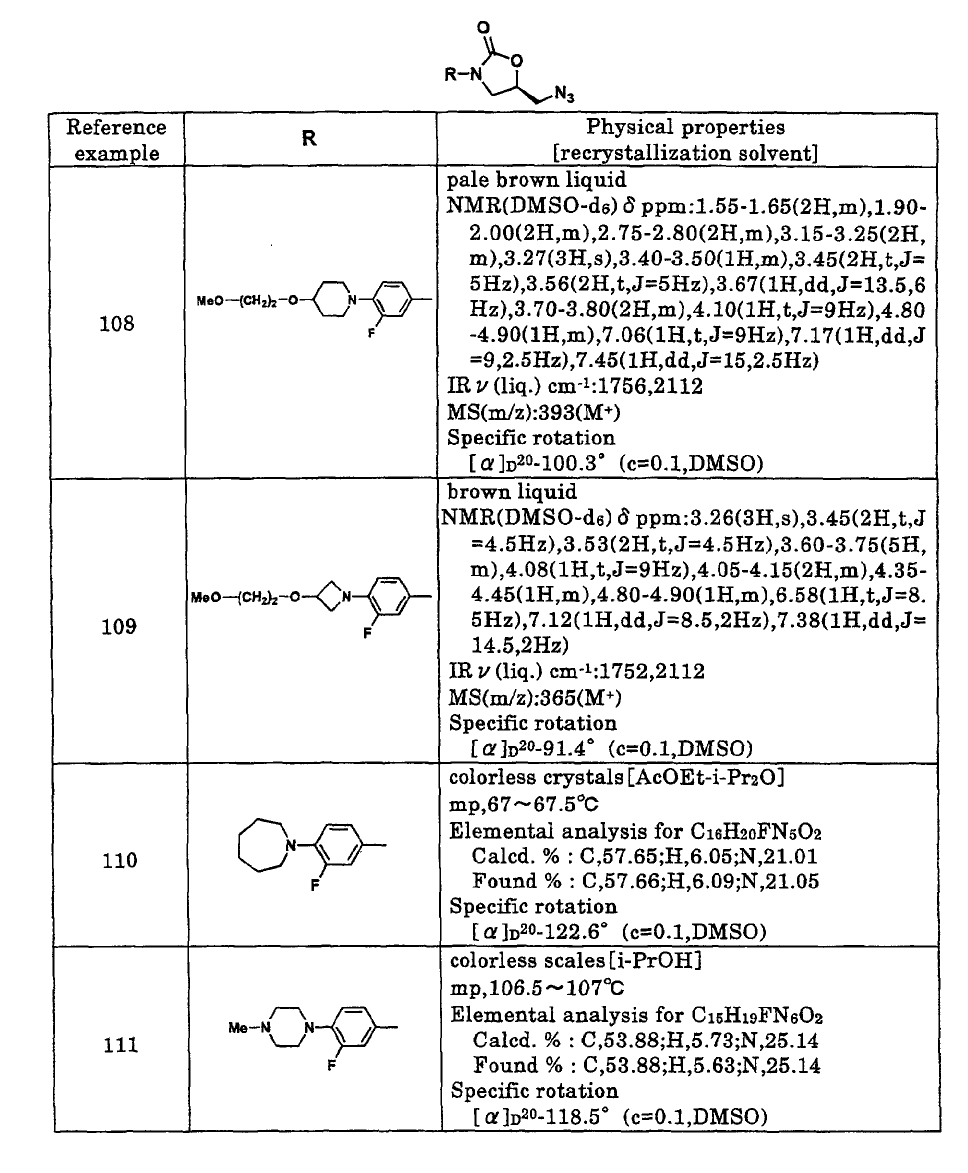

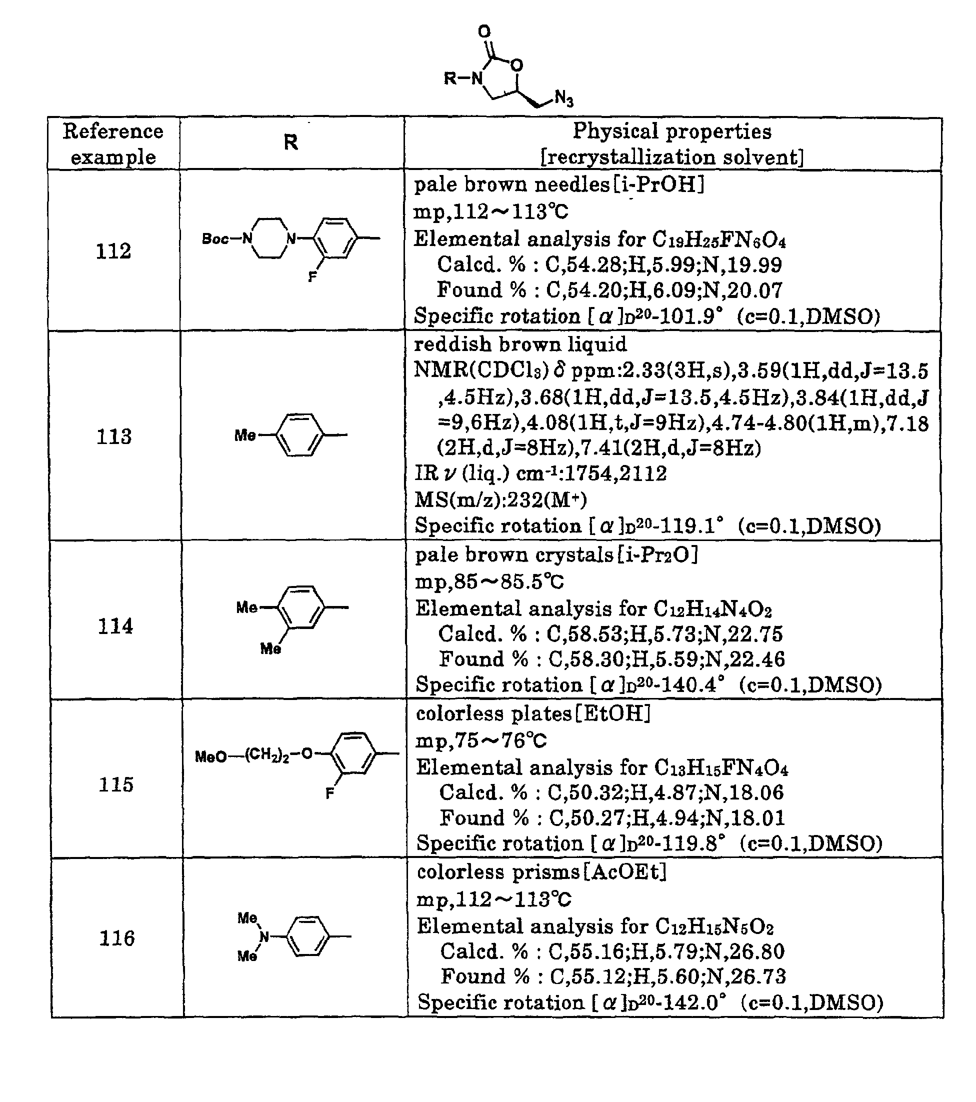

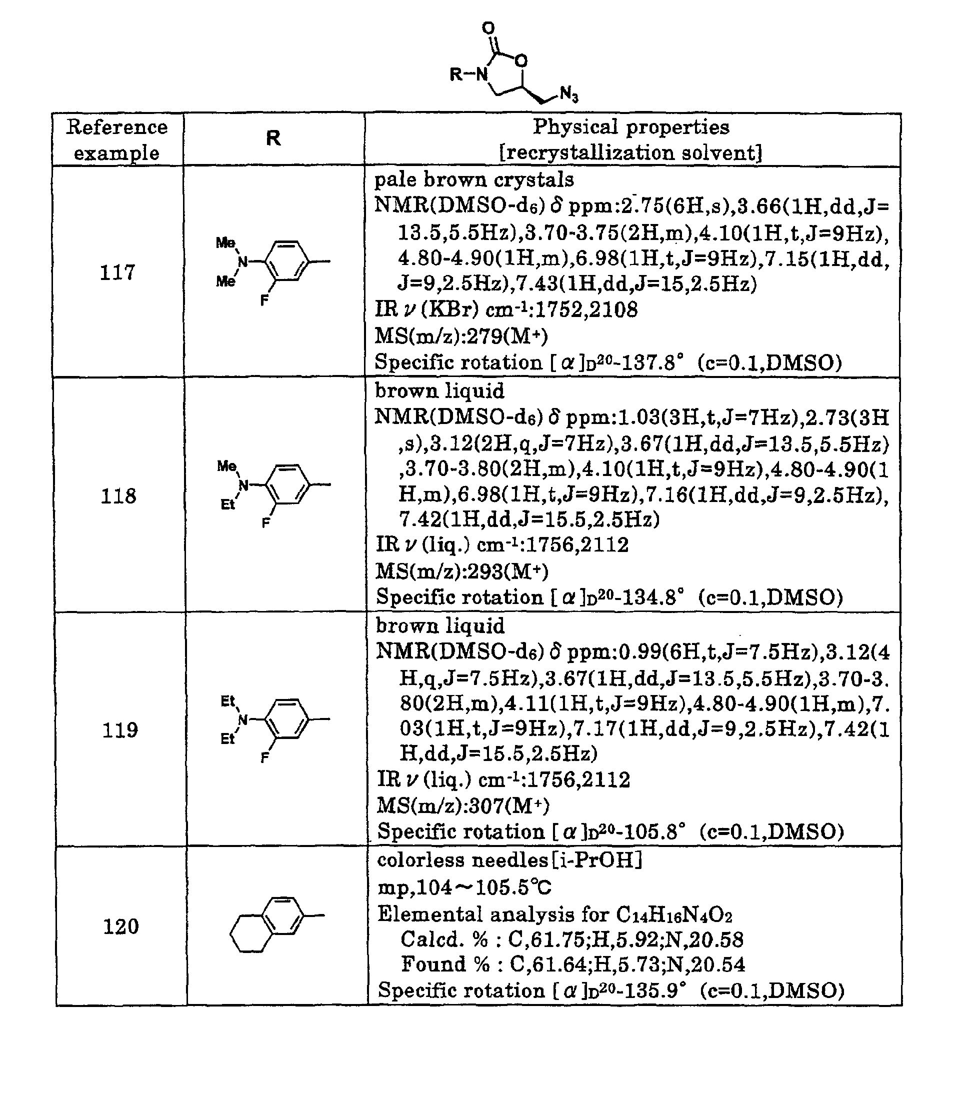

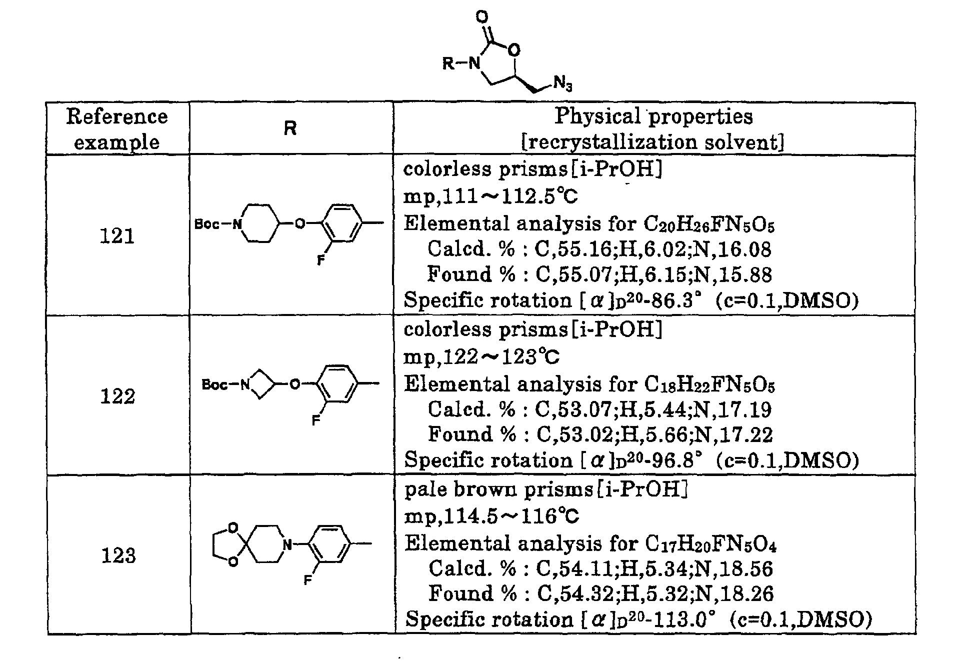

### Reference example 124

(R)-5-Azidomethyl-3-[3-fluoro-4-(piperazin-1-yl)phenyl]-2-oxooxazolidine

To 1.00 g of (R)-5-azidomethyl-3-[4-(4-tert-butoxycarbonylpiperazin-1-yl)-3-fluorophenyl]-2-oxooxazolidine, 20 ml of 16 % hydrogen chloride ethyl acetate solution was added, and the mixture was stirred at room temperature for 30 minutes. The precipitated crystals were collected by filtration. The crystals were added with aqueous sodium hydroxide, and then the resulting alkaline mixture was extracted with ethyl acetate. The extract was washed with saturated brine and dried over sodium sulfate, and then the solvent was evaporated under reduced pressure to obtain 0.72 g of pale brown crystals. Recrystallization from isopropanol gave colorless crystals having the melting point of from 114 to 115°C.

| Elemental analysis for C₁₄H₁₇FN₆O₂ | | | | | | |
|---|---|---|---|---|---|---|
| Calculated % | C, | 52.49; | H, | 5.35; | N, | 26.24 |
| Found % | C, | 52.24; | H, | 5.21; | N, | 26.15 |

Specific rotation [α]_{D}²⁰ -127.3° (c = 0.1, DMSO)

The compounds of Reference examples 125 through 126 were obtained in the same manner as in Reference example 124.

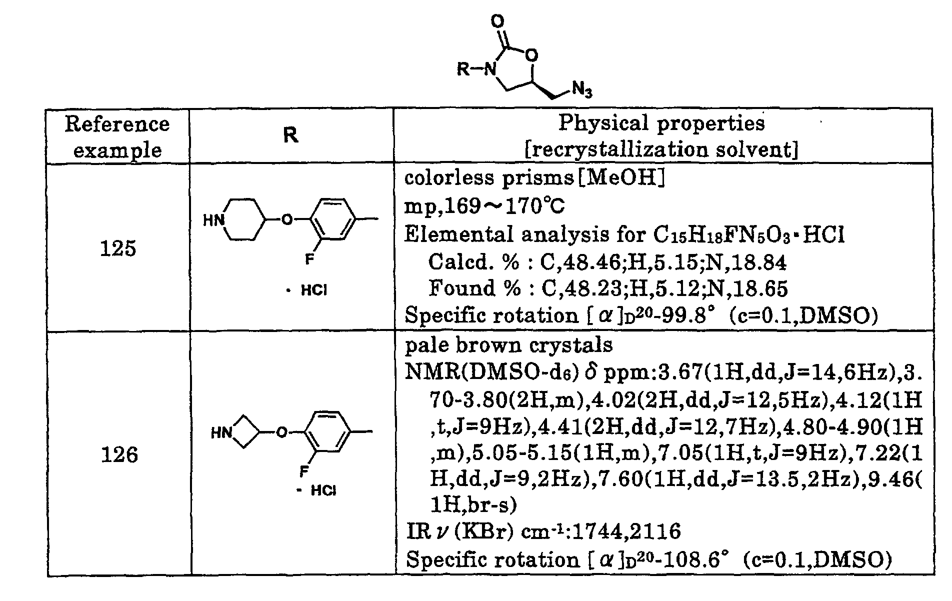

### Reference example 127

(R)-5-Azidomethyl-3-[4-(4-ethylpiperazin-1-yl)-3-fluorophenyl]-2-oxo-oxazolidine

To a solution of 5.00 g of (R)-5-azidomethyl-3-[3-fluoro-4-(piperazin-1-yl)phenyl]-2-oxooxazolidine and 2.16 g of potassium carbonate in 50 ml of dried N,N-dimethylformamide, 1.40 ml of ethyl iodide was added dropwise at room temperature, and the mixture was stirred at room temperature for 3 hours. The reaction solution was added with water, and extracted with ethyl acetate. The extract was washed with saturated brine, and dried over sodium sulfate, and then the solvent was evaporated under reduced pressure to obtain 4.53 g of pale brown crystals. Recrystallization from a mixture of ethyl acetate and n-heptane gave colorless crystals having the melting point of from 90 to 91°C.

| Elemental analysis for C₁₆H₂₁FN₆O₂ | | | | | | |
|---|---|---|---|---|---|---|
| Calculated % | C, | 55.16; | H, | 6.08; | N, | 24.12 |
| Found % | C, | 55.22; | H, | 6.20; | N, | 24.03 |

Specific rotation [α]_{D}²⁰ -120.9° (c = 0.1, DMSO)

The compounds of Reference examples 128 through 136 were obtained in the same manner as in Reference example 127.

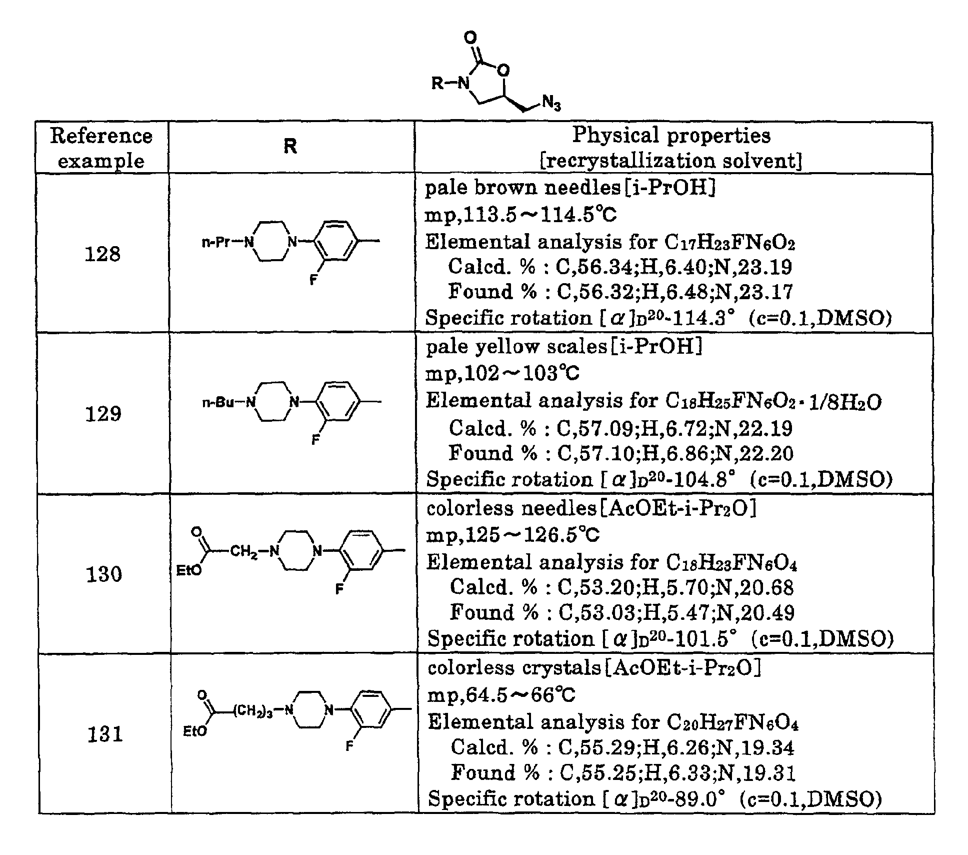

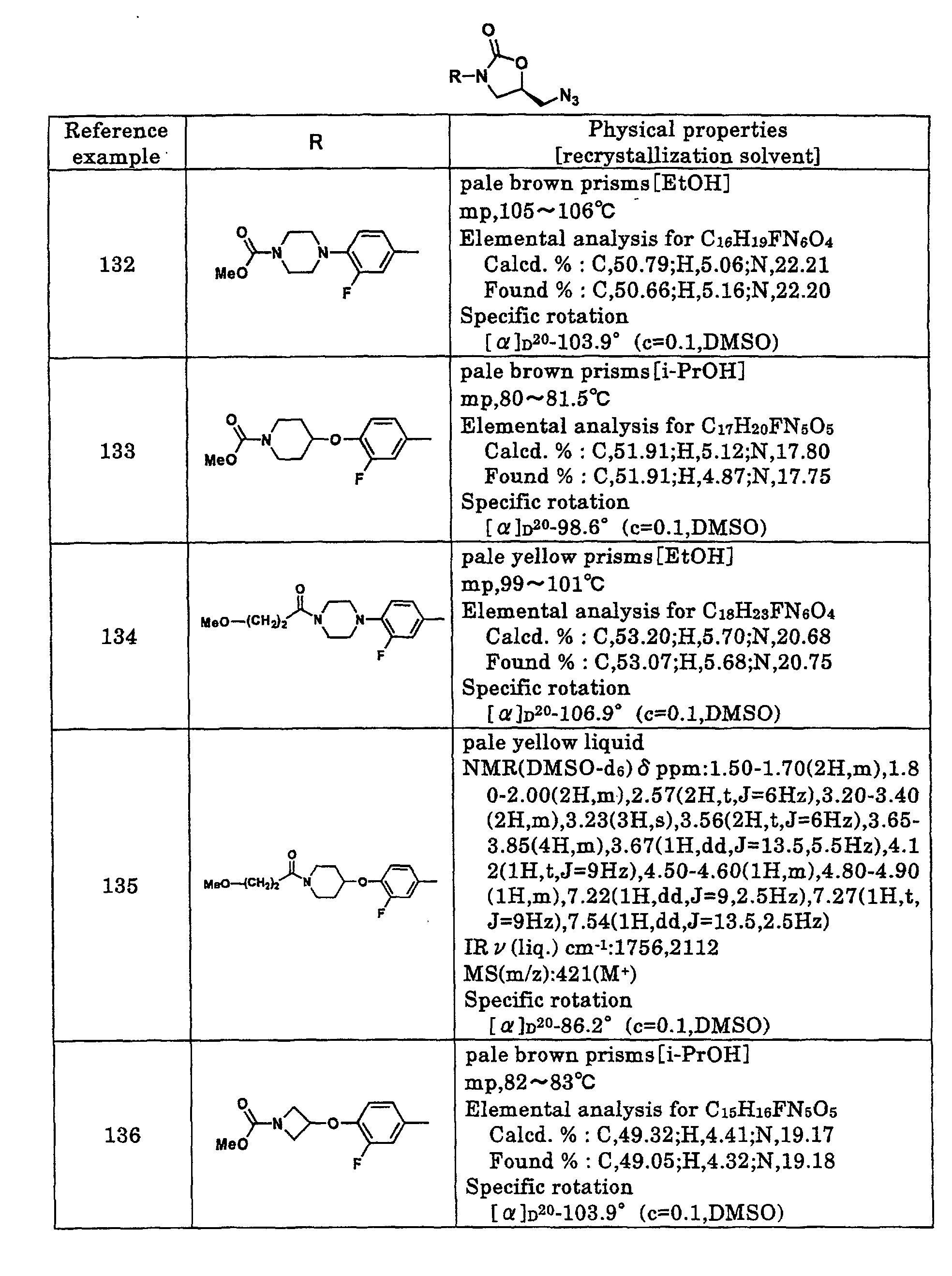

### Reference example 137

Ethyl (R)-3-[4-[4-(5-azidomethyl-2-oxooxazolidin-3-yl)-2-fluorophenyl]-piperazin-1-yl]propionate

A solution of 7.00 g of (R)-5-azidomethyl-3-[3-fluoro-4-(piperazin-1-yl)-phenyl]-2-oxooxazolidine and 3.56 ml of ethyl acrylate_ in 70 ml of ethanol was heated under reflux for 1 hour. The solvent was evaporated under reduced pressure, and the residue was purified by column chromatography (silica gel, diethyl ether) to obtain 7.50 g of colorless crystals. Recrystallization from isopropanol gave colorless crystals having the melting point of from 82 to 83°C.

| Elemental analysis for C₁₉H₂₅FN₆O₄ | | | | | | |
|---|---|---|---|---|---|---|
| Calculated % | C, | 54.28; | H, | 5.99; | N, | 19.99 |
| Found % | C, | 53.99; | H, | 5.88; | N, | 19.97 |

Specific rotation [α]_{D}²⁰ -95.0° (c = 0.1, DMSO)

The compounds of Reference examples 138 through 139 were obtained in the same manner as in Reference example 137.

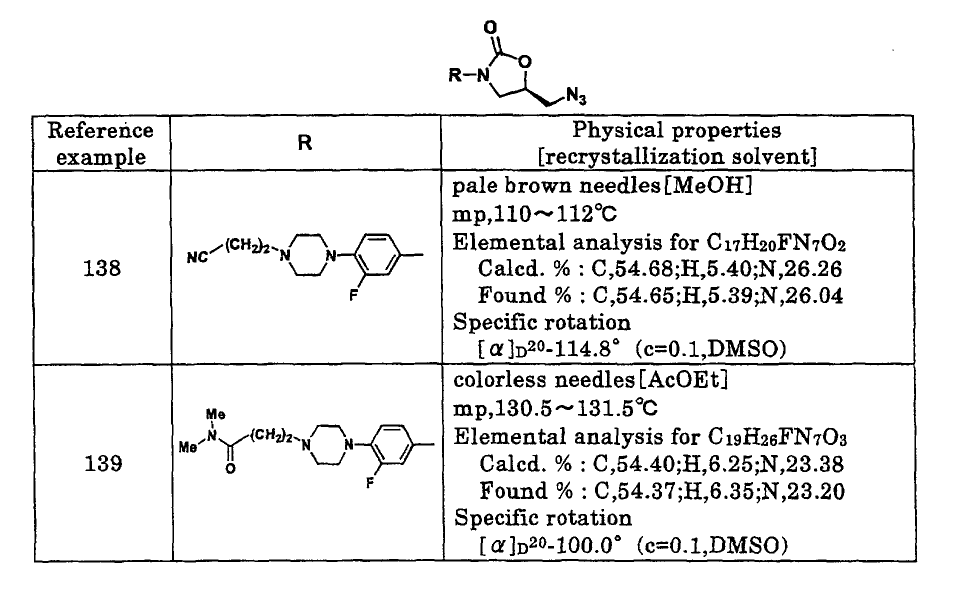

### Reference example 140

(R)-5-Azidomethyl-3-[3-fluoro-4-[4-(3-phthalimidopropyl)piperazin-1-yl]-phenyl]-2-oxooxazolidine

To a suspension of 5.00 g of (R)-5-azidomethyl-3-[3-fluoro-4-(piperazin-1-yl)phenyl]-2-oxooxazolidine and 2.16 g of potassium _ carbonate in 110 ml of dried N,N-dimethyformamide, 4.60 g of N-(3-bromopropyl)phthalimide was added, and the mixture was stirred with heating at an outer temperature of 50°C for 3 hours. The reaction solution was added with water, and extracted with ethyl acetate. The extract was washed with saturated brine, and dried over sodium sulfate, and then the solvent was evaporated under reduced pressure. The residue was washed with diisopropyl ether to obtain 6.54 g of pale brown crystals. Recrystallization from ethyl acetate gave colorless needles having the melting point of from 153 to 154.5°C.

| Elemental analysis for C₂₅H₂₆FN₇O₄ | | | | | | |
|---|---|---|---|---|---|---|
| Calculated % | C, | 59.16; | H, | 5.16; | N, | 19.32 |
| Found % | C, | 58.99; | H, | 5.02; | N, | 19.29 |

Specific rotation [α]_{D}²⁰ -95.3° (c = 0.1, DMSO)

The compound of Reference example 141 was obtained in the same manner as in Reference example 140.

### Reference example 141

(R)-5-Azidomethyl-3-[3-fluoro-4-[4-(2-phthalimidoethyl)piperazin-1-yl]-phenyl]-2-oxooxazolidine
Appearance : pale yellow crystals (recrystallization solvent : DMF-H₂O)
Melting point : 210.5 to 212°C

| Elemental analysis for C₂₄H₂₄FN₇O₄ | | | | | | |
|---|---|---|---|---|---|---|
| Calculated % | C, | 58.41; | H, | 4.90; | N, | 19.87 |
| Found % | C, | 58.04; | H, | 4.67; | N, | 19.72 |

Specific rotation [α]_{D}²⁰ -95.1° (c = 0.1, DMSO)

### Reference example 142

(R)-3-[4-[4-(3-Aminopropyl)piperazin-1-yl]-3-fluorophenyl]-5-azidomethyl-2-oxooxazolidine

To a solution of 6.24 g of (R)-5-azidomethyl-3-[3-fluoro-4-[4-(3-phthalimidopropyl)piperazin-1-yl]phenyl]-2-oxooxazolidine in 60 ml of ethanol, 0.66 ml of hydrazine hydrate was added, and the mixture was heated under reflux for 4 hours. The reaction solution was added with water, and acidified with 10 % hydrochloric acid, and the aqueous layer was washed with ethyl acetate. The aqueous layer was alkalified with aqueous sodium hydroxide, and extracted with ethyl acetate. The extract was washed with saturated brine, and dried over sodium sulfate, and then the solvent was evaporated under reduced pressure to obtain 4.35 g of a pale brown liquid.
NMR spectrum (DMSO-d₆) δ ppm:
1.55(2H,quin,J=7Hz),2,37(2H,t,J=7Hz),2.51(4H,t,J=4.5Hz),2.62(2H,t,J=7Hz), 2.99(4H,t,J=4.5Hz),3.67(1H,dd,J=13.5,5.5Hz),3.70-3.80(2H,m),4.10(1H,t,J=9Hz), 4.80-4.90(1H,m),7.04(1H,t,J=9Hz),7.18(1H,dd,J=9,2.5Hz),7.46(1H,dd,J=15,2.5Hz)
IR spectrum ν (liq.) cm⁻¹: 1752,2112
Mass spectrum (m/z) : 377(M⁺)
Specific rotation [α]_{D}²⁰ -116.9° (c = 0.1, DMSO)

### Reference example 143

(R)-3-[4-[4-(3-Acetylaminopropyl)piperazin-1-yl]-3-fluorophenyl]-5-azidomethyl-2-oxooxazolidine

To a solution of 2.00 g of (R)-3-[4-[4-(3-aminopropyl)piperazin-1-yl]-3-fluorophenyl]-5-azidomethyl-2-oxooxazolidine in 20 ml of pyridine, 1.50 ml of acetic anhydride was added under ice-cooling and with stirring, and the mixture was stirred at the same temperature for 1 hour. The solvent was evaporated under reduced pressure, and then the residue was added with aqueous sodium hydroxide, and the resulting alkaline mixture was extracted with ethyl acetate. The extract was washed with saturated brine, and dried over sodium sulfate, and then the solvent was evaporated under reduced pressure. The residue was purified by column chromatography (alumina, ethyl acetate to ethyl acetate : methanol = 20 : 1) to obtain 1.63 g of a pale brown liquid.
NMR spectrum (DMSO-d₆) δ ppm:
1.57(2H,quin,J=7.5Hz),1.79(3H,s),2.34(2H,t,J=7.5Hz),2.51(4H,t,J=5Hz), 2.99(4H,t,J=5Hz),3.00-3.10(2H,m),3.67(1H,dd,J=13.5,5.5Hz),3.70-3.80(2H,m), 4.10(1H,t,J=9Hz),4.80-4.90(1H,m),7.05(1H,t,J=9Hz),7.18(1H,dd,J=9,2.5Hz), 7.46(1H,dd,J=15,2.5Hz),7.68(1H,br-s)
Mass spectrum (m/z) : 419(M⁺)
Specific rotation [α]_{D}²⁰ -96.2° (c = 0.1, DMSO)

### Reference example 144

(R)-5-Azidomethyl-3-[3-fluoro-4-[4-(3-mesylaminopropyl)piperazin-1-yl]-phenyl]-2-oxooxazolidine

To a solution of 0.90 g of (R)-3-[4-[4-(3-aminopropyl)piperazin-1-yl]-3-fluorophenyl]-5-azidomethyl-2-oxooxazolidine in 20 ml of dried tetrahydrofuran, 0.21 ml of methanesulfonyl chloride was added under ice-cooling and with stirring, and the mixture was stirred under ice-cooling for 2 hours. The reaction solution was alkalified with aqueous sodium hydroxide, and extracted with ethyl acetate. The extract was washed with saturated brine, and dried over sodium sulfate, and then the solvent was evaporated under reduced pressure. The residue was purified by column chromatography (alumina, ethyl acetate : n-heptane = 1 : 1) to obtain 0.62 g of pale brown crystals.
NMR spectrum (DMSO-d₆) δ ppm:
1.65(2H,quin,J=7Hz),2.39(2H,t,J=7Hz),2.52(4H,t,J=4.5Hz),2.88(3H,s), 2.99(4H,t,J=4.5Hz),3.00(2H,t,J=7Hz),3.67(1H,dd,J=13.5,5.5Hz),3.70-3.80(2H,m), 4.10(1H,t,J=9Hz),4.80-4.90(1H,m),6.87(1H,t,J=5.5Hz),7.05(1H,t,J=9Hz), 7.18(1H,dd,J=9,2.5Hz),7.46(1H,dd,J=14.5,2.5Hz)
IR spectrum ν (KBr) cm⁻¹: 1734,2112
Mass spectrum (m/z) : 455(M⁺)
Specific rotation [α]_{D}²⁰ -93.3° (c = 0.1, DMSO)

### Reference example 145

(R)-5-Azidomethyl-3-[3-fluoro-4-(1-oxidothiomorpholin-4-yl)phenyl]-2-oxo-oxazolidine

To a solution of 5.30 g of sodium metaperiodate in 56 ml of water, a mixture of 8.00 g of (R)-5-azidomethyl-3-[3-fluoro-4-(thiomorpholin-4-yl)phenyl]-2-oxooxazolidine in 80 ml of acetonitrile and 80 ml of methanol was added dropwise, and the mixture was stirred at room temperature for 18 hours. The reaction solution was added with water, and extracted with 1,2-dichloroethane. The extract was washed with saturated brine, and dried over sodium sulfate, and then the solvent was evaporated under reduced pressure to obtain 7.98 g of pale brown crystals. Recrystallization from isopropanol gave colorless prisms having the melting point of from 123.5 to 125°C.

| Elemental analysis for C₁₄H₁₆FN₅O₃S | | | | | | |
|---|---|---|---|---|---|---|
| Calculated % | C, | 47.58; | H, | 4.56; | N, | 19.82 |
| Found % | C, | 47.58; | H, | 4.56; | N, | 19.69 |

Specific rotation [α]_{D}²⁰ -114.1° (c = 0.1, DMSO)

### Reference example 146

(R)-5-Azidomethyl-3-[3-fluoro-4-(1,1-dioxidothiomorpholin-4-yl)phenyl]-2-oxo oxazolidine

To a suspension of 5.00 g of (R)-5-azidomethyl-3-[3-fluoro-4-(thiomorpholin-4-yl)phenyl]-2-oxooxazolidine in 25 ml of water and 75 ml of acetone, 10 ml of 50 % aqueous 4-methylmorpholine-N-oxide and 3.77 g of osmium tetroxide were added under stirring at room temperature, and the mixture was stirred at the same temperature for 10 minutes. The reaction solution was added with water, and extracted with 1,2-dichloroethane. The extract was washed with saturated brine and dried over sodium sulfate, and then the solvent was evaporated under reduced pressure to obtain 4.71 g of pale brown crystals. Recrystallization from acetone gave pale brown prisms having the melting point of from 146 to 148°C.

| Elemental analysis for C₁₄H₁₆FN₅O₄S | | | | | | |
|---|---|---|---|---|---|---|
| Calculated % | C, | 45.52; | H, | 4.37; | N, | 18.96 |
| Found % | C, | 45.63; | H, | 4.32; | N, | 18.84 |

Specific rotation [α]_{D}²⁰ -108.8° (c = 0.1, DMSO)

### Reference example 147

(R)-1-[4-[4-(5-Azidomethyl-2-oxooxazolidin-3-yl)-2-fluorophenyl]piperazine]-carbothioamide
1) To a solution of 5.00 g of (R)-5-azidomethyl-3-[3-fluoro-4-(piperazin-1-yl)phenyl]-2-oxooxazolidine and 2.60 ml of triethylamine in 40 ml of dried tetrahydrofuran, 1.40 ml of thiophosgene was added dropwise under ice-cooling, and the mixture was stirred at the same temperature for 30 minutes. The reaction solution was added with water, and extracted with ethyl acetate. The extract was washed with saturated brine, and dried over sodium sulfate, and then the solvent was evaporated under reduced pressure to obtain 4.32 g of (R)-5-azidomethyl-3-[4-(4-chlorothiocarbonylpiperazin-1-yl)-3-fluorophenyl]-2-oxooxazolidine as brown crystals.
2) A solution of 4.32 g of the crystals obtained in 1) in 43 ml of dried tetrahydrofuran was stirred with bubbling of ammonia gas at room temperature for 3 hours. The reaction solution was concentrated under reduced pressure, and the residue was purified by column chromatography (silica gel, 1,2-dichloroethane : methanol = 20 : 1) to obtain 2.95 g of pale brown crystals.
   NMR spectrum (DMSO-d₆) δ ppm:
   2.99(4H,t,J=5Hz),3.66(1H,dd,J=13.5,5.5Hz),3.70-3.80(2H,m),3.91(4H,t,J=5Hz), 4.11(1H,t,J=9Hz),4.80-4.90(1H,m),7.09(1H,t,J=9Hz),7.20(1H,dd,J=9,2.5Hz), 7.39(2H,br-s),7.50(1H,dd,J=14.5,2.5Hz)
   IR spectrum ν (KBr) cm⁻¹: 1738,2108,3184,3286,3424
   Specific rotation [α]_{D}²⁰ -109.1° (c = 0.1, DMSO)

### Reference example 148

(R)-N,N-Dimethyl-1-[4-[4-(5-azidomethyl-2-oxooxazolidin-3-yl)-2-fluorophenyl]piperazine]carbothioamide

To a solution of 5.00 g of the crystals obtained by the method of Reference example 147-1) in 20 ml of dried tetrahtdrofuran, 10 ml of 50% aqueous dimethylamine was added at room temperature, and the mixture was stirred at the same temperature for 18 hours. The reaction solution was concentrated under reduced pressure, and the residue was washed successively with water and ethanol to obtain 4.25 g of pale brown crystals. Recrystallization from acetonitrile gave pale brown prisms having the melting point of from 160 to 162°C.

| Elemental analysis for C₁₇H₂₂FN₇O₂S | | | | | |
|---|---|---|---|---|---|
| Calculated % | C, | 50.11; | H, | 5.44; | N, 24.06 |
| Found % | C, | 50.38; | H, | 5.44; | N, 23.95 |

Specific rotation [α]_{D}²⁰ -101.7° (c = 0.1, DMSO)

### Reference example 149

O-Methyl (R)-1-[4-[4-(5-azidomethyl-2-oxooxazolidin-3-yl)-2-fluorophenyl]-piperazine]thiocarboxylate

A solution of 5.00 g of the crystals obtained by the method of Reference example 147-1) in 50 ml of methanol was stirred with heating to 60°C for 30 minutes, and then stirred at room temperature for 1 hour. The precipitated crystals were collected by filtration, and washed with diisopropyl ether to obtain 4.00 g of pale brown crystals. Recrystallization from methanol gave pale brown needles having the melting point of from 138 to 139°C.

| Elemental analysis for C₁₆H₁₉FN₆O₃S | | | | | | |
|---|---|---|---|---|---|---|
| Calculated % | C, | 48.72; | H, | 4.86; | N, | 21.31 |
| Found % | C, | 48.79; | H, | 4.84; | N, | 20.94 |

Specific rotation [α]_{D}²⁰ -105.4° (c = 0.1, DMSO)

The compounds of Reference examples 150 through 151 were obtained in the same manner as in Reference example 149.

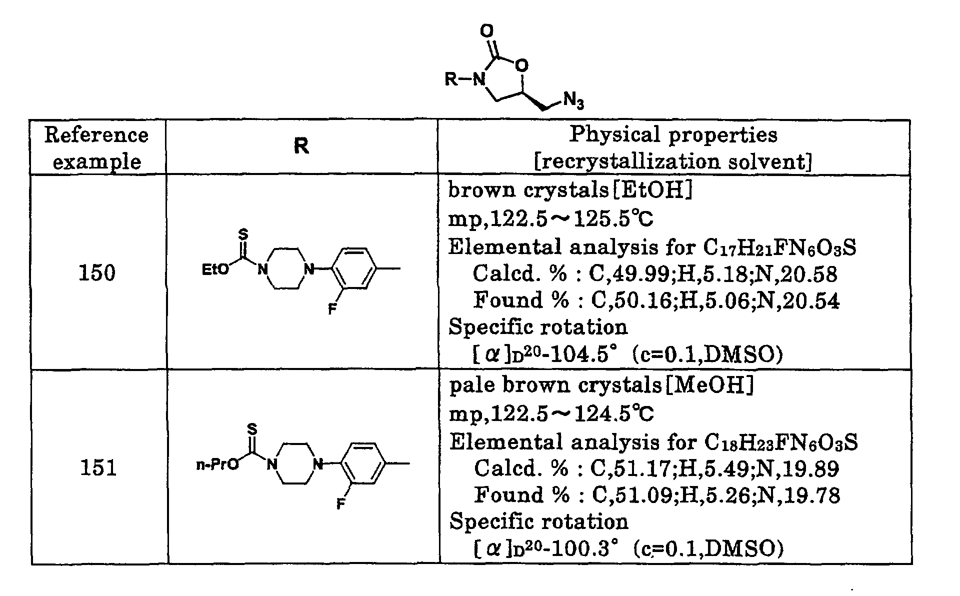

### Reference example 152

Methyl (R)-1-[4-[4-(5-azidomethyl-2-oxooxazolidin-3-yl)-2-fluorophenyl]-piperazine]carbodithioate

To a solution of 5.00 g of (R)-5-azidomethyl-3-[3-fluoro-4-(piperazin-1-yl)-phenyl]-2-oxooxazolidine and 2.20 ml of triethylamine in 50 ml of dried tetrahydrofuran, 2.00 ml of carbon disulfide was added dropwise under ice-cooling, and the mixture was stirred at room temperature for 18 hours. Furthermore, the mixture was added with 1.00 ml of methyl iodide under ice-cooling, and stirred at the same temperature for 30 minutes. The reaction solution was added with water, and extracted with ethyl acetate. The extract was washed with saturated brine, and dried over sodium sulfate, and then the solvent was evaporated under reduced pressure. The residue was washed with ethanol to obtain 5.96 g of colorless crystals. Recrystallization from acetonitrile gave pale brown prisms having the melting point of from 139.5 to 140°C.

| Elemental analysis for C₁₆H₁₉FN₆O₂S₂ | | | | | | |
|---|---|---|---|---|---|---|
| Calculated % | C, | 46.81; | H, | 4.67; | N, | 20.47 |
| Found % | C, | 46.96; | H, | 4.68; | N, | 20.41 |

Specific rotation [α]_{D}²⁰ -104.6° (c = 0.1, DMSO)

### Reference example 153

(R)-N-Methyl-1-[4-[4-(5-azidomethyl-2-oxooxazolidin-3-yl)-2-fluorophenyl]-piperazine]carbothioamide

To a solution of 3.00 g of (R)-5-azidomethyl-3-[3-fluoro-4-(piperazin-1-yl)-phenyl]-2-oxooxazolidine in 30 ml of dried tetrahydrofuran, 0.71 ml of methyl isothiocyanate was added under ice-cooling and with stirring, and the mixture was stirred at the same temperature for 1 hour. The reaction solution was added with water, and the precipitated crystals were collected by filtration, and then washed with diisopropyl ether to obtain 3.63 g of colorless crystals. Recrystallization from ethyl acetate gave colorless needles having the melting point of from 156.5 to 158°C.

| Elemental analysis for C₁₆H₂₀FN₇O₂S | | | | | | |
|---|---|---|---|---|---|---|
| Calculated % | C, | 48.84; | H, | 5.12; | N, | 24.92 |
| Found % | C, | 48.70; | H, | 5.09; | N, | 24.88 |

Specific rotation [α]_{D}²⁰ -111.7° (c = 0.1, DMSO)

The compounds of Reference examples 154 through 156 were obtained in the same manner as in Reference example 153.

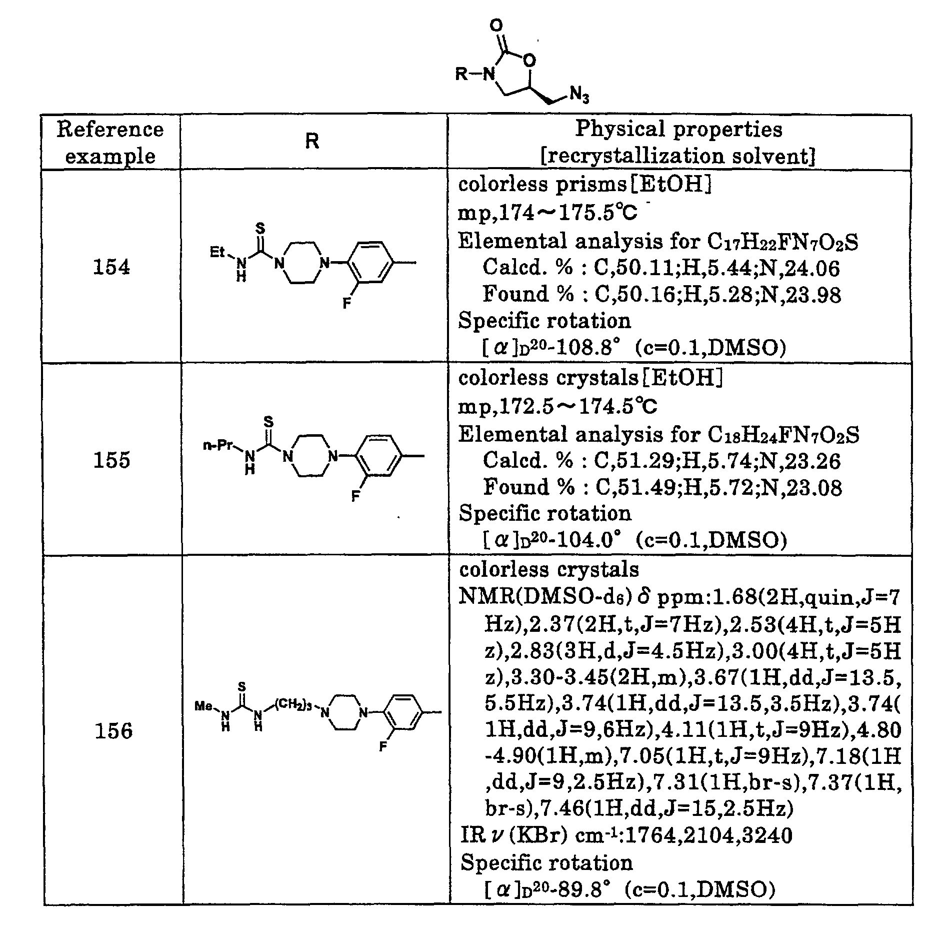

### Reference example 157

(R)-3-[4-[4-(5-Azidomethyl-2-oxooxazolidin-3-yl)-2-fluorophenyl]piperazin-1-yl]propyl isothiocyanate

To a solution of 2.00 g of (R)-3-[4-[4-(3-aminopropyl)piperazin-1-yl]-3-fluorophenyl]-5-azidomethyl-2-oxooxazolidine and 0.74 ml of triethylamine in 20 ml of dried tetrahydrofuran, 0.64 ml of carbon disulfide was added dropwise under ice-cooling, and the mixture was stirred at the same temperature for 5 hours. The mixture was added with 0.51 ml of ethyl chlorocarbonate, and further stirred at the same temperature for 1.5 hours. The reaction solution was added with water, and extracted with ethyl acetate. The extract was washed with saturated brine, and dried over sodium sulfate, and then the solvent was evaporated under reduced pressure. The residue was purified by column chromatography (silica gel, ethyl acetate) to obtain 0.85 g of a brown liquid.
NMR spectrum (DMSO-d₆) δ ppm :
1.84(2H,quin,J=6.5Hz),2.43(2H,t,J=6.5Hz),2.53(4H,t,J=4.5Hz),3.00(4H,t,J=4.5Hz), 3.60-3.80(5H,m),4.11(1H,t,J=9Hz),4.80-4.90(1H,m),7.05(1H,t,J=9Hz), 7.18(1H,dd,J=9,2.5Hz),7.46(1H,dd,J=14.5,2.5Hz)
IR spectrum ν (liq.) cm⁻¹: 1754,2112,2184
Mass spectrum (m/z) : 419(M⁺)
Specific rotation [α]_{D}²⁰ -89.1° (c = 0.1, DMSO)

### Reference example 158

Methyl (R)-N-[3-[4-[4-(5-azidomethyl-2-oxooxazolidin-3-yl)-2-fluorophenyl]-piperazin-1-yl]propyl]thiocarbamate

To 25 ml of dried methanol, 0.87 g of 60 % sodium hydride was added under ice-cooling and with stirring, and the mixture was stirred at room temperature for 30 minutes. Then, the mixture was added with a solution of 4.56 g of (R)-3-[4-[4-(5-azidomethyl-2-oxooxazolidin-3-yl)-2-fluorophenyl]piperazin-1-yl]propyl isothiocyanate in 30 ml of dried methanol-dried tetrahydrofuran (2 : 1), and stirred at room temperature for 1.5 hours. The reaction mixture was acidified with ice water and 10 % hydrochloric acid, and extracted with ethyl acetate. The extract was washed with saturated brine, and dried over sodium sulfate, and then the solvent was evaporated under reduced pressure. The residue was purified by column chromatography (silica gel, ethyl acetate : n-heptane = 2 : 1) to obtain 4.32 g of a brown liquid.
NMR spectrum (DMSO-d₆) δ ppm:
1.60-1.75(2H,m),2.39(2H,t,J=6.5Hz),2.53(4H,t,J=5Hz),3.02(4H,t,J=5Hz), 3.40-3.50(2H,m),3.65(1H,dd,J=13.5,5.5Hz),3.72(1H,dd,J=13.5,3.5Hz), 3.74(1H,dd,J=9,6Hz),3.87(3H,s),4.10(1H,t,J=9Hz),4.80-4.90(1H,m),7.03(1H,t,J=9Hz), 7.17(1H,dd,J=9,2.5Hz),7.41(1H,dd,J=15.5,2.5Hz),8.77(1H,br-s)
IR spectrum ν (liq.) cm⁻¹: 1748,2112,3284
Mass spectrum (m/z) : 451(M⁺)
Specific rotation [α]_{D}²⁰ -85.5° (c = 0.1, DMSO)

### Reference example 159

(R)-5-Azidomethyl-3-[3-fluoro-4-(4-oxopiperidin-1-yl)phenyl]-2-oxooxazolidine

A suspension of 1.45 g of (R)-5-azidomethyl-3-[4-(1,4-dioxa-8-azaspiro[4.5]-decan-8-yl)-3-fluorophenyl]-2-oxooxazolidine and 1.10 g of p-toluenesulfonic acid in 60 ml of acetone-water (1:1) was heated under reflux for 18 hours. The solvent was evaporated under reduced pressure, and the residue was neutralized with aqueous sodium hydrogencarbonate, and then extracted with ethyl acetate. The extract was washed with saturated brine, and dried over sodium sulfate, and then the solvent was evaporated under reduced pressure. The residue was purified by column chromatography (silica gel, ethyl acetate : n-heptane = 1 : 1) to obtain 1.20 g of colorless crystals. Recrystallization from ethanol gave colorless crystals having the melting point of from 99.5 to 101°C.

| Elemental analysis for C₁₅H₁₆FN₅O₃ | | | | | | |
|---|---|---|---|---|---|---|
| Calculated % | C, | 54.05; | H, | 4.84; | N, | 21.01 |
| Found % | C, | 54.02; | H, | 4.87; | N, | 21.18 |

Specific rotation [α]_{D}²⁰ -118.4° (c = 0.1, DMSO)

### Reference example 160

(R)-5-Azidomethyl-3-[3-fluoro-4-(4-hydroxyiminopiperidin-1-yl)phenyl]-2-oxo-oxazolidine

A suspension of 6.75 g of (R)-5-azidomethyl-3-[3-fluoro-4-(4-oxopiperidin-1-yl)phenyl]-2-oxooxazolidine, 1.55 g of hydroxylamine hydrochloride and 3.66 g of sodium acetate in 135 ml of methanol was stirred at room temperature for 1 hour. The reaction solution was added with water, and the precipitated crystals were collected by filtration, and then washed with diisopropyl ether to obtain 6.52 g of pale brown crystals. Recrystallization from ethyl acetate gave colorless prisms having the melting point of from 155 to 156°C.

| Elemental analysis for C₁₅H₁₇FN₆O₃ | | | | | | |
|---|---|---|---|---|---|---|
| Calculated % | C, | 51.72; | H, | 4.92; | N, | 24.13 |
| Found % | C, | 51.72; | H, | 4.81; | N, | 24.22 |

Specific rotation [α]_{D}²⁰ -131.7° (c = 0.1, DMSO)

### Reference example 161

(R)-5-Azidomethyl-3-[3-fluoro-4-(4-thiobenzoylpiperazin-1-yl)phenyl]-2-oxo-oxazolidine

To a solution of 2.00 g of (R)-5-azidomethyl-3-[3-fluoro-4-(piperazin-1-yl)-phenyl]-2-oxooxazolidine and 1.97 ml of triethylamine in 20 ml of dried 1,2-dichloroethane, 10.0 g of thiobenzoyl chloride was added dropwise under ice-cooling and with stirring, and the mixture was stirred at the same temperature for 1 hour. The reaction solution was added with water, and extracted with 1,2-dichloroethane. The extract was washed with saturated brine, and dried over sodium sulfate, and then the solvent was evaporated under reduced pressure. The residue was purified by column chromatography (silica gel, ethyl acetate : n-heptane = 1 : 4) to obtain 3.30 g of a green liquid.
NMR spectrum (CDCl₃) δ ppm:
3.04(2H,t,J=5Hz),3.27(2H,t,J=5Hz),3.59(1H,dd,J=13.5,4.5Hz), 3.71(1H,dd,J=13.5,4.5Hz),3.76(2H,t,J=5Hz),3.88(1H,dd,J=8.5,6Hz), 4.05(1H,t,J=8.5Hz),4.61(2H,t,J=5Hz),4.75-4.80(1H,m),6.94(1H,t,J=9Hz), 7.13(1H,d,J=9Hz),7.30-7.40(5H,m),7.48(1H,dd,J=14,2.5Hz)
Mass spectrum (m/z) : 440(M⁺)
Specific rotation [α]_{D}²⁰ -85.3° (c = 0.1, DMSO)

### Reference example 162

(S)-5-Aminomethyl-2-oxo-3-[4-(thiomorpholin-4-yl)phenyl]oxazolidine

A solution of 8.50 g of (R)-5-azidomethyl-2-oxo-3-[4-(thiomorpholin-4-yl)-phenyl]oxazolidine and 7.68 g of triphenylphosphine in 130 ml of dried tetrahydrofuran was stirred at room temperature for 15 hours. Furthermore, the mixture was added with 4.8 ml of water, and stirred with heating to 40°C for 14 hours. The reaction solution was cooled, then added with 100 ml of water, acidified with 10 % hydrochloric acid, and then extracted with diethyl ether. The aqueous layer was alkalified with potassium carbonate, and extracted with a mixture of dichloromethane-methanol (30 : 1). The extract was washed with saturated brine, and dried over sodium sulfate, and then the solvent was evaporated under reduced pressure to obtain 6.88 g of colorless crystals. Recrystallization from ethyl acetate gave colorless crystals having the melting point of from 119.5 to 121°C.

| Elemental analysis for C₁₄H₁₉N₃O₂S | | | | | | |
|---|---|---|---|---|---|---|
| Calculated % | C, | 57.31; | H, | 6.53; | N, | 14.32 |
| Found % | C, | 57.36; | H, | 6.45; | N, | 14.06 |

Specific rotation [α]_{D}²⁰ -35.9° (c = 0.1, DMSO)

The compounds of Reference examples 163 through 212 were obtained in the same manner as in Reference example 162.

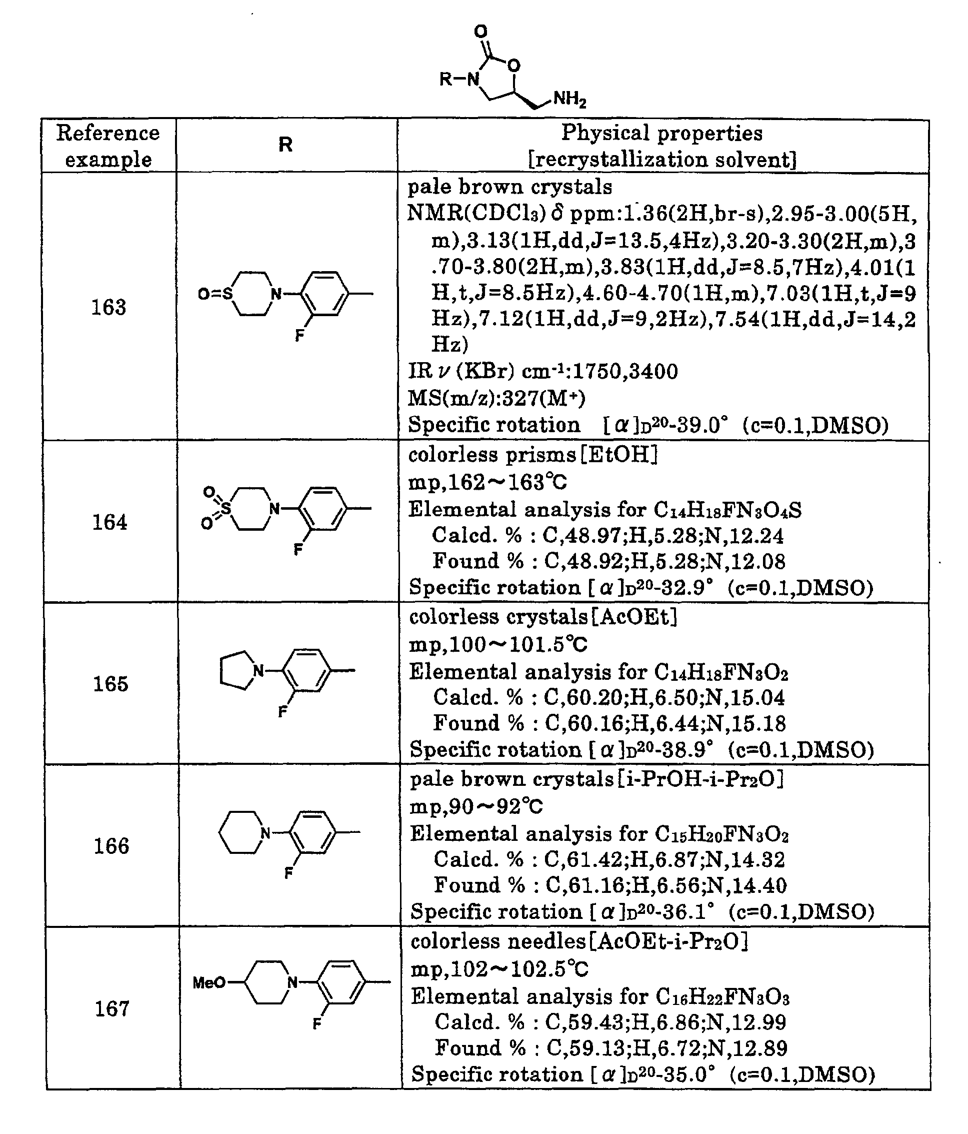

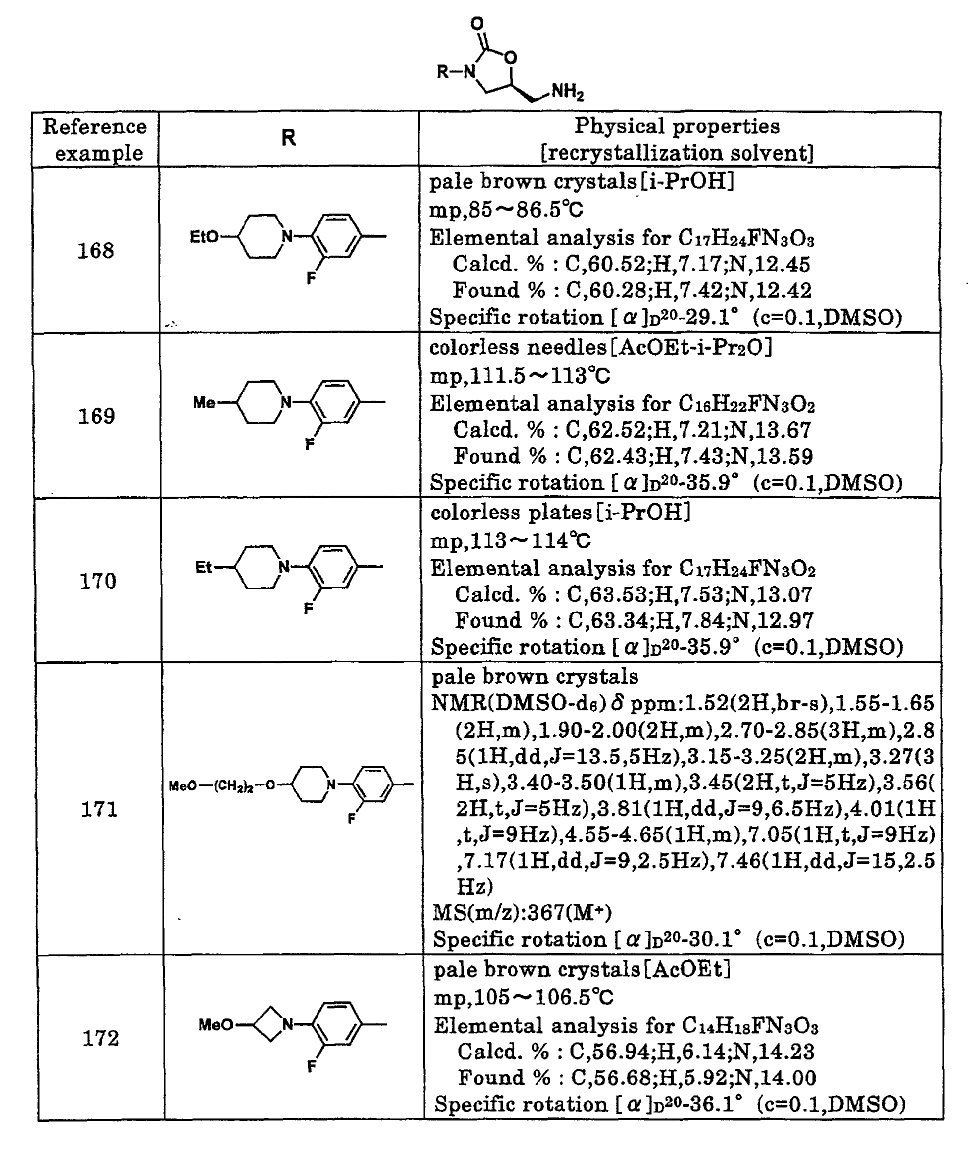

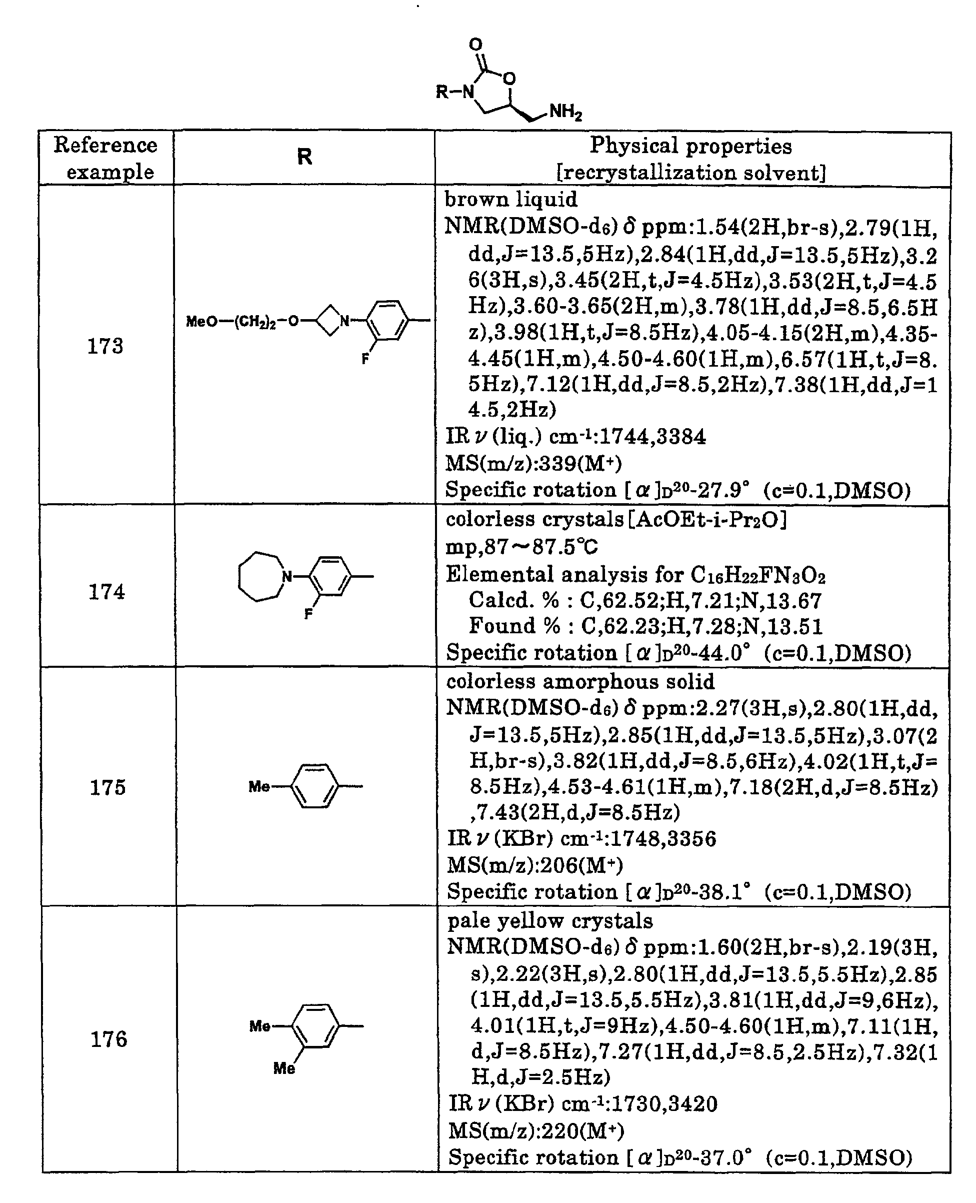

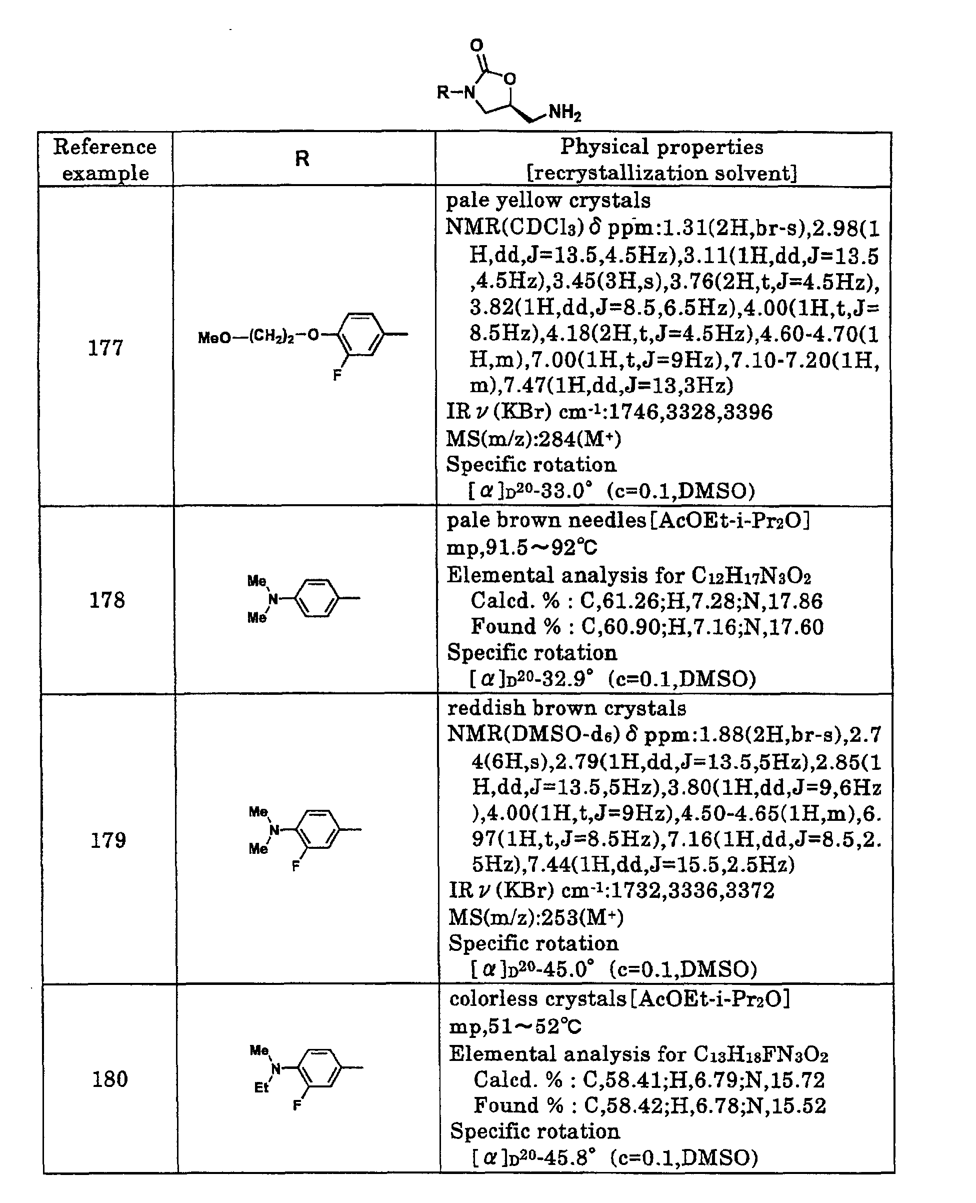

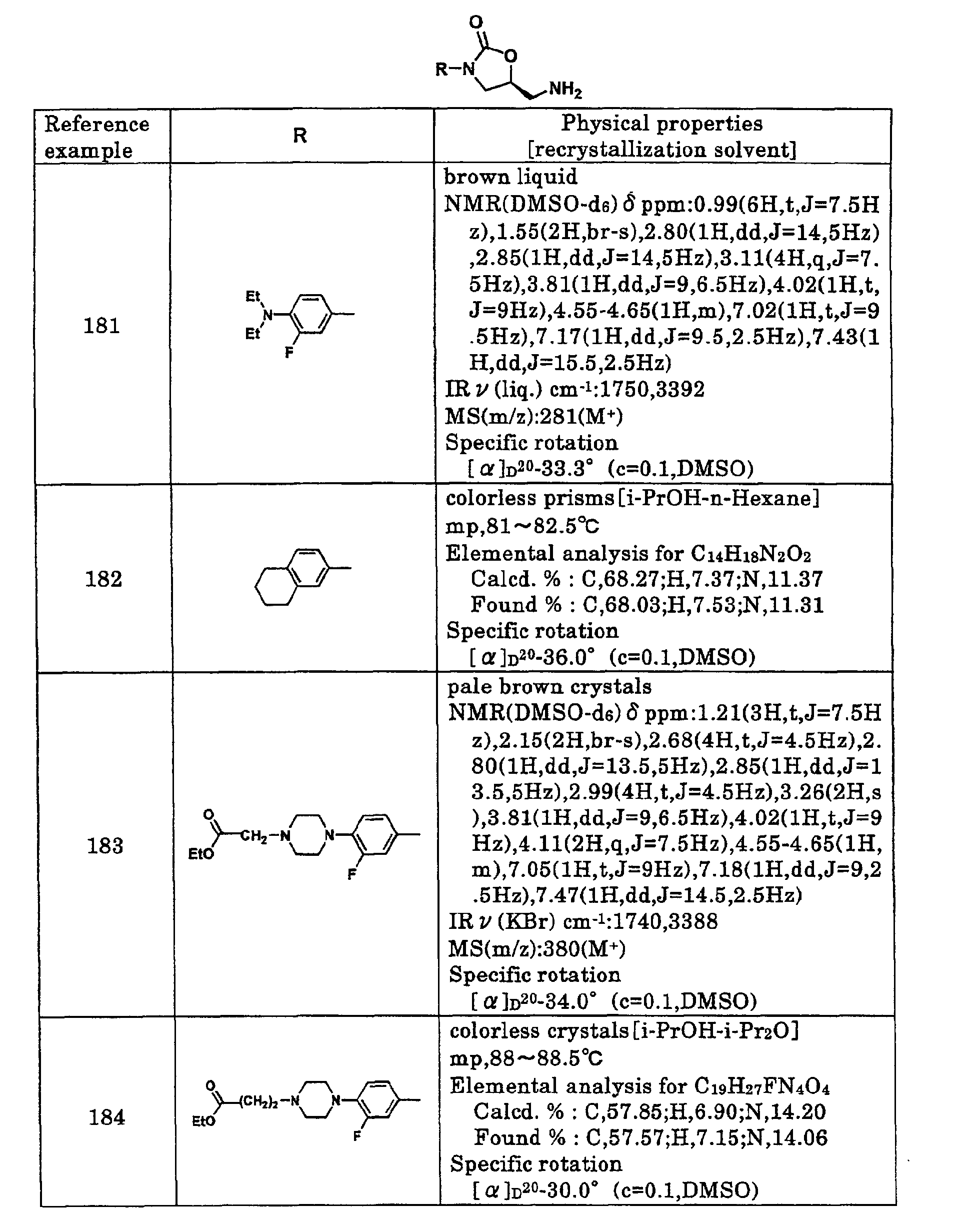

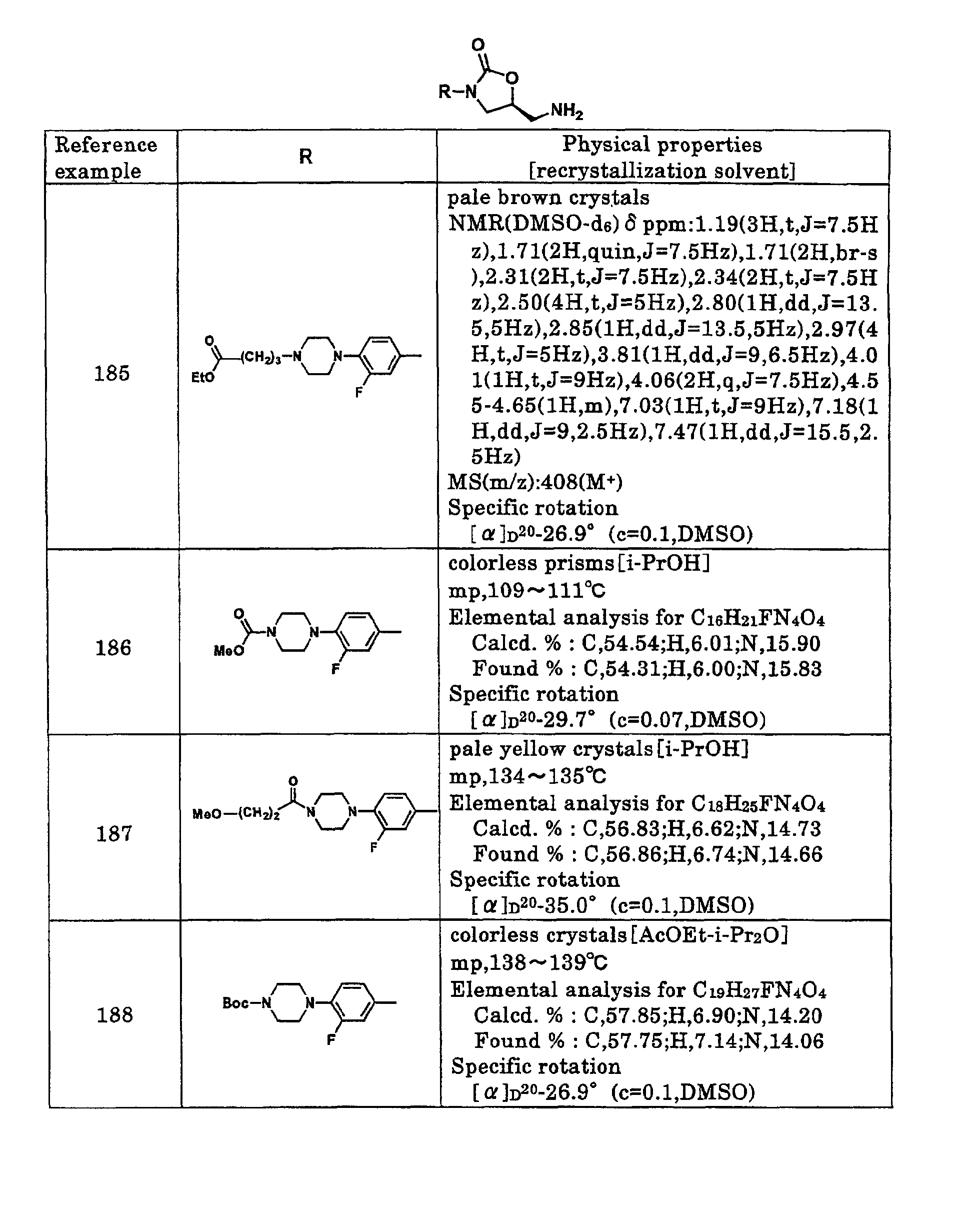

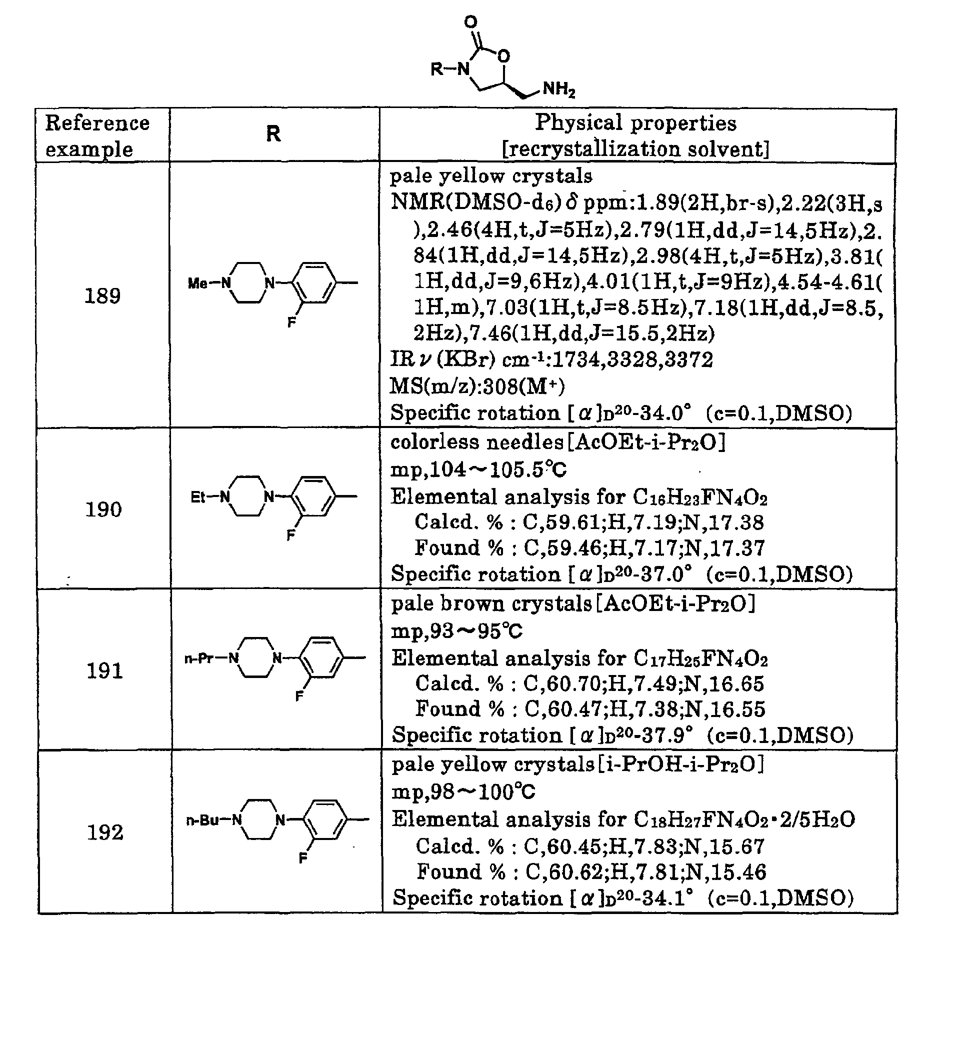

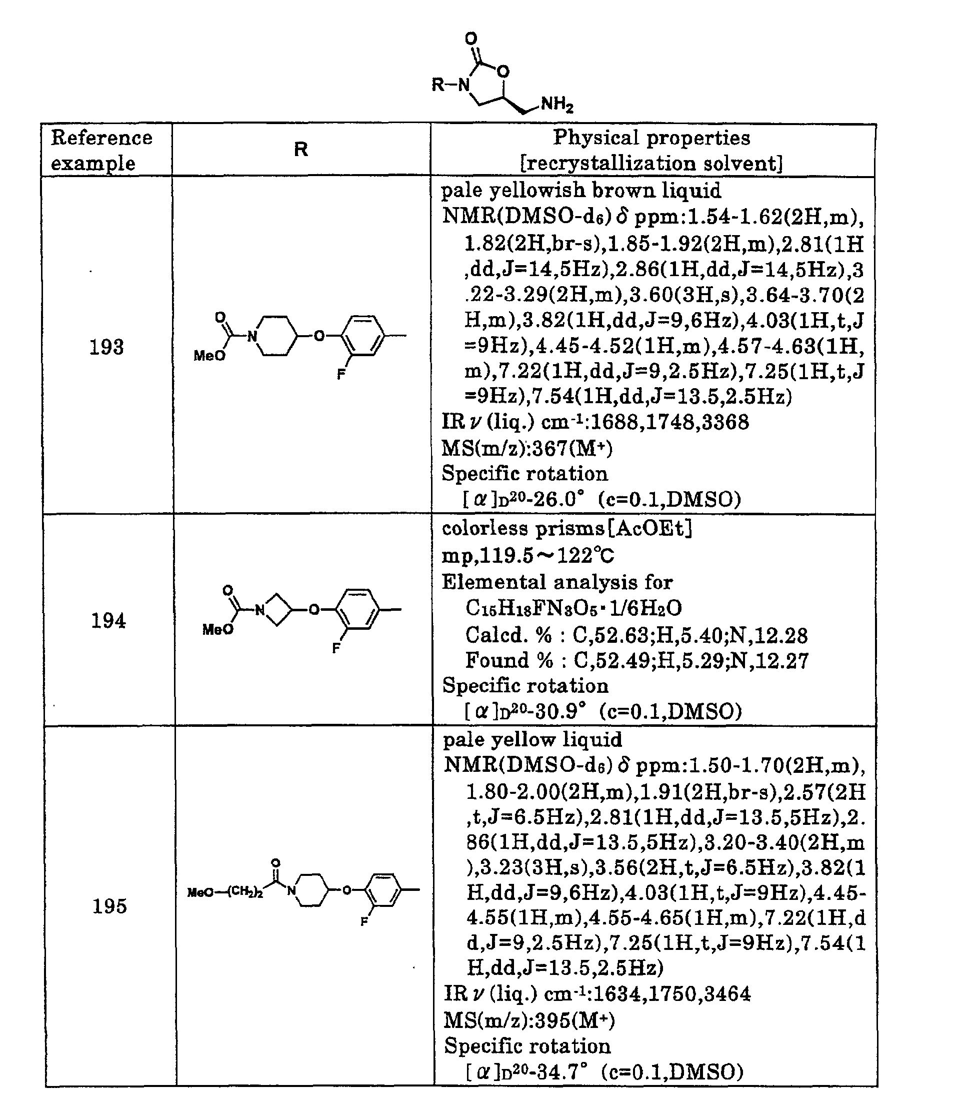

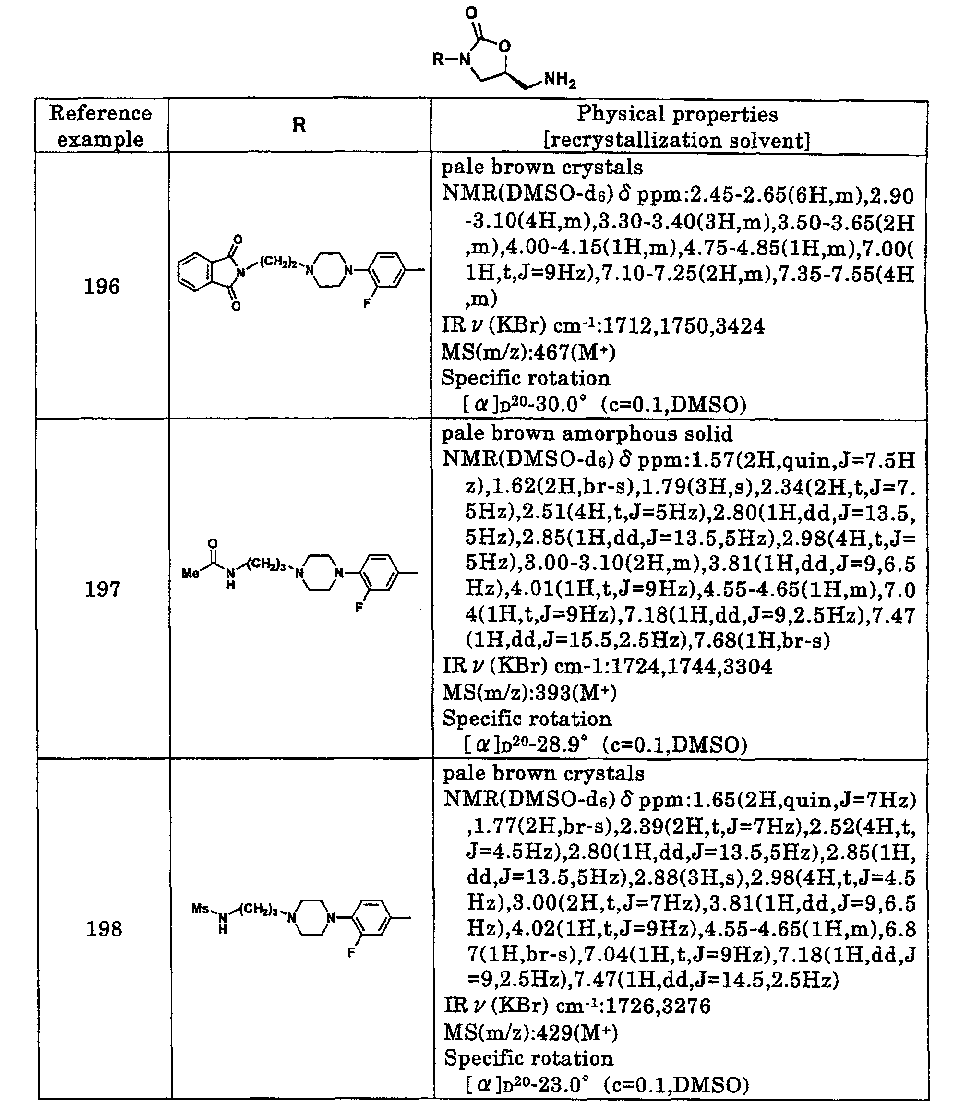

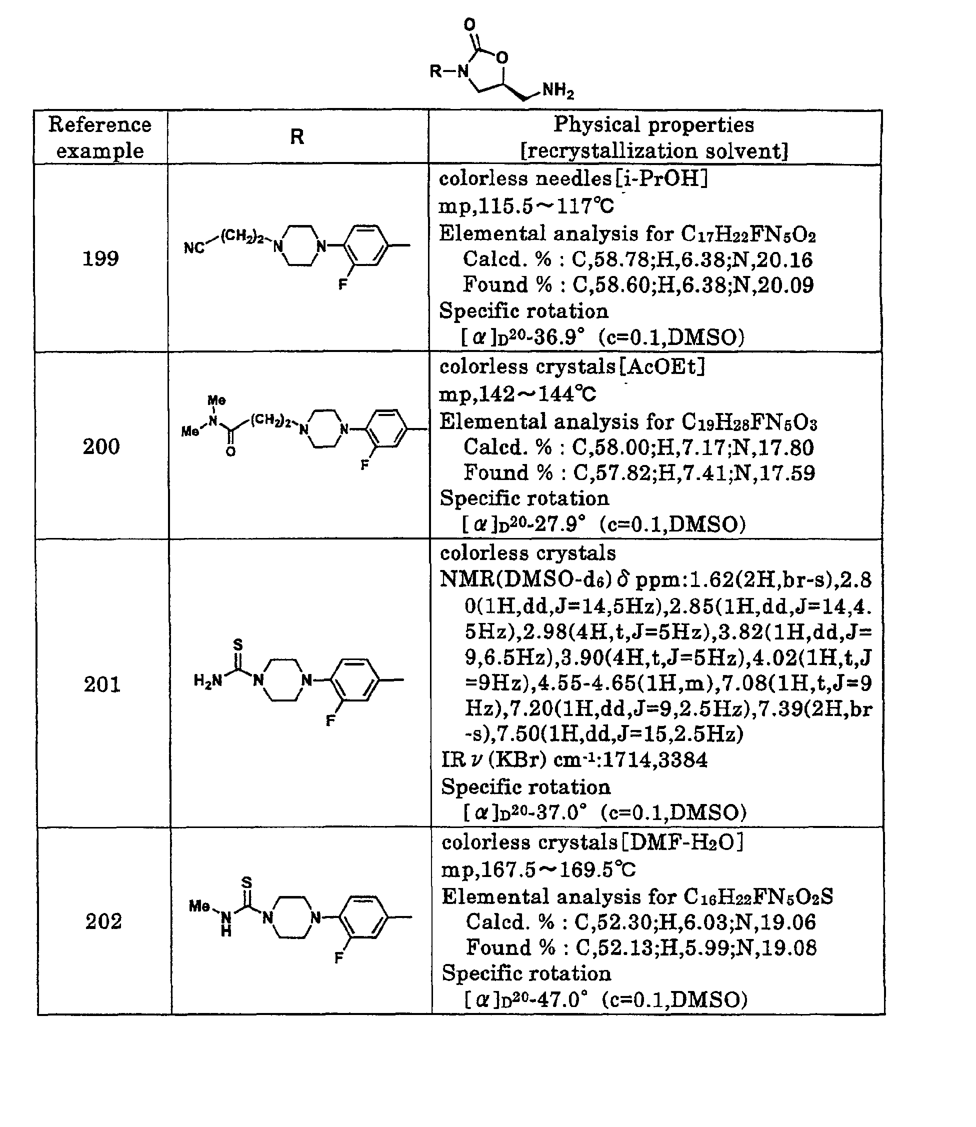

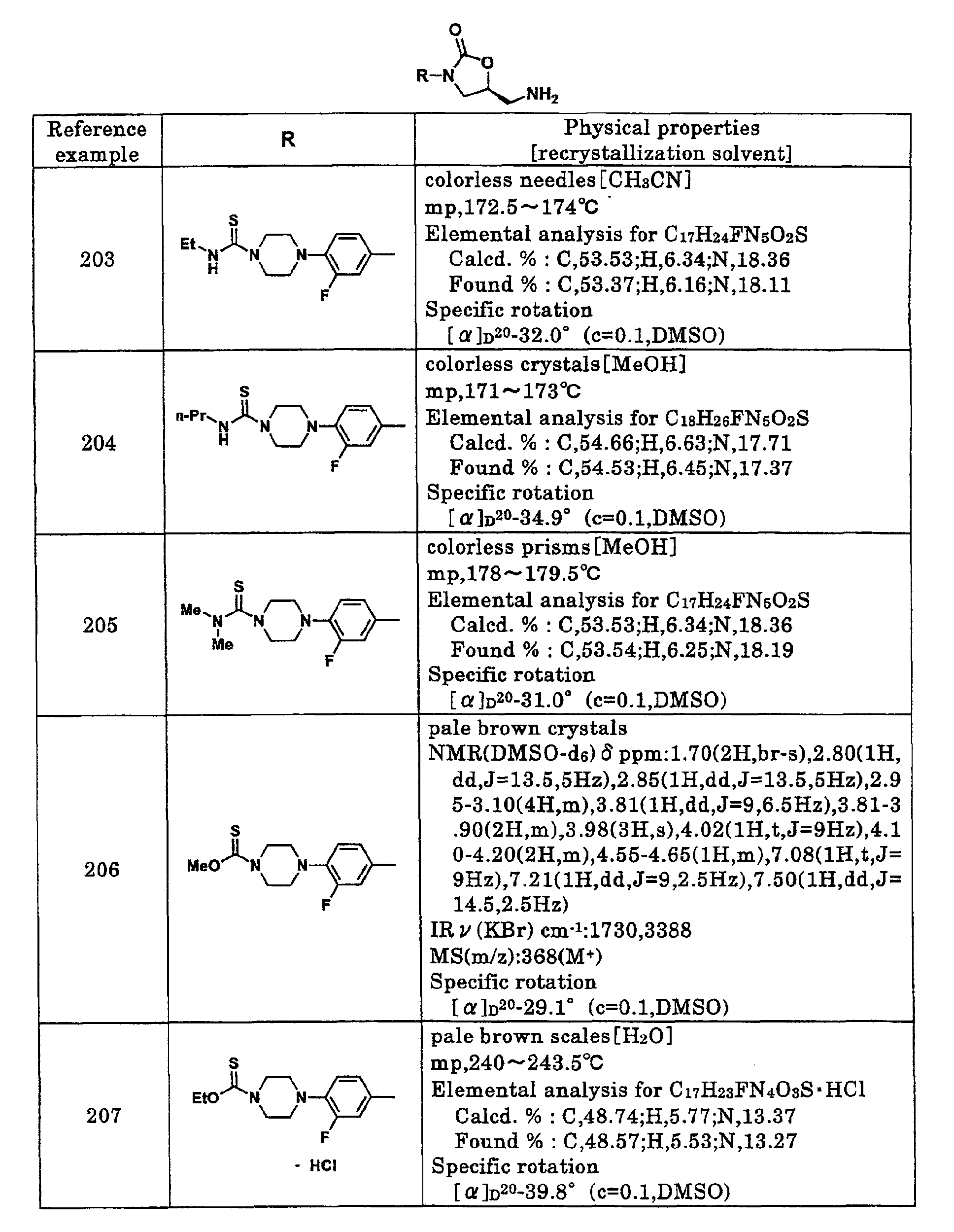

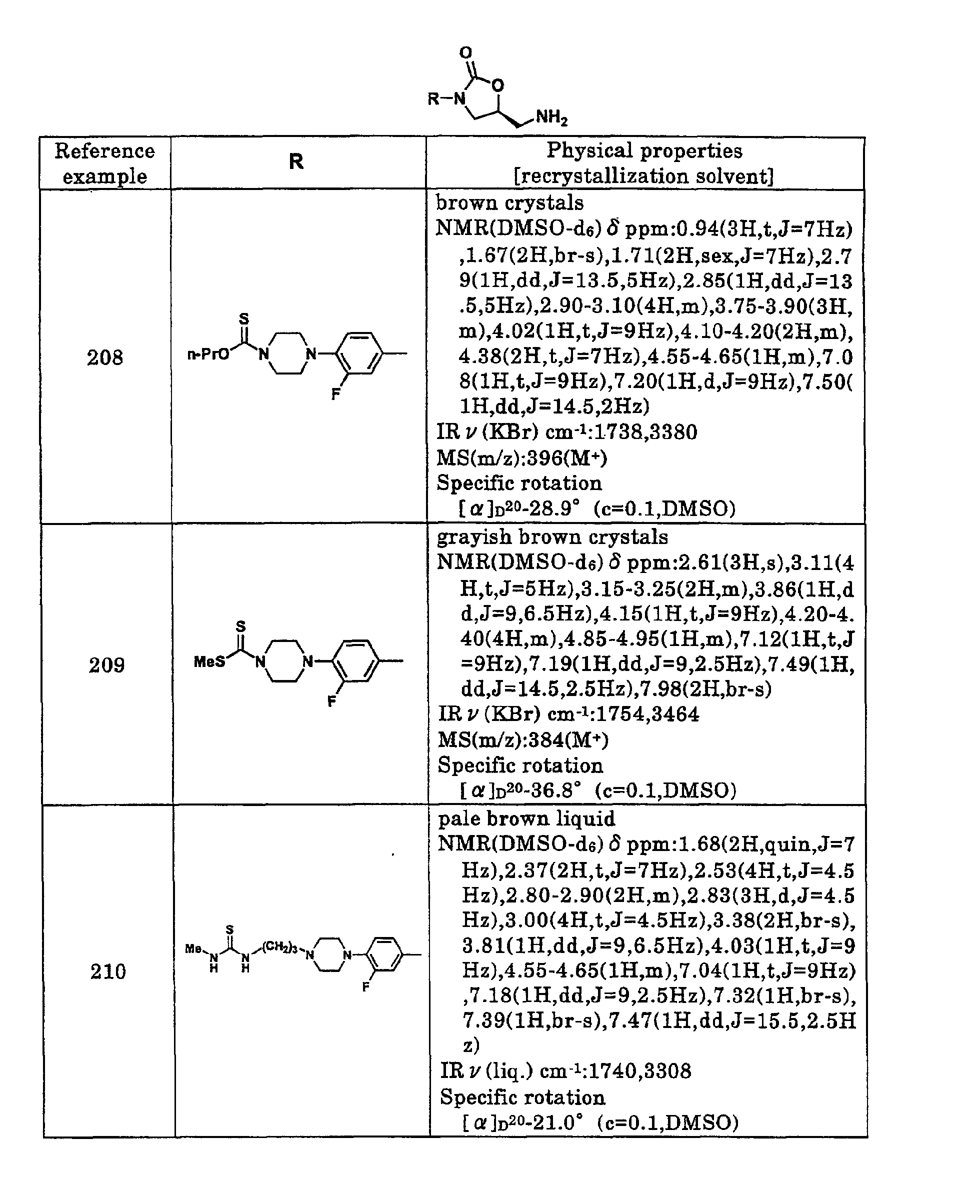

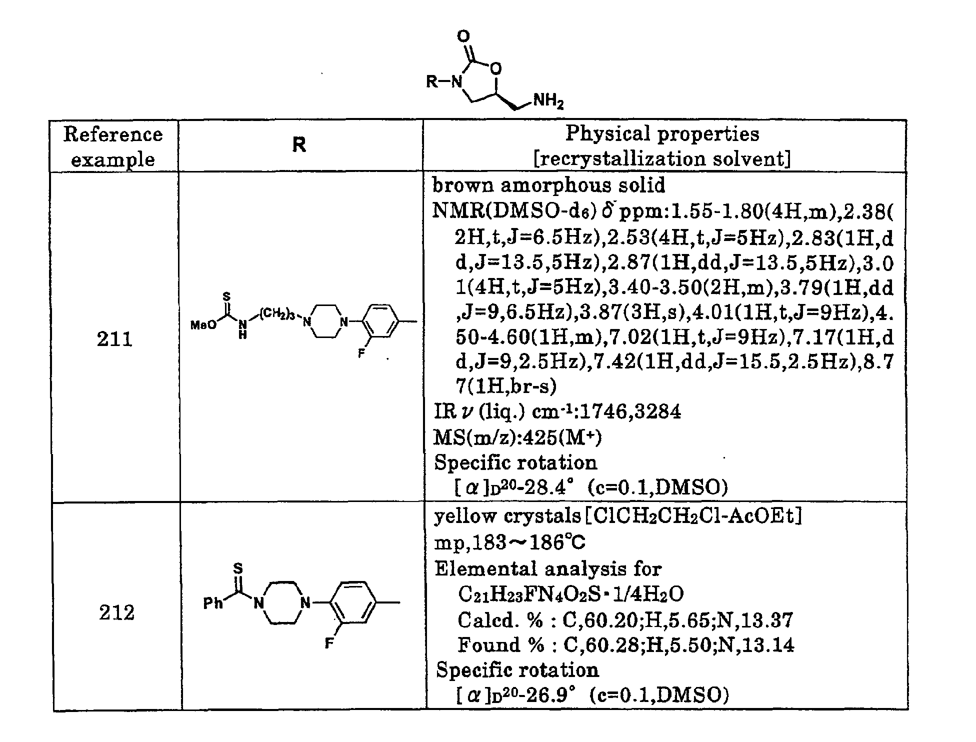

### Reference example 213

(S)-5-Aminomethyl-3-[4-(4-aminopiperidin-1-yl)-3-fluorophenyl]-2-oxooxazoli dine dihydrochloride

A suspension of 4.70 g of (S)-5-azidomethyl-3-[3-fluoro-4-(4-hydroxyiminopiperidin-1-yl)phenyl]-2-oxooxazolidine and 4.70 ml of Raney nickel in 95 ml of 10 % ammonia methanol solution was stirred at 40°C at a hydrogen pressure of under 30 atm for 6 hours. The catalyst was filtered off, and the filtrate was concentrated under reduced pressure. The residue was added with ethanol and 33% hydrogen chloride ethanol solution, and the mixture was stirred under ice-cooling for 1 hour. The precipitated crystals were collected by filtration to obtain 5.66 g of pale brown crystals.
NMR spectrum (DMSO-d₆) δ ppm:
1.65-1.80(2H,m),2.00-2.10(2H,m),2.70-2.80(2H,m),3.10-3.25(3H,m),3.30-3.40(2H,m), 3.89(1H,dd,J=9,6.5Hz),4.15(1H,t,J=9Hz),4.90-5.00(1H,m),7.10(1H,t,J=9Hz), 7.17(1H,dd,J=9,2.5Hz),7.45(1H,dd,J=14.5,2.5Hz),8.29(3H,br-s),8.47(3H,br-s)
IR spectrum ν (KBr) cm⁻¹: 1744,3440
Specific rotation [α]_{D}²⁰ -33.8° (c = 0.1, DMSO)

### Reference example 214

(R)-N-[2-Oxo-3-[4-(thiomorpholin-4-yl)phenyl]oxazolidin-5-yl]methyl isothiocyanate

To a mixture of 1.0 g of (S)-5-aminomethyl-2-oxo-3-[4-(thiomorpholin-4-yl)-phenyl]oxazolidine in 10 ml of benzene and 1 ml of N,N-dimethylformamide, 0.50 ml of triethylamine and 0.20 ml of carbon disulfide were added, and the mixture was stirred at room temperature for 6 hours. The reaction solution was concentrated under reduced pressure, and the residue was added with 10 ml of dichloromethane and 0.50 ml of triethylamine. The mixture was added with 0.35 ml of ethyl chlorocarbonate under ice-cooling and with stirring, and stirred at the same temperature for 30 minutes. The reaction solution was added with water, and the precipitated crystals were collected by filtration to obtain 0.98 g of colorless crystals. Recrystallization from a mixture of N,N-dimethylformamide-water gave colorless crystals having the melting point of from 194.5 to 195.5°C.

| Elemental analysis for C₁₅H₁₇N₃O₂S₂ | | | | | | |
|---|---|---|---|---|---|---|
| Calculated % | C, | 53.71; | H, | 5.11; | N, | 12.53 |
| Found % | C, | 53.53; | H, | 5.07; | N, | 12.54 |

Specific rotation [α]_{D}²⁰ -151.8° (c =0.1, DMSO)

The compounds of Reference examples 215 through 264 were obtained in the same manner as in Reference example 214.

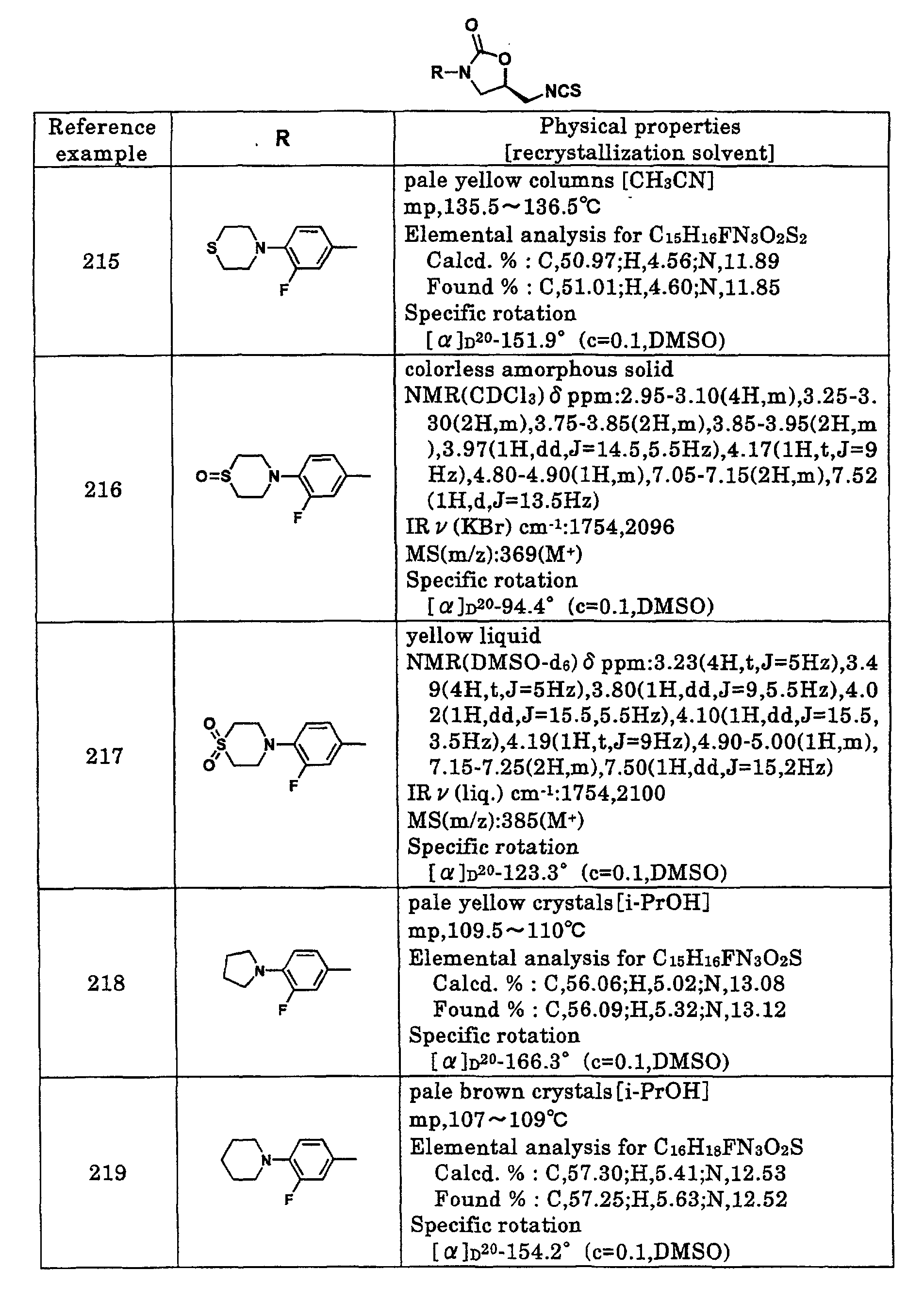

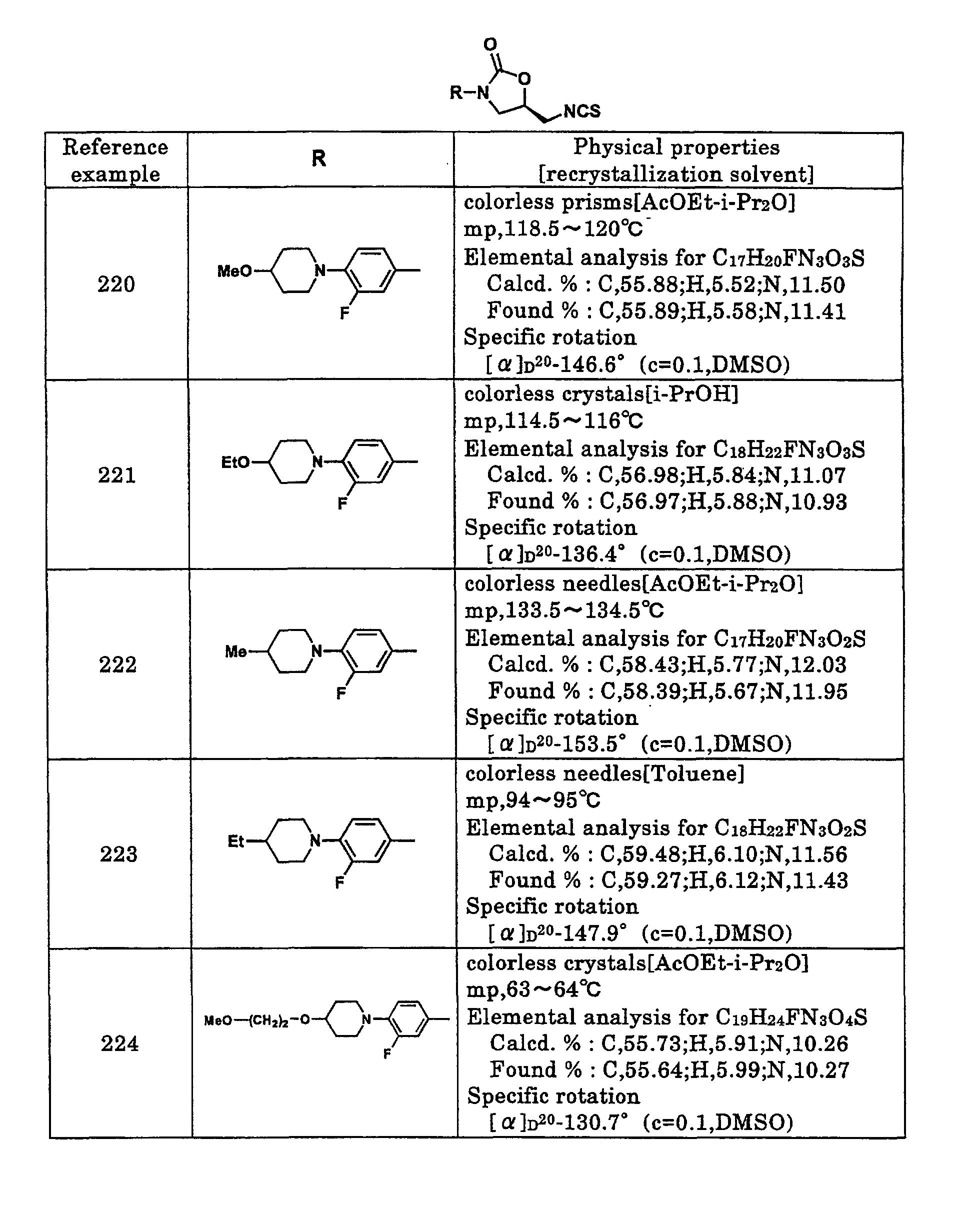

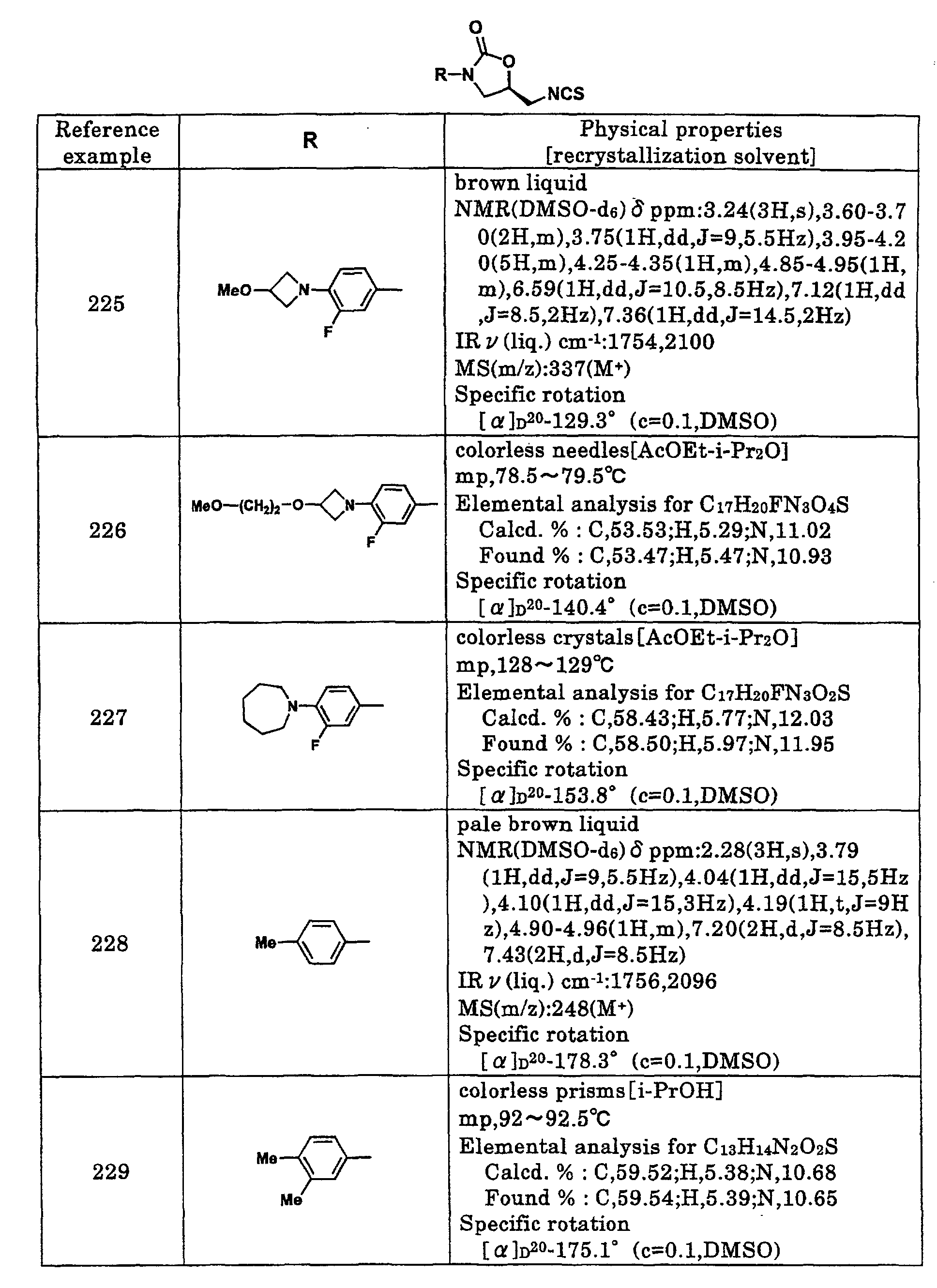

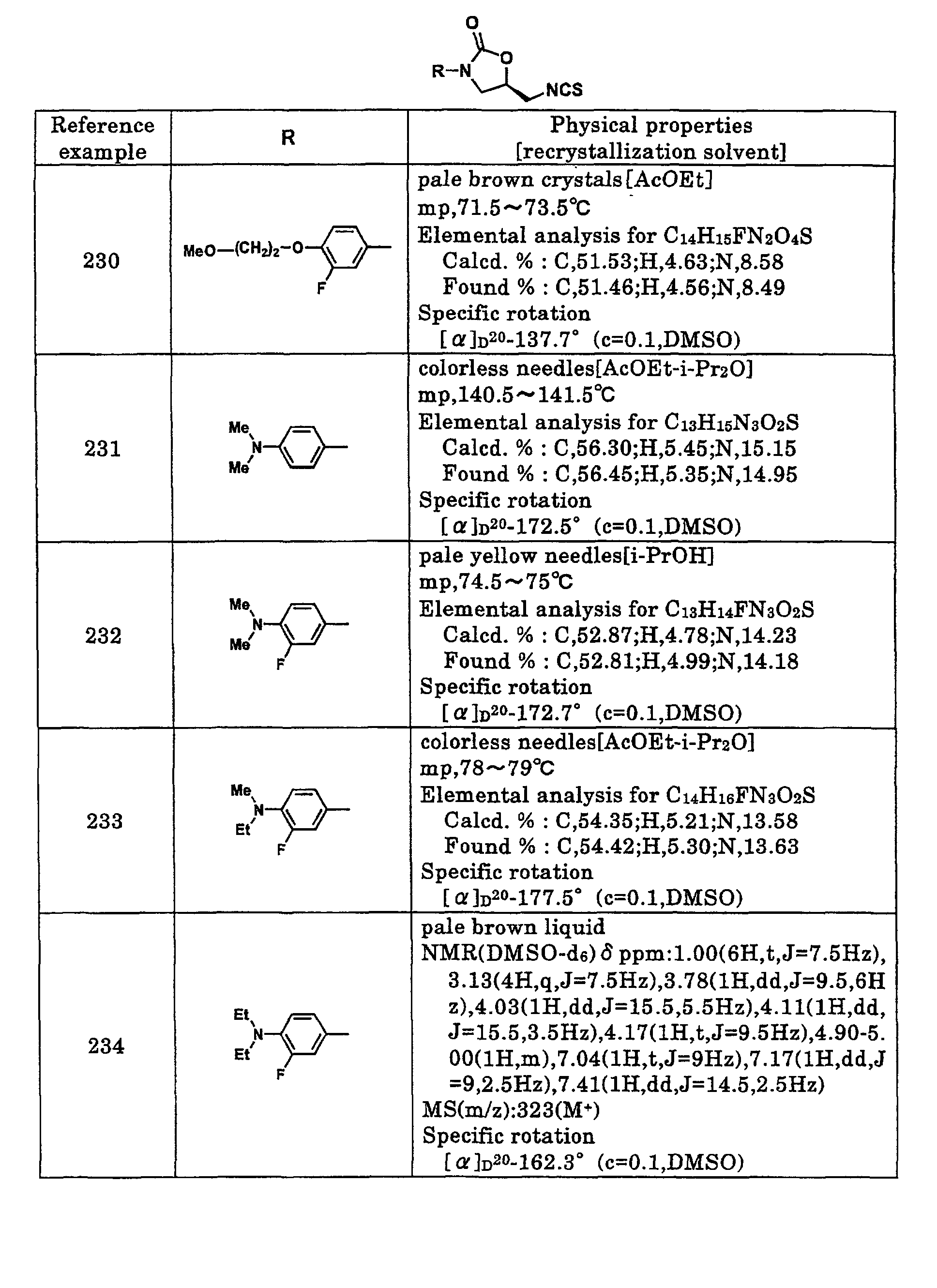

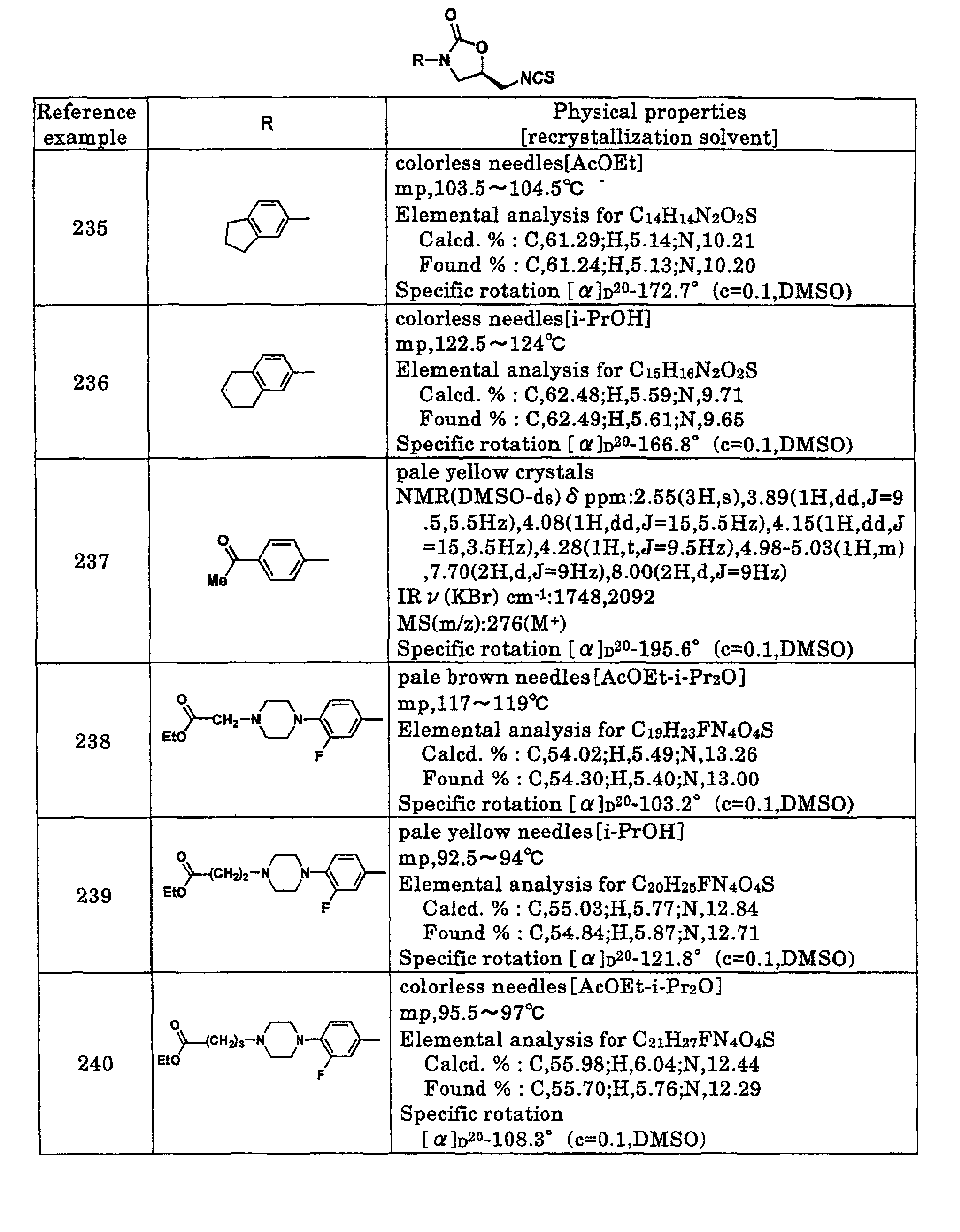

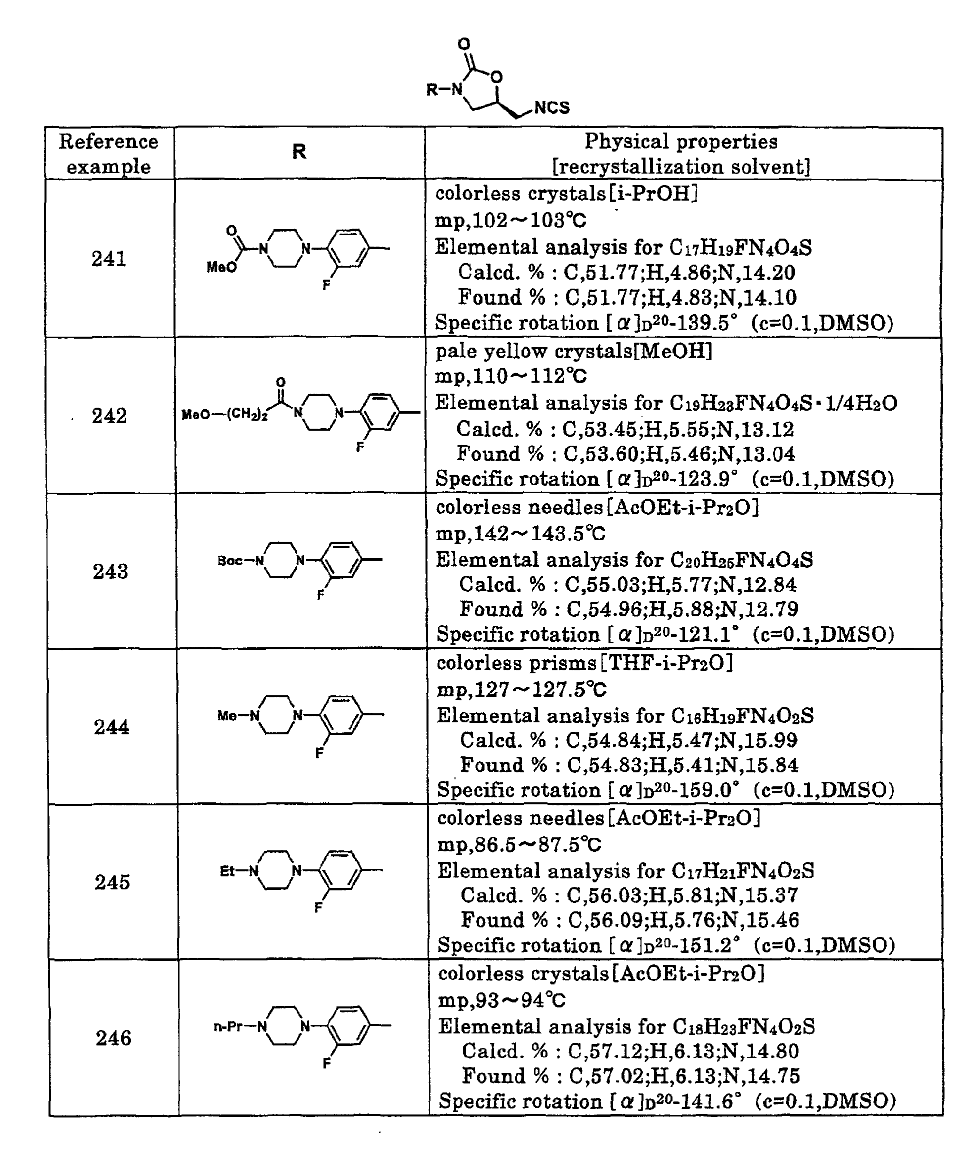

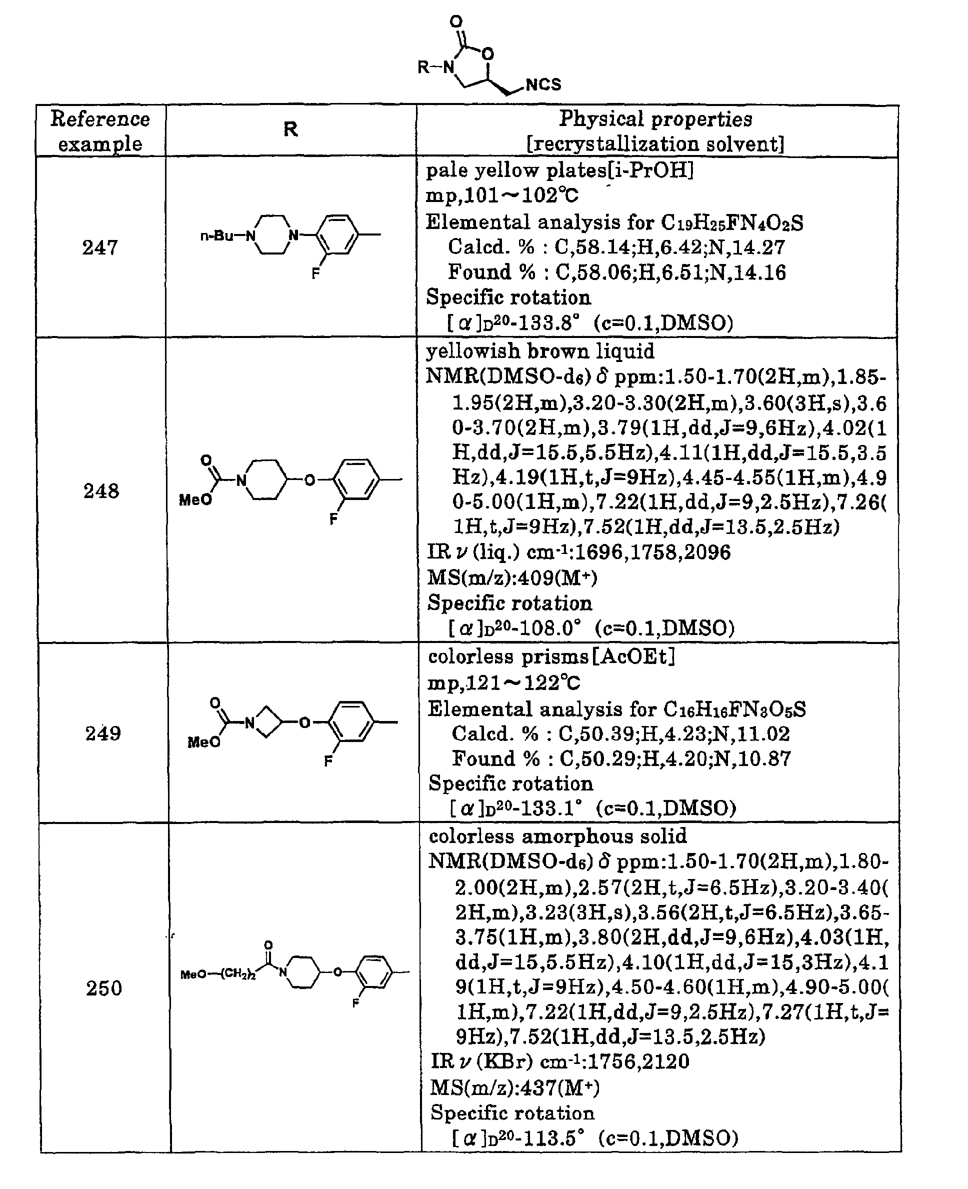

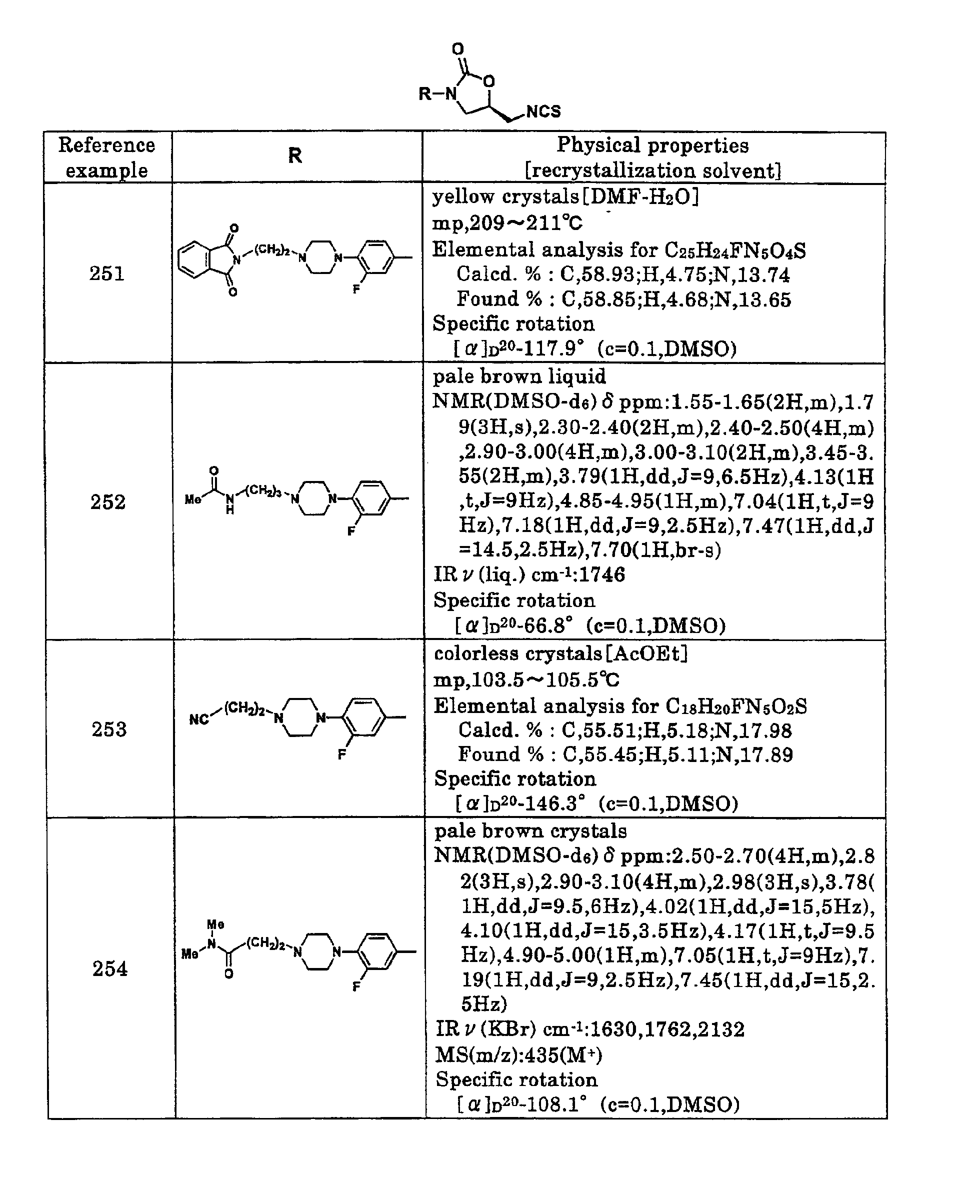

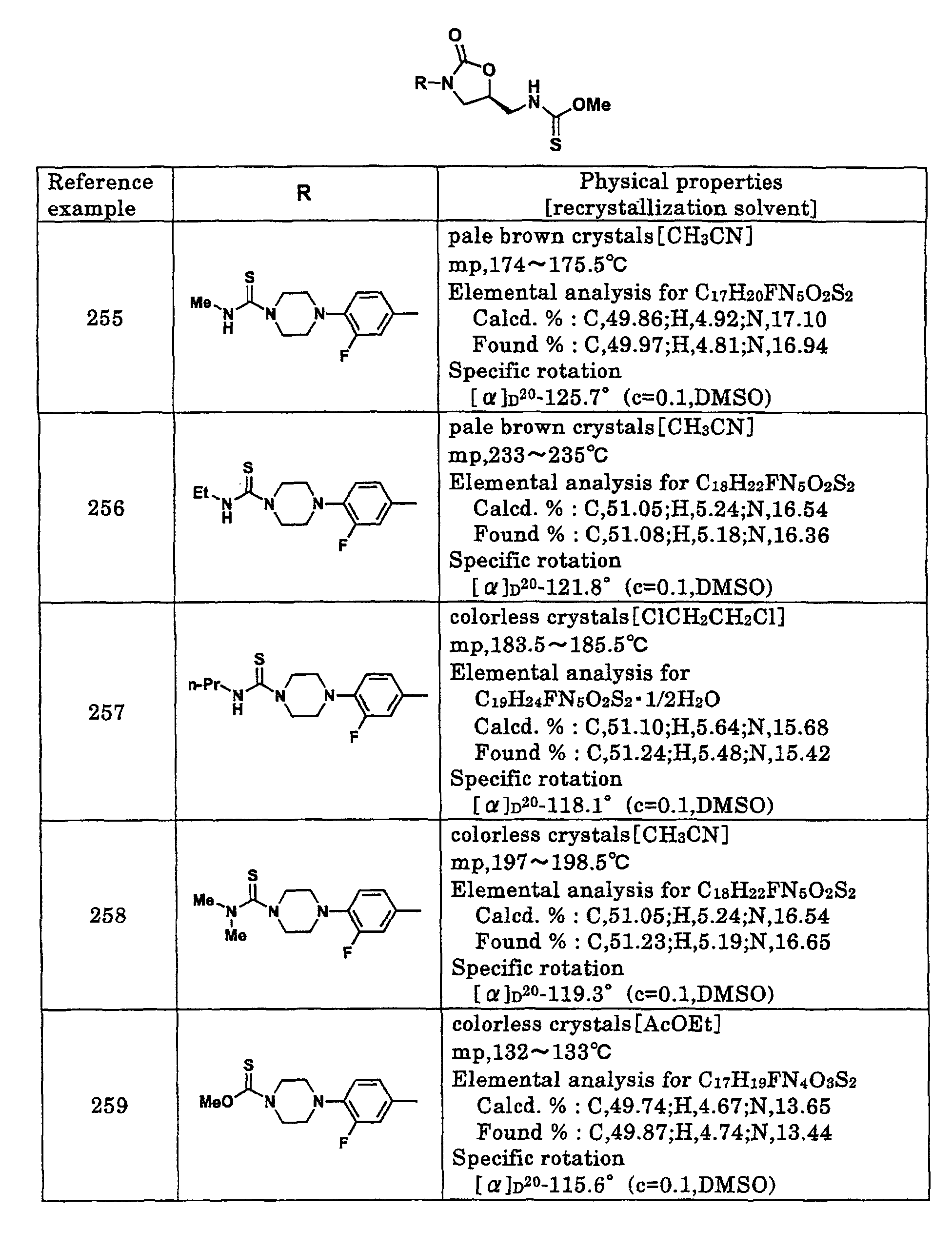

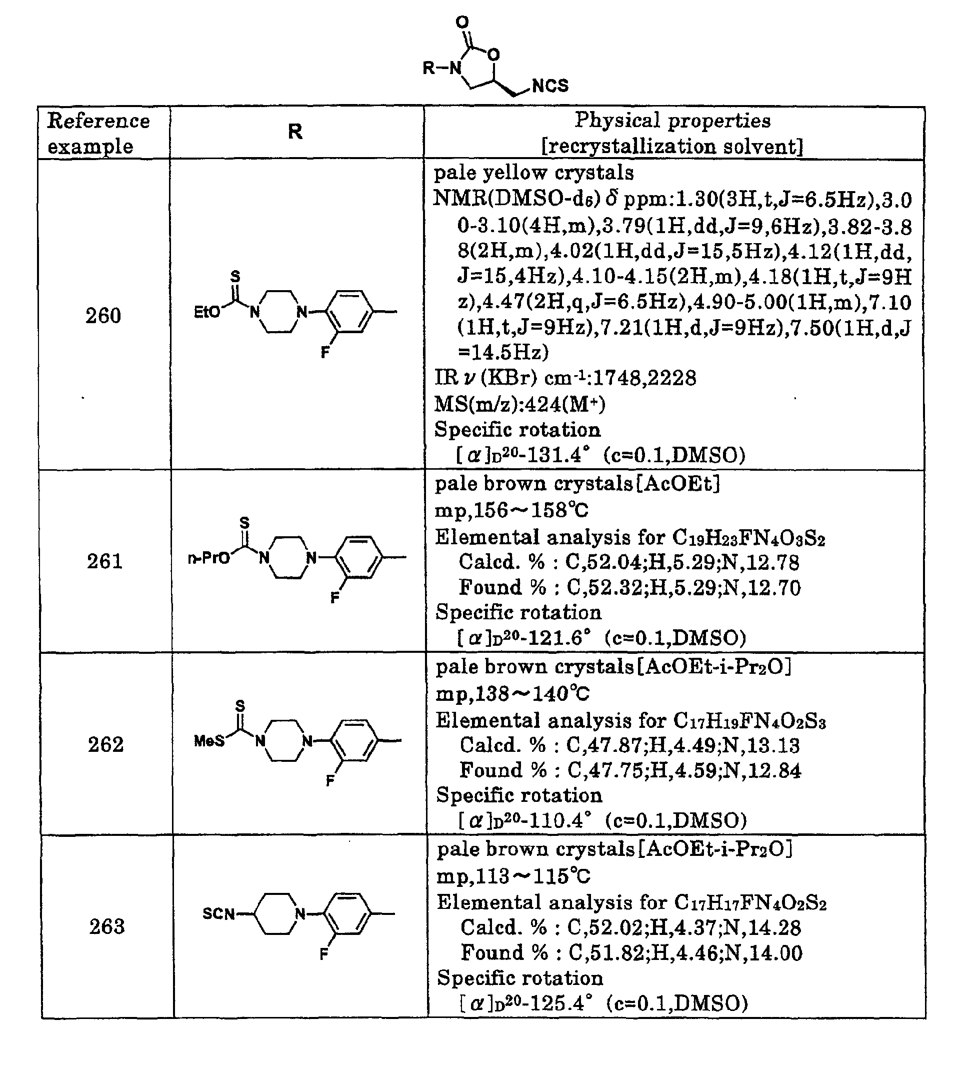

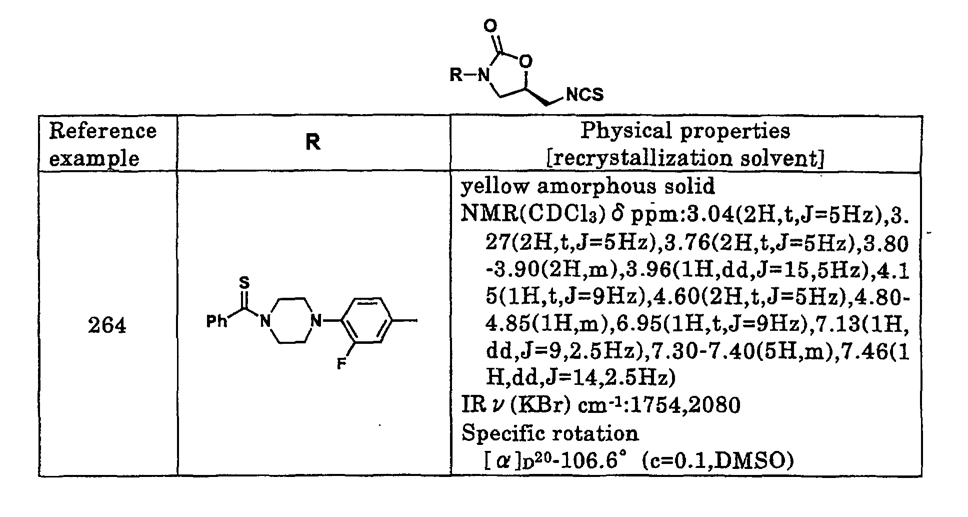

### Reference example 265

(R)-1-[4-[2-Fluoro-4-(5-isothiocyanatomethyl-2-oxooxazolidin-3-yl)phenyl]-piperazine]carbothioamide

To a solution of 0.79 g of 1,1'-thiocarbonyl diimidazole and 0.52 ml of triethylamine in 20 ml of 1,2-dichloroethane, 1.20 g of (R)-1-[4-[4-(5-aminomethyl-2-oxooxazolidin-3-yl)-2-fluorophenyl]piperazine]carbothioamide was added under ice-cooling, and the mixture was stirred at room temperature for 18 hours. The reaction solution was added with water and 1,2-dichloroethane, and the insoluble solid was removed. Then, the 1,2-dichloroethane layer was washed with saturated brine, and dried over sodium sulfate, and then the solvent was evaporated under reduced pressure. The residue was purified by column chromatography (silica gel, 1,2-dichloroethane : methanol = 20 : 1) to obtain 0.25 g of yellow crystals.
NMR spectrum (DMSO-d₆) δ ppm:
2.99(4H,t,J=5Hz),3.79(1H,dd,J=9.5,5.5Hz),3.90(4H,t,J=5Hz),4.02(1H,dd,J=15,5.5Hz), 4.10(1H,dd,J=15,4Hz),4.18(1H,t,J=9Hz),4.90-5.00(1H,m),7.09(1H,t,J=9Hz), 7.20(1H,dd,J=9,2.5Hz),7.39(2H,br-s),7.48(1H,dd,J=14.5,2.5Hz)
IR spectrum ν (KBr) cm⁻¹: 1746,2220
Specific rotation [α]_{D}²⁰ -130.4° (c = 0.1, DMSO)

The compounds of Reference examples 266 through 267 were obtained in the same manner as in Reference example 265.

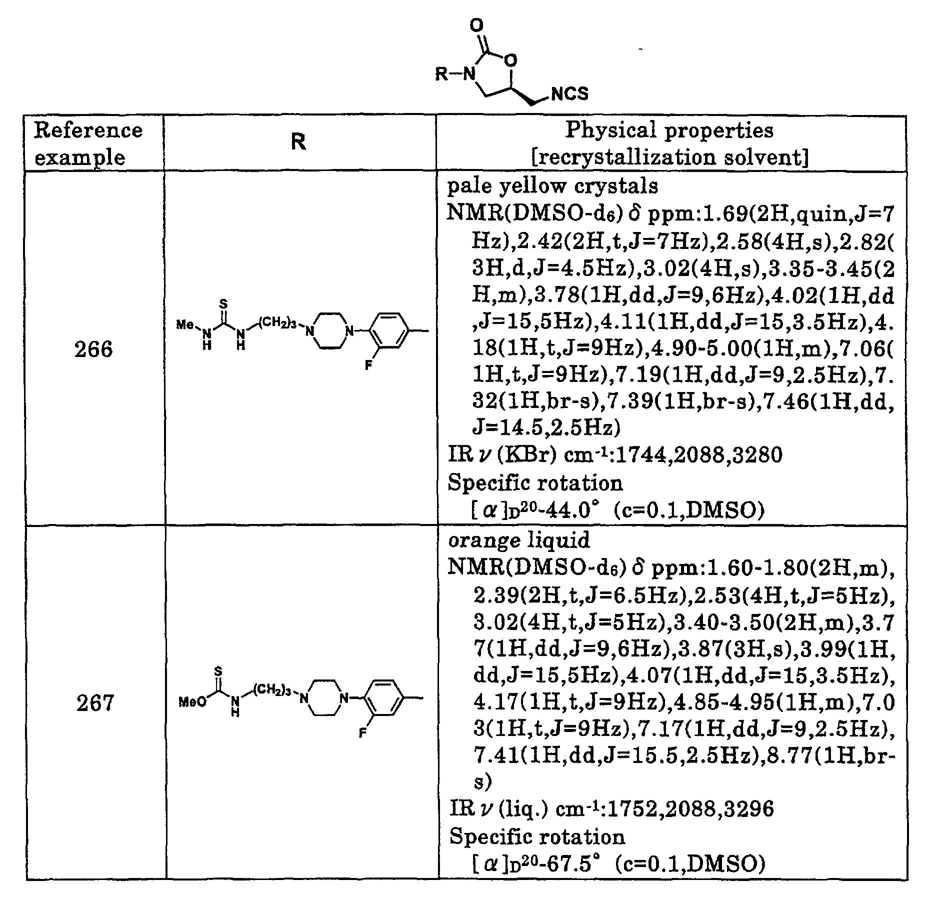

### Example 1

O-Methyl (S)-N-[3-[3-fluoro-4-(thiomorpholin-4-yl)phenyl]-2-oxooxazolidin-5-yl]methylthiocarbamate

To 44 ml of dried methanol, 0.53 g of 60 % sodium hydride was added under ice-cooling and with stirring, and the mixture was stirred at room temperature for 30 minutes. Then, the mixture was added with 4.41 g of (R)-N-[3-[3-fluoro-4-(thiomorpholin-4-yl)phenyl]-2-oxooxazolidin-5-yl]methyl isothiocyanate, and stirred at room temperature for 3 hours. The reaction mixture was neutralized with ice water and 10% hydrochloric acid, and the precipitated crystals were collected by filtration to obtain 4.68 g of pale brown crystals. Recrystallization from ethanol gave pale brown crystals having the melting point of from 141.5 to 143°C.

| Elemental analysis for C₁₆H₂₀FN₃O₃S₂ | | | | | | |
|---|---|---|---|---|---|---|
| Calculated % | C, | 49.85; | H, | 5.23; | N, | 10.90 |
| Found % | C, | 49.58; | H, | 5.05; | N, | 10.82 |

Speck rotation [α]_{D}²⁰ -25.9° (c =0.1, DMSO)

The compounds of Examples 2 through 60 were obtained in the same manner as in Example 1.

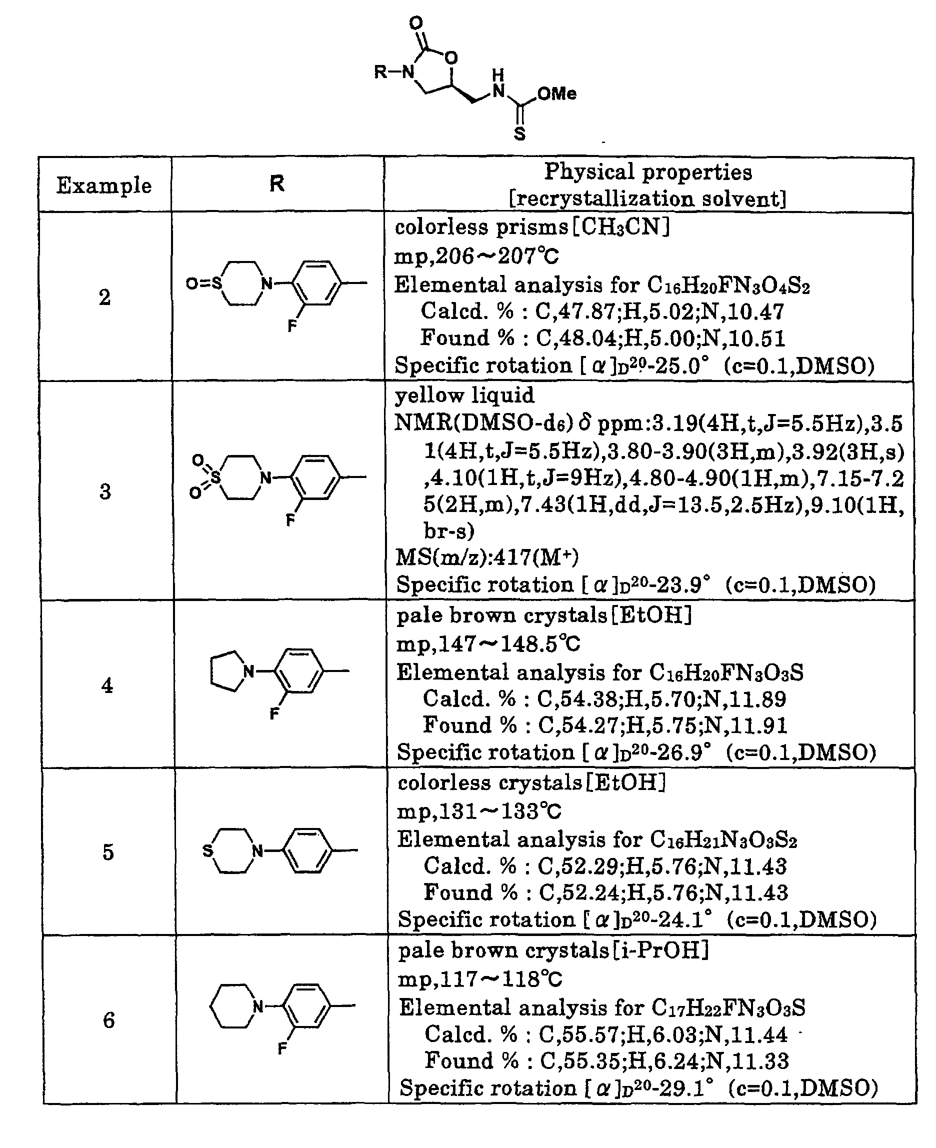

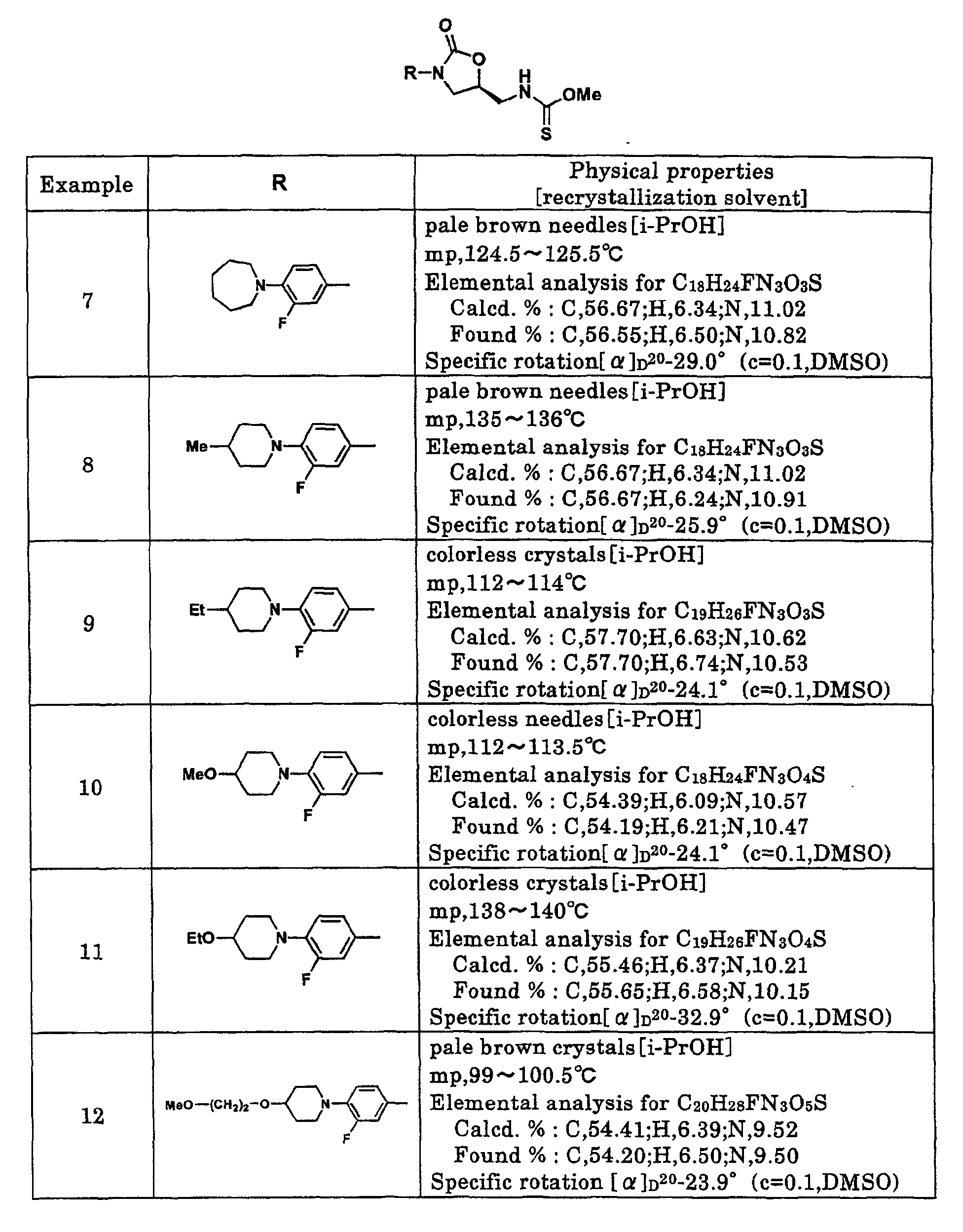

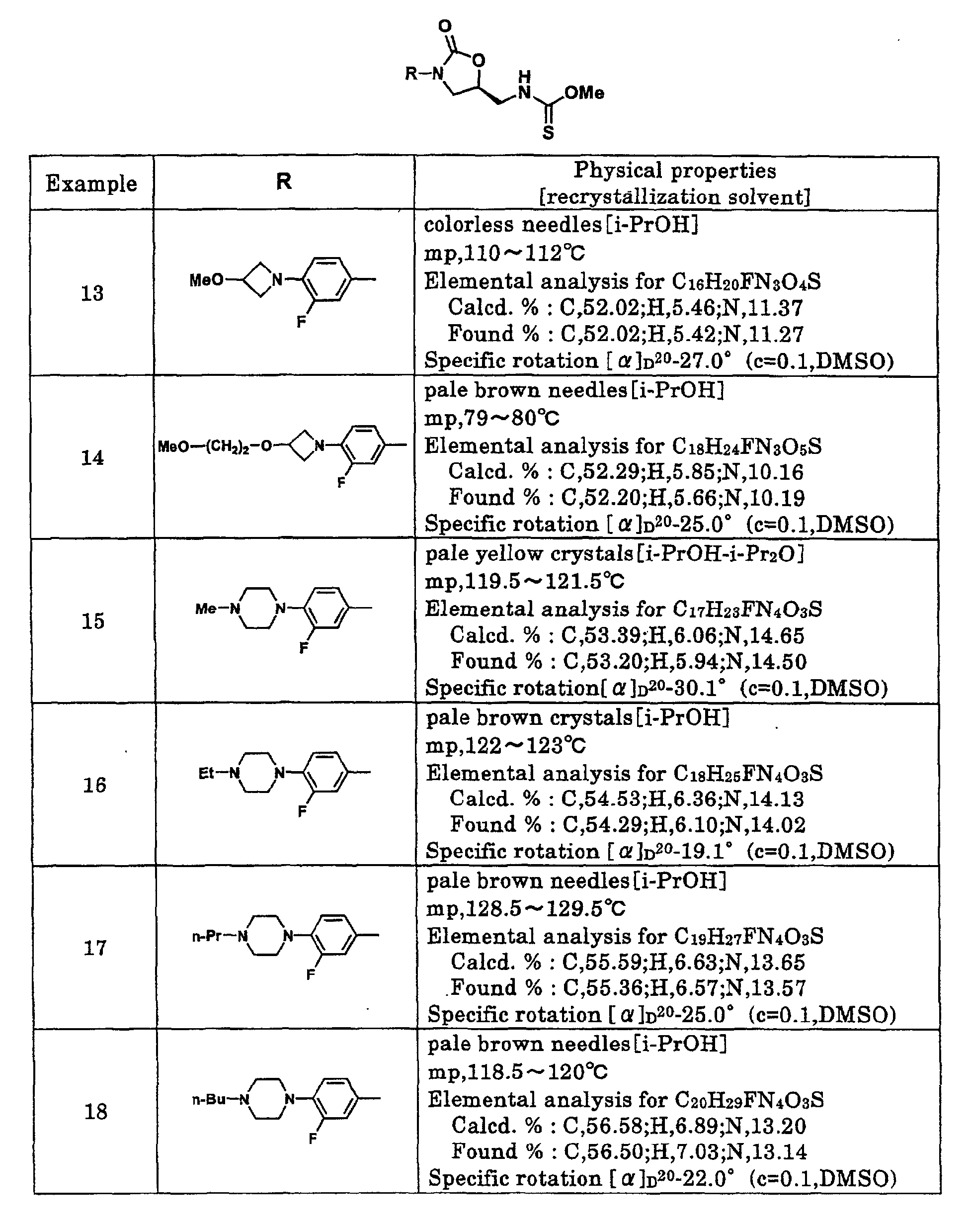

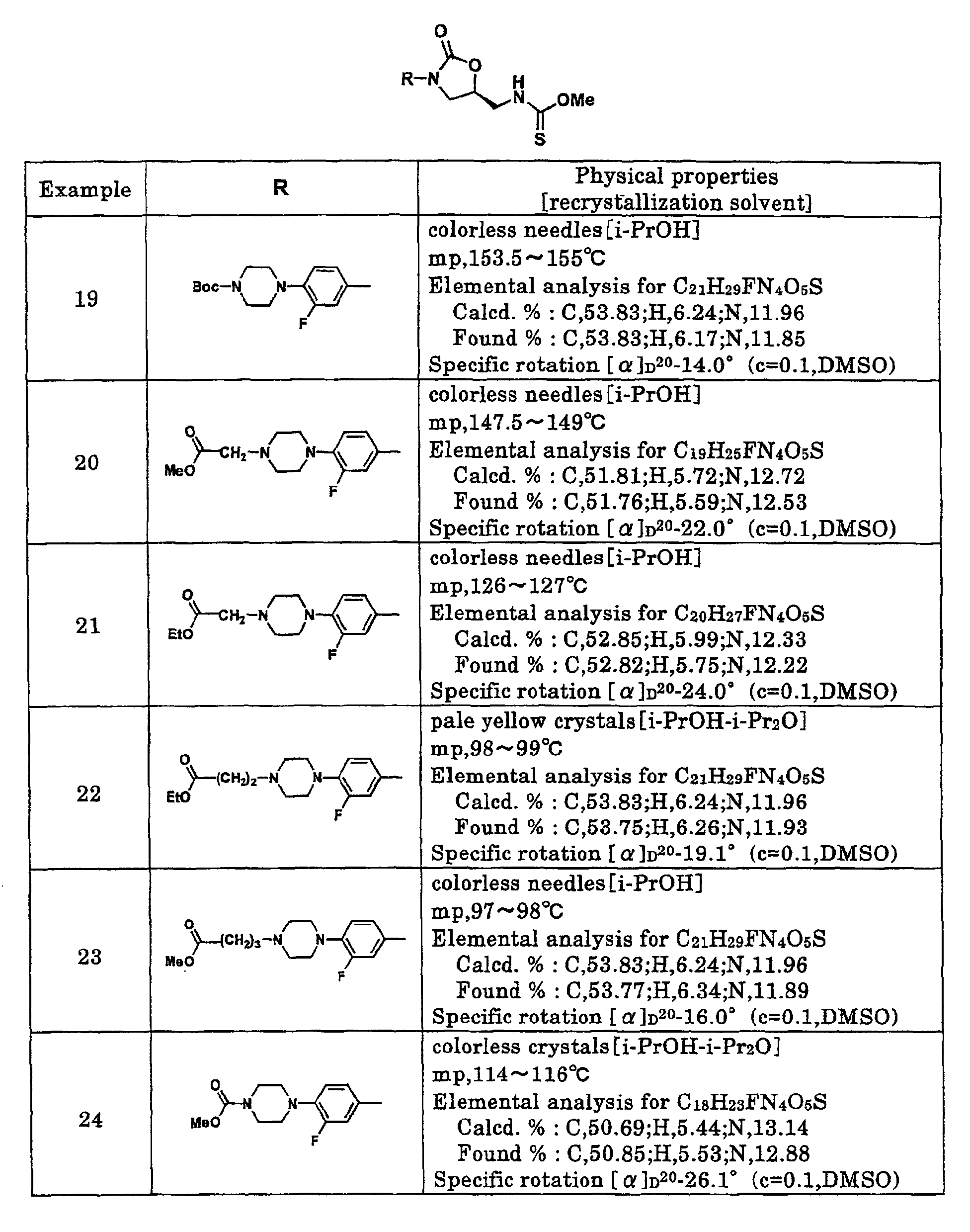

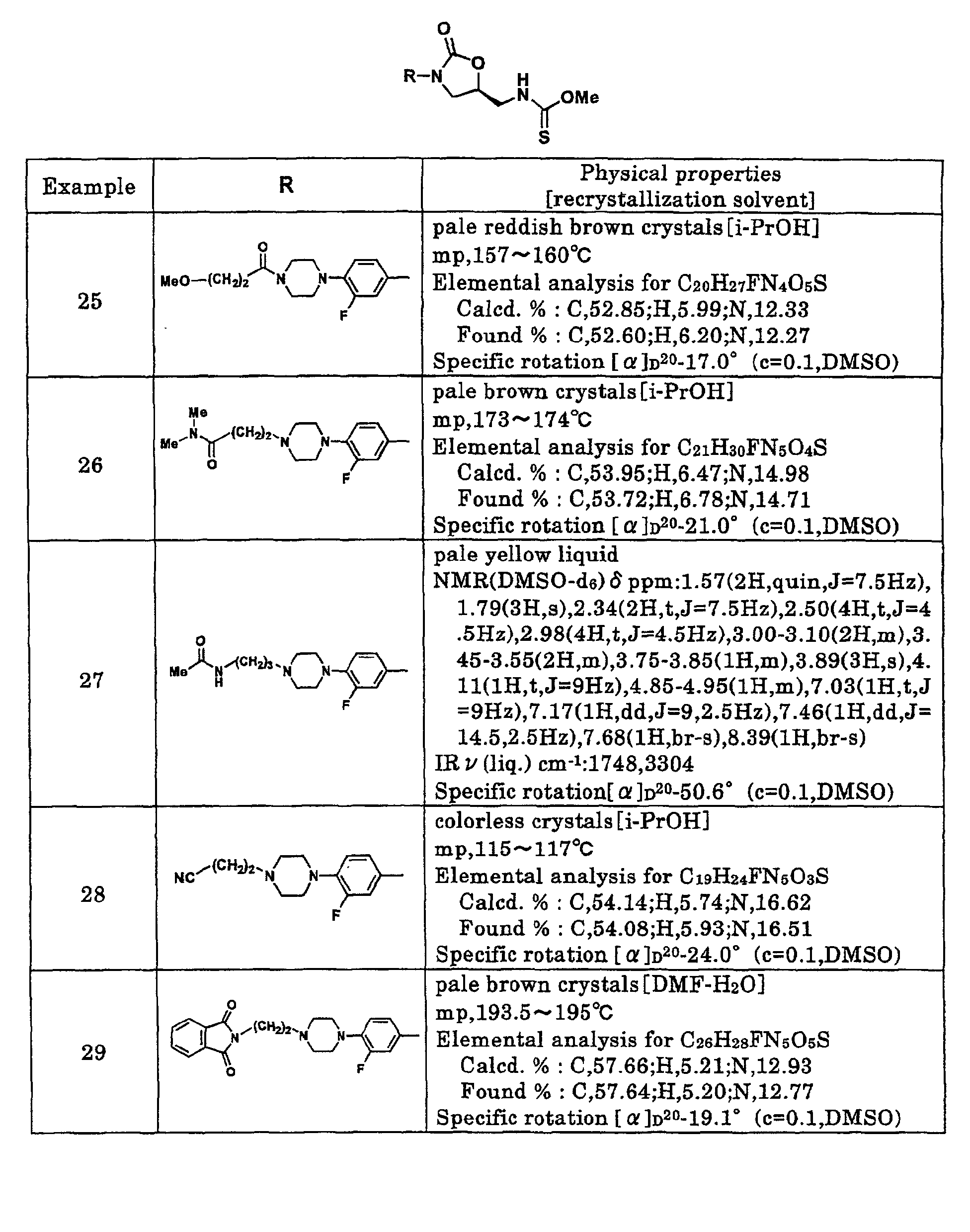

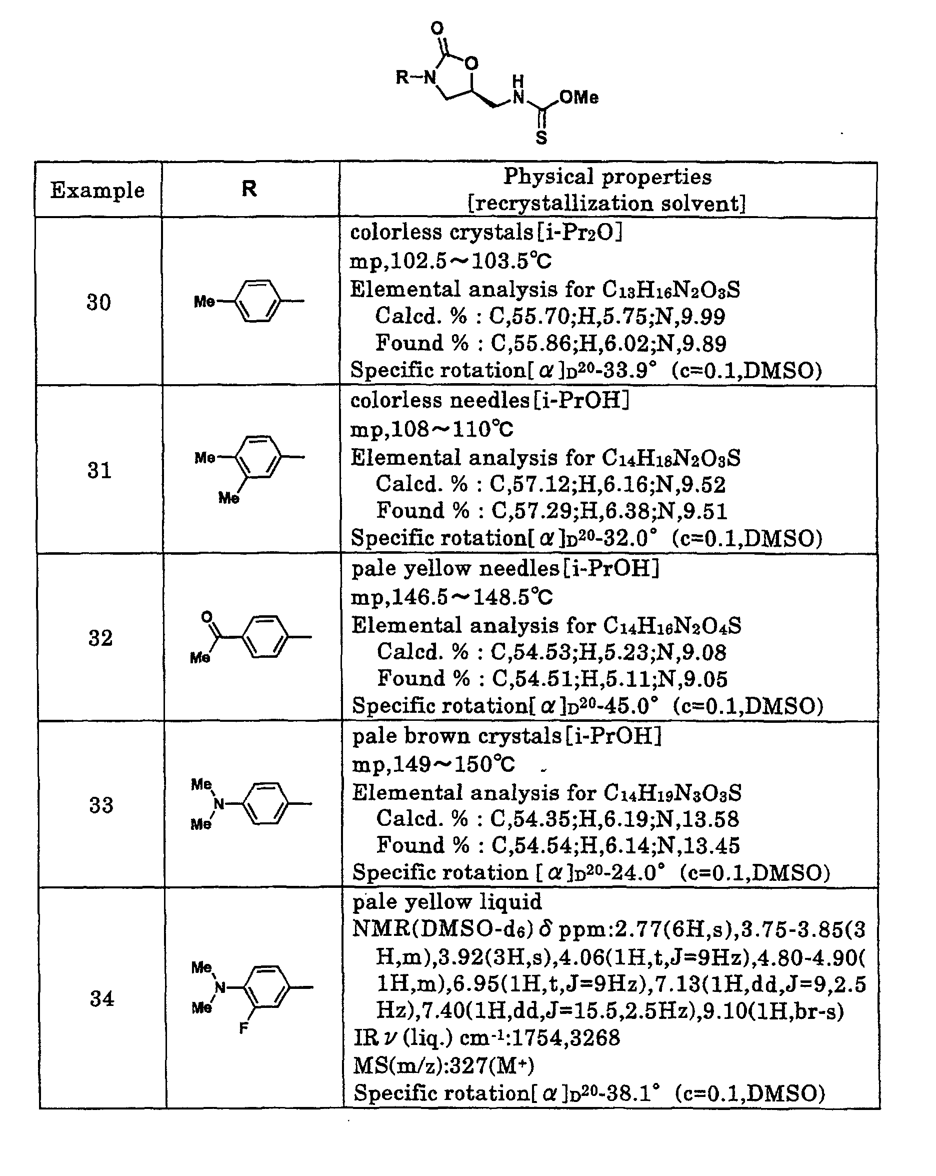

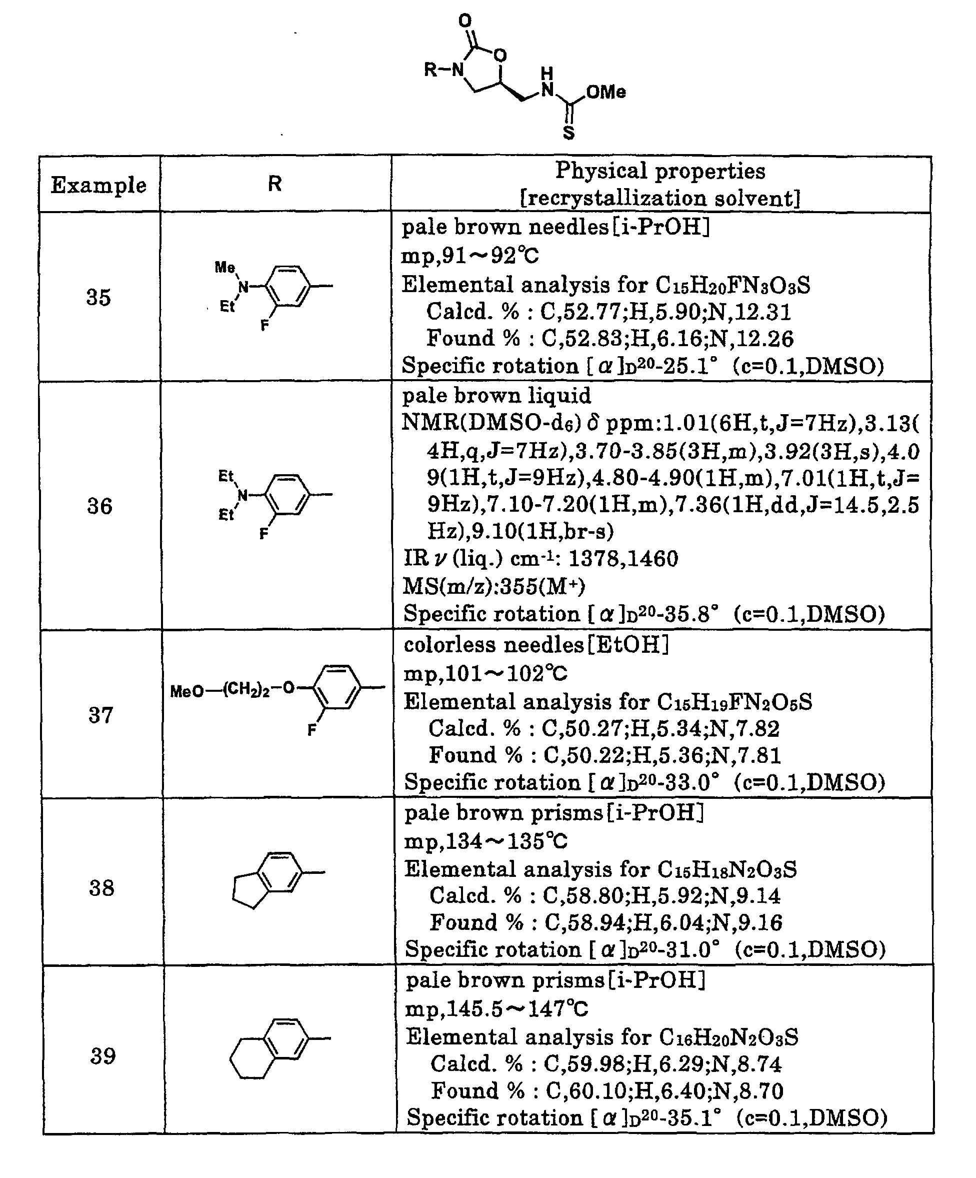

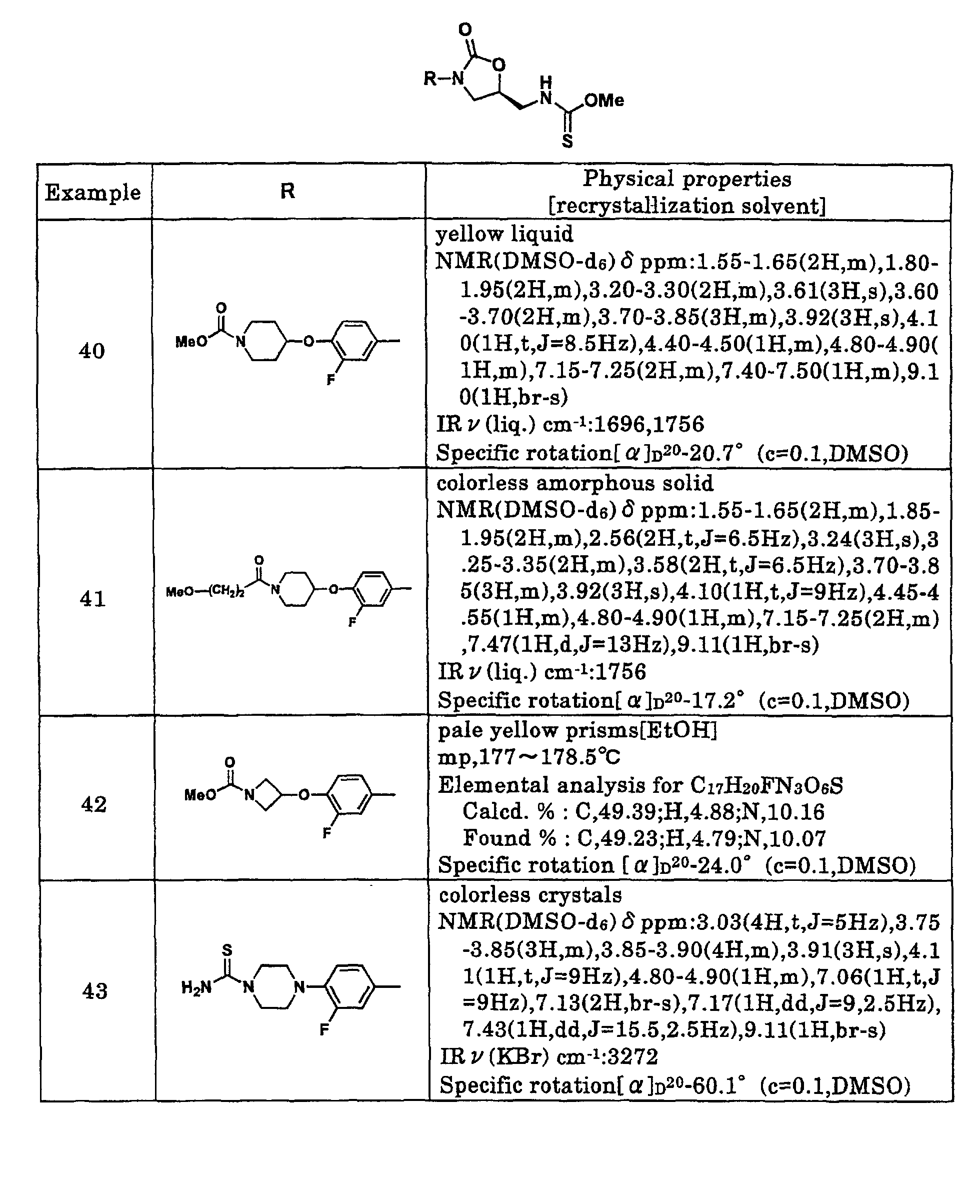

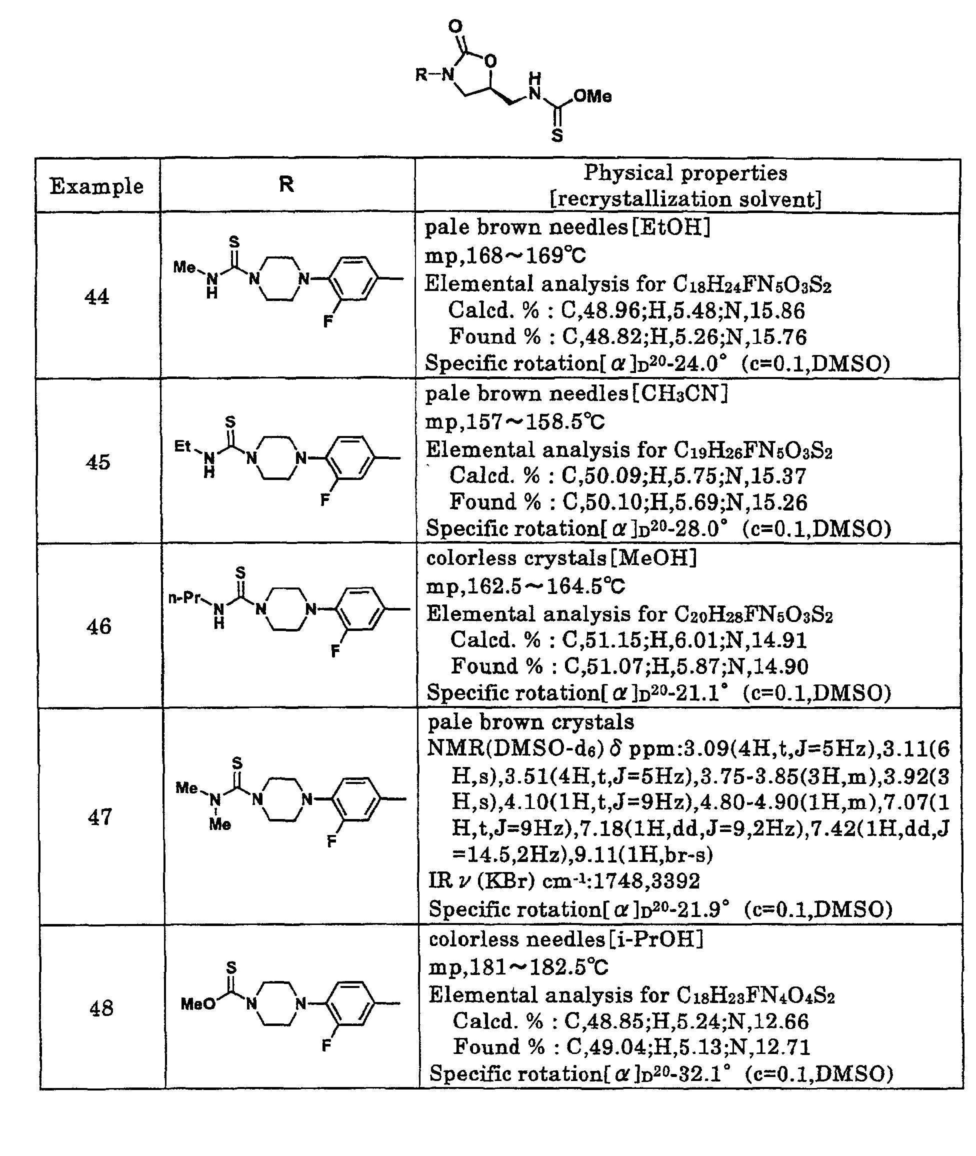

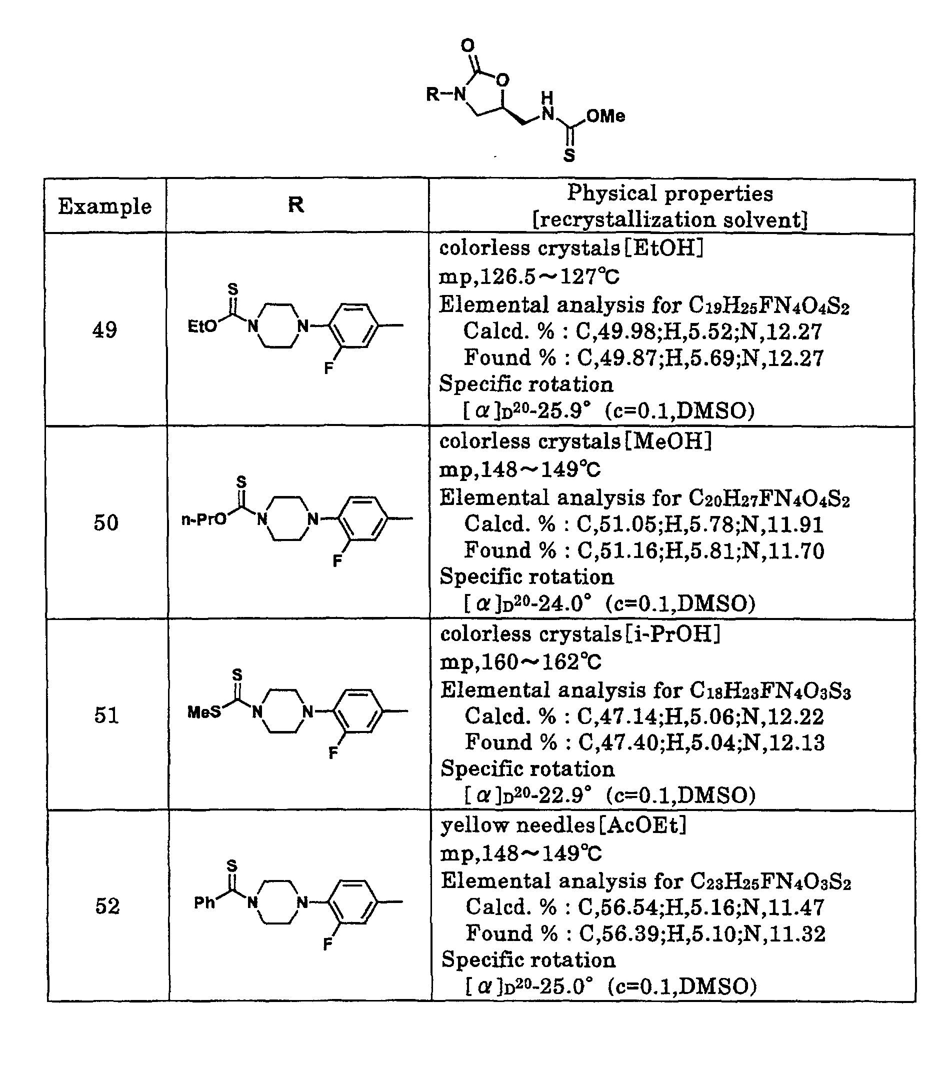

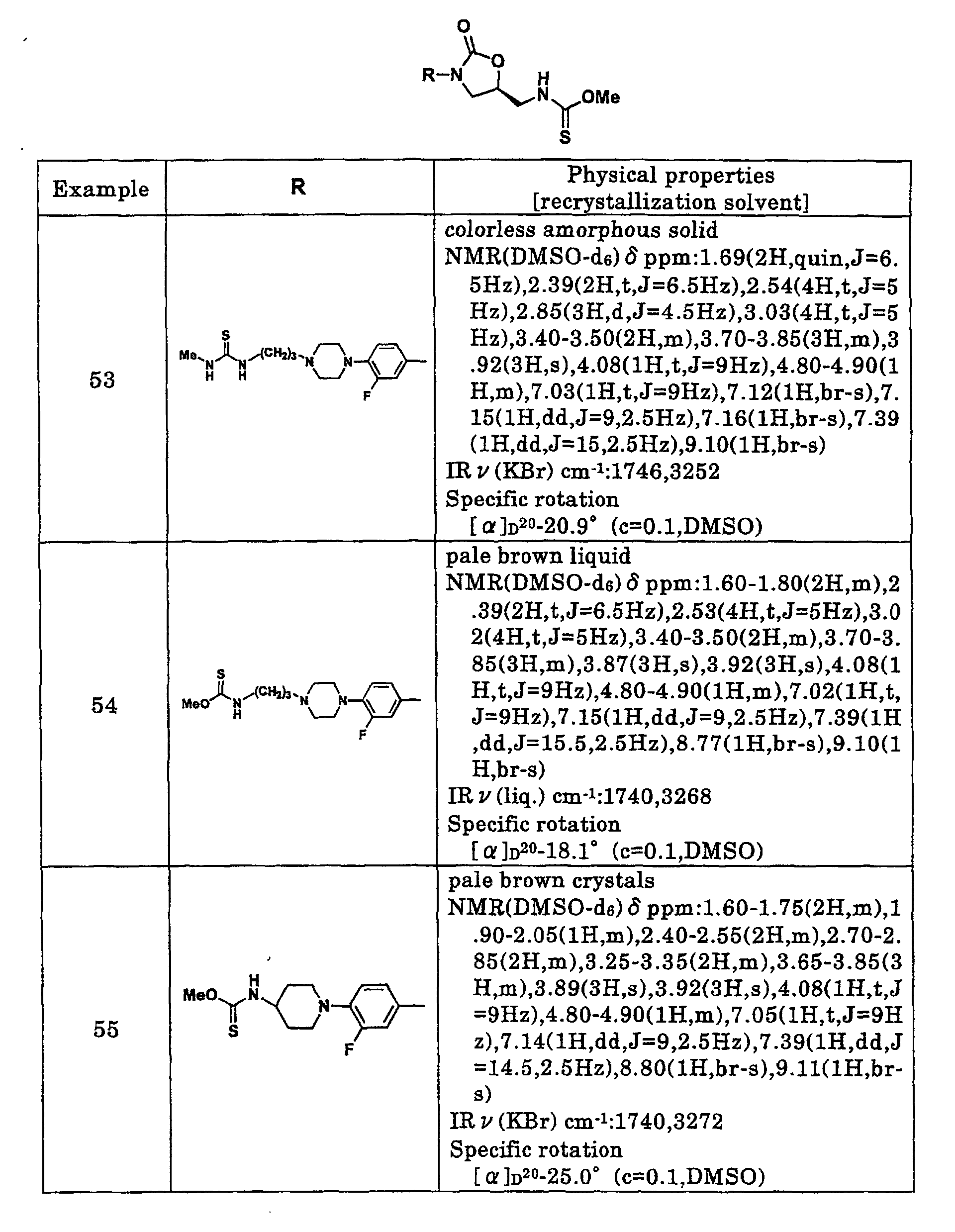

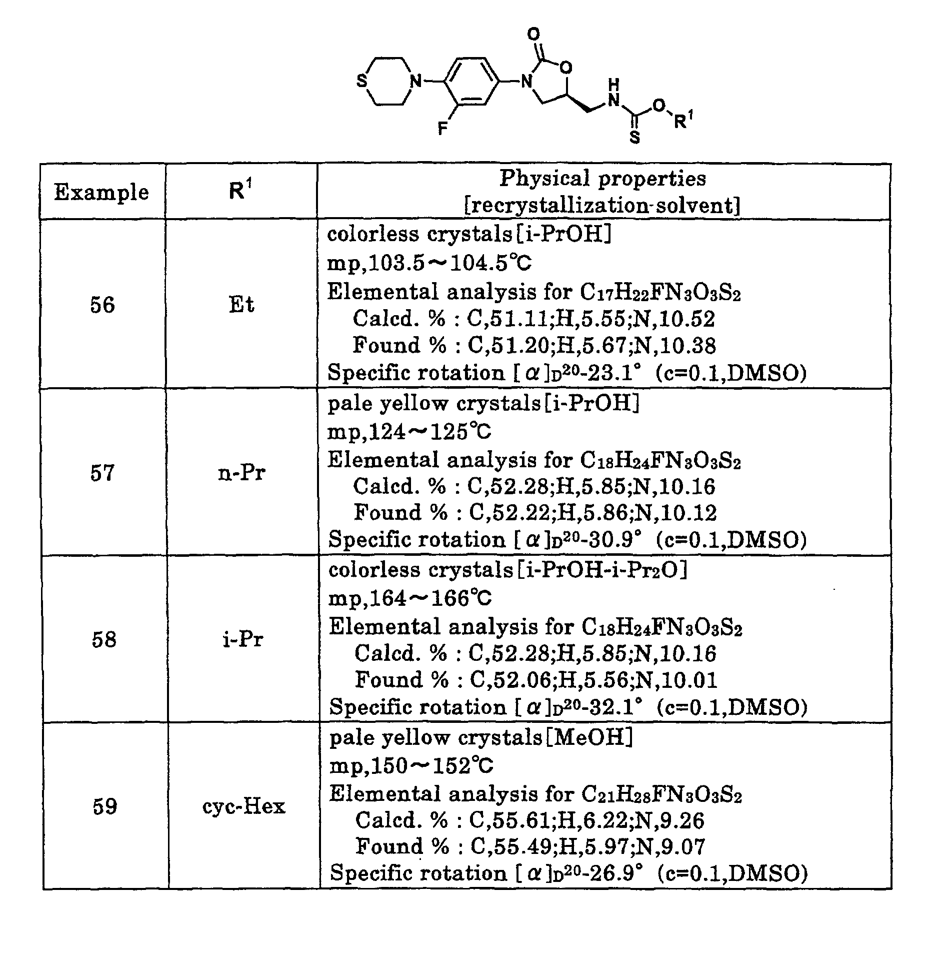

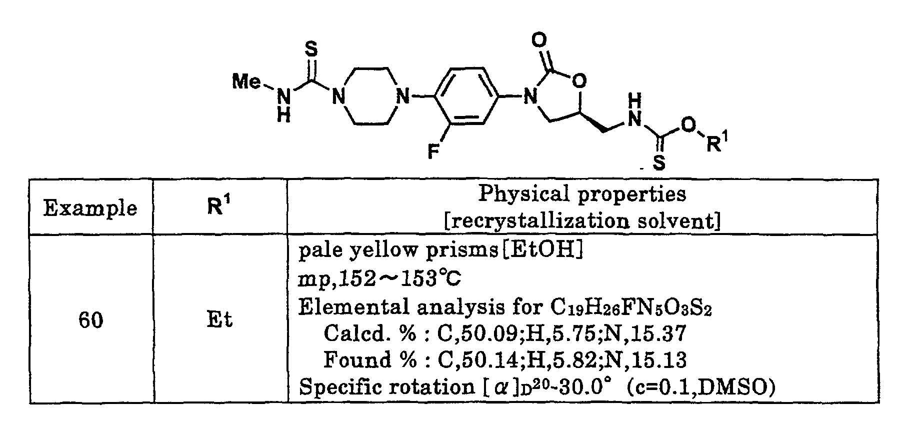

### Example 61

O-Methyl (S)-N-[3-[3-fluoro-4-[4-(3-mesylaminopropyl)piperazin-1-yl]-phenyl]-2-oxooxazolidin-5-yl]methylthiocarbamate
1) To a solution of 0.40 g of (S)-5-aminomethyl-3-[3-fluoro-4-[4-(3-mesylaminopropyl)piperazin-1-yl]phenyl]-2-oxooxazolidine and 0.13 ml of triethylamine in 4 ml of dried tetrahydrofuran, 0.12 ml of carbon disulfide was added under ice-cooling and with stirring, and the mixture was stirred at the same temperature for 5 hours. The mixture was added with 0.09 ml of ethyl chlorocarbonate under ice-cooling and with stirring, and further stirred at the same temperature for 2 hours. The reaction solution was added with water, and extracted with ethyl acetate. The extract was washed with saturated brine, and dried over sodium sulfate, and then the solvent was evaporated under reduced pressure to obtain 0.39 g of a pale brown amorphous solid.
2) To 3.5 ml of dried methanol, 0.06 g of 60 % sodium hydride was added under stirring at room temperature, and the mixture was stirred at room temperature for 30 minutes. Then, the mixture was added with 0.35 g of the amorphous solid obtained in 1), and stirred at room temperature for 1 hour. The reaction mixture was neutralized with ice water and 10 % hydrochloric acid, and extracted with ethyl acetate. The extract was washed with saturated brine, and dried over sodium sulfate, and then the solvent was evaporated under reduced pressure. The residue was purified by column chromatography (alumina, ethyl acetate) to obtain 0.14 g of pale brown crystals. Recrystallization from isopropanol gave colorless needles having the melting point of from 120 to 121.5°C.

| Elemental analysis for C₂₀H₃₀FN₅O₅S₂ | | | | | | |
|---|---|---|---|---|---|---|
| Calculated % | C, | 47.70; | H, | 6.00; | N, | 13.91 |
| Found % | C, | 47.76; | H, | 5.88; | N, | 13.70 |

Specific rotation [α]_{D}²⁰ -19.0° (c = 0.1, DMSO)

### Example 62

O-Methyl (S)-N-[3-[3-fluoro-4-(piperazin-1-yl)phenyl]-2-oxooxazolidin-5-yl]-methylthiocarbamate

To 4 ml of 10 % hydrogen chloride ethyl acetate solution, 0.20 g of O-methyl (S)-N-[3-[4-(4-tert-butoxycarbonylpiperazin-1-yl)-3-fluorophenyl]-2-oxooxazolidin-5-yl]methylthiocarbamate was added under ice-cooling and with stirring, and the mixture was stirred at room temperature for 6 hours. After the reaction, the solvent was evaporated under reduced pressure, and the residue was added with aqueous sodium hydrogencarbonate, and extracted with 1,2-dichloroethane. The extract was washed successively with water and saturated brine, and dried over sodium sulfate, and then the solvent was evaporated under reduced pressure to obtain 0.10 g of a colorless amorphous solid.
NMR spectrum (DMSO-d₆) δ ppm:
3.00(8H,s),3.21(1H,br-s),3.75-3.85(3H,m),3.89(3H,s),4.11(1H,t,J=9Hz), 4.85-4.90(1H,m),7.07(1H,t,J=9.5Hz),7.15-7.19(1H,m),7.45(1H,dd,J=15,3Hz), 9.39(1H,br-s)
IR spectrum ν (KBr) cm⁻¹: 1750,3228
Specific rotation [α]_{D}²⁰ -30.1° (c = 0.1, DMSO)

### Example 63

O-Methyl (S)-N-[3-[3-fluoro-4-[4-(3-oxobutyl)piperazin-1-yl]phenyl]-2-oxo-oxazolidin-5-yl]methylthiocarbamate

To a solution of O-methyl (S)-N-[3-[3-fluoro-4-(piperazin-1-yl)phenyl]-2-oxo-oxazolidin-5-yl]methylthiocarbamate in 1 ml of methanol, 0.01 ml of methyl vinyl ketone was added under stirring at room temperature, and the mixture was stirred at the same temperature for 30 minutes. After the reaction, the solvent was evaporated under reduced pressure, and the residue was purified by column chromatography (alumina, ethyl acetate) to obtain 0.06 g of colorless crystals.
NMR spectrum (CDCl₃) δ ppm:
2.19(3H,s),2.63(4H,t,J=5Hz),2.66(2H,t,J=6.5Hz),2.74(2H,t,J=6.5Hz),3.07(4H,t,J=5Hz), 3.82(1H,dd,J=9,6.5Hz),4.00(3H,s),3.90-4.15(3H,m),4.85-4.95(1H,m),6.69(1H,t,J=6Hz), 6.92(1H,t,J=9Hz),7.07(1H,dd,J=9,2.5Hz)17.41(1H,dd,J=14,2.5Hz)
IR spectrum ν (KBr) cm⁻¹: 1714,1746,3284
Specific rotation [α]_{D}²⁰ -20.8° (c = 0.1, DMSO)

### Example 64

O-Methyl (S)-N-[3-[4-(4-carbamoylpiperazin-1-yl)-3-fluorophenyl]-2-oxo-oxazolidin-5-yl]methylthiocarbamate

To a solution of 0.50 g of O-methyl (S)-N-[3-[3-fluoro-4-(piperazin-1-yl)-phenyl]-2-oxooxazolidin-5-yl]methylthiocarbamate in 5.0 ml of acetic acid, a solution of 0.21 g of sodium cyanate in 5.0 ml of water was added under stirring at room temperature, and the mixture was stirred at the same temperature for 2 hours. The reaction solution was neutralized with 40% aqueous sodium hydroxide, and then extracted with ethyl acetate. The extract was washed successively with water and saturated brine, and dried over sodium sulfate, and then the solvent was evaporated under reduced pressure. The residue was washed with ethyl acetate to obtain 0.15 g of colorless crystals.
NMR spectrum (DMSO-d₆) δ ppm:
2.90-3.00(4H,m),3.40-3.50(5H,m),3.70-3.85(2H,m),3.92(3H,s),4.09(1H,t,J=9Hz), 4.70-4.90(1H,m),5.64(2H,br-s),7.05(1H,t,J=9Hz),7.10-7.20(1H,m), 7.41(1H,dd,J=14.5,2.5Hz),9.10(1H,br-s)
IR spectrum ν (KBr) cm⁻¹: 1740
Specific rotation [α]_{D}²⁰ -32.0° (c = 0.1, DMSO)

### Example 65

O-Methyl (S)-N-[3-[3-fluoro-4-(4-thioacetylpiperazin-1-yl)phenyl]-2-oxooxazolidin-5-yl]methylthiocarbamate

To a solution of 0.50 g of O-methyl (S)-N-[3-[3-fluoro-4-(piperazin-1-yl)-phenyl]-2-oxooxazolidin-5-yl]methylthiocarbamate in 5.0 ml of tetrahydrofuran, 0.17 ml of ethyl dithioacetate was added under stirring at room temperature, and the mixture was stirred at the same temperature for 18 hours. After the reaction, the solvent was evaporated under reduced pressure, and the residue was washed with ethyl acetate to obtain 0.27 g of colorless crystals. Recrystallization from acetonitrile gave colorless crystals having the melting point of from 176 to 178°C.

| Elemental analysis for C₁₈H₂₃FN₄O₃S₂ | | | | | | |
|---|---|---|---|---|---|---|
| Calculated % | C, | 50.69; | H, | 5.44; | N, | 13.14 |
| Found % | C, | 50.71; | H, | 5.34; | N, | 13.26 |

Specific rotation [α]_{D}²⁰ -33.0° (c = 0.1, DMSO)

To evaluate excellent effectiveness of the thiocarbamic acid derivatives of the present invention, antibacterial tests against type strains, clinical isolates, and atypical acid-fast bacteria were performed.

### [Antibacterial spectra against type strains, clinical isolates, and atypical acid-fast bacteria]

Antibacterial activities (minimum inhibitory concentration: MIC) were determined according to the standard method of the Japan Society of Chemotherapy [Chemotherapy, Vol. 29, p.76 (1981)] by using type strains and strains isolated from patients with infectious disease (including clinical isolates and atypical acid-fast bacteria), and applying 10⁶/ml of colony forming bacteria. Linezolid was used as the reference compound [a compound described in Journal of Medicinal Chemistry, Vol. 39, p.673 (1996), hereinafter referred to as "reference compound"].

The compounds of the present invention had excellent antibacterial activities compared to the reference compound against type strains, clinical isolates, and atypical acid-fast bacteria.

Names of the bacteria in the table are as follows:

| | |
|---|---|
| Type strain | *Staphylococcus aureus (S*. *aureus) Bacillus subtilis (B*. *subtilis)* |
| Clinical isolates | Methicillin-resistant *Staphylococcus aureus* (MRSA) *Staphylococcus epidermidis (S*. *epidermidis) Enterococcus faecalis (E*. *faecalis) Enterococcus faecium (E*. *faecium)* |
| Atypical acid-fast | bacteria *Mycobacterium avium (M*. *avium) Mycobacterium intracellulare (M*. *intracellulare)* |

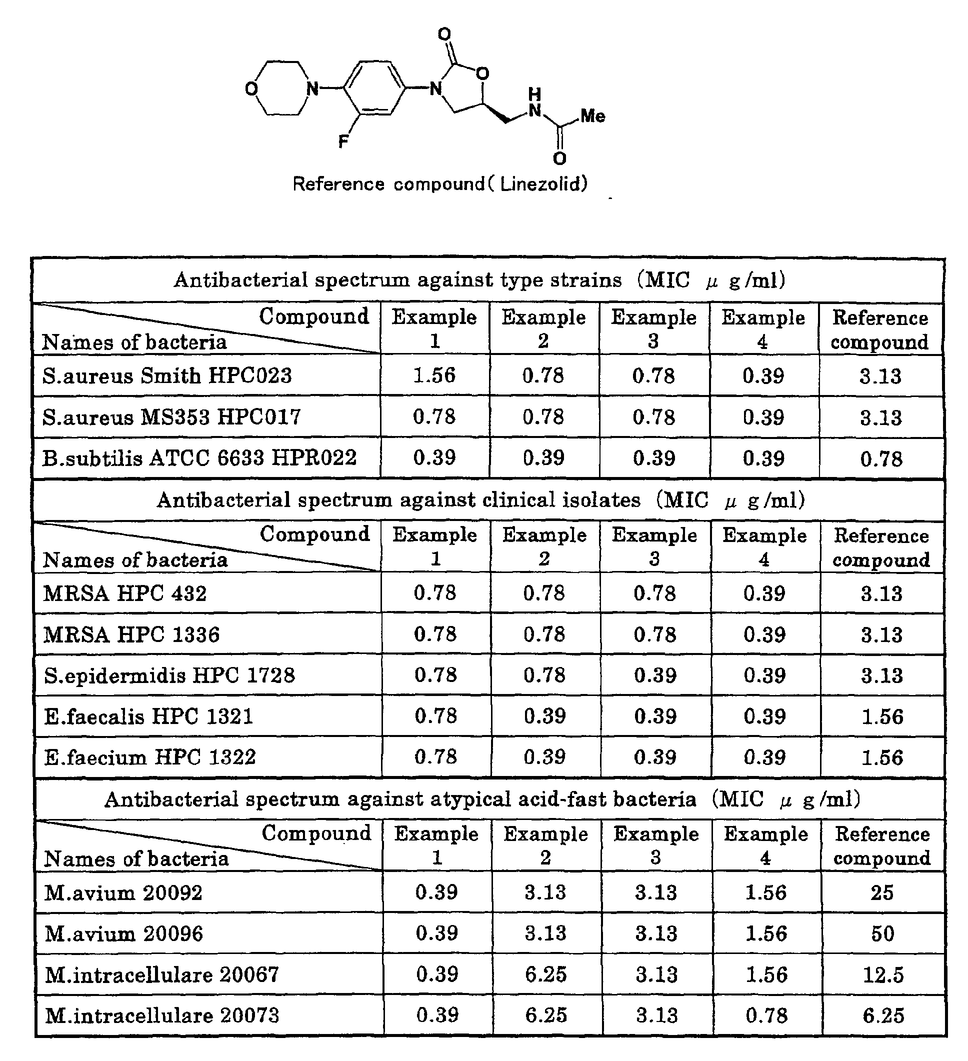

### Industrial Applicability

The novel thiocarbamic acid derivatives or salts thereof according to the present invention have excellent antibacterial activity against clinical isolates and atypical acid-fast bacteria including multiple drug-resistant bacteria as well as type strains, and are extremely useful as antibacterial agents.

## Claims

1. A thiocarbamic acid derivative represented by the following general formula or a salt thereof: 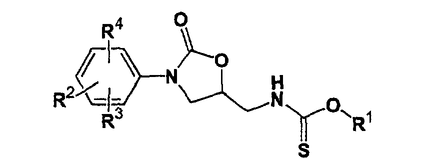 wherein R¹ represents an alkyl group which may be substituted, or a cycloalkyl group which may be substituted; and R², R³, and R⁴ independently represent hydrogen atom, a halogen atom, an alkyl group which may be substituted, an alkoxyl group which may be substituted, an amino group which may be substituted, an alkanoyl group which may be substituted, a cycloalkyloxy group which has a heteroatom as a ring constituting atom and which may be substituted, or a saturated heterocyclic group which may be substituted; or any two of R², R³, and R⁴ may bind to each other to form, together with the benzene ring, a condensed hydrocarbon ring which may be substituted.

2. A thiocarbamic acid derivative represented by the following general formula or a salt thereof: 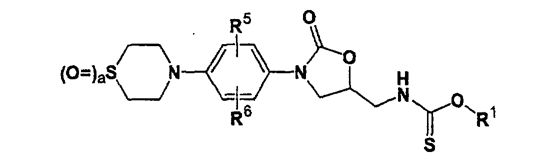 wherein R¹ represents an alkyl group which may be substituted, or a cycloalkyl group which may be substituted; R⁵ and R⁶ independently represent hydrogen atom, or a halogen atom; and symbol "a" represents an integer of from 0 to 2.

3. A thiocarbamic acid derivative represented by the following general formula or a salt thereof: 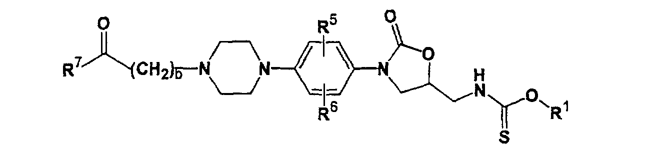 wherein R¹ represents an alkyl group which may be substituted, or a cycloalkyl group which may be substituted; R⁵ and R⁶ independently represent hydrogen atom, or a halogen atom; R⁷ represents an alkyl group which may be substituted, an amino group which may be substituted, or an alkoxyl group which may be substituted; and symbol "b" represents an integer of from 1 to 4.

4. A thiocarbamic acid derivative represented by the following general formula or a salt thereof: 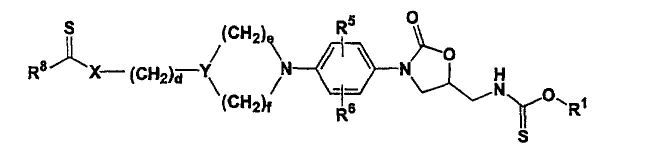 wherein R¹ represents an alkyl group which may be substituted, or a cycloalkyl group which may be substituted; R⁵ and R⁶ independently represent hydrogen atom, or a halogen atom; R⁸ represents an alkyl group which may be substituted, a cycloalkyl group which may be substituted, an alkenyl group which may be substituted, an alkynyl group which may be substituted, an alkoxyl group which may be substituted, an alkylthio group which may be substituted, an amino group which may be substituted, a saturated heterocyclic group which may be substituted, an aryl group which may be substituted, or an aralkyl group which may be substituted; Y represents CH or nitrogen atom; X represents NH or single bond; symbol "d" represents an integer of from 0 to 3; and symbols "e" and "f" independently represent an integer of from 1 to 3.

5. A medicament which comprises the compound or a salt thereof according to any one of claims 1 to 4 as an active ingredient.

6. The medicament according to claim 5 which is an antibacterial agent.

7. A use of the compound or a salt thereof according to any one of claims 1 to 4 for the manufacture of the medicament according to claim 5 or 6.

8. A method for preventive and/or therapeutic treatment of an infectious disease, which comprises the step of administering a preventively and/or therapeutically effective amount of the compound or a salt thereof according to any one of claims 1 to 4 to a mammal including a human.
